# EUROPEAN PATENT APPLICATION

(11) **EP 2 636 680 A2**
(43) Date of publication of application: **11.09.2013**
(21) Application number: 13162980.0
(22) Date of filing: 25.05.2007
(51) Int. Cl.: C07K 14/575, C07K 14/605, A61K 38/22

(54) **Composition and methods for treatment of congestive heart failure**

(30) Priority: 26.05.2006 US 808810 P
(62) Divisional of application: 07777280.4
(71) Applicant: Amylin Pharmaceuticals, LLC, San Diego, CA 92121 (US); AstraZeneca Pharmaceuticals LP, Wilmington, DE (US)
(72) Inventor: Ghosh, Soumitra, Shankar, San Diego, CA 92121 (US); Lewis, Diana, Y., San Diego, CA 92121 (US); Janssen, Samuel, San Diego, CA 92121 (US); Srivastava, Ved, San Diego, CA 92121 (US); Liu, Que, San Diego, CA 92121 (US); Jodka, Carolyn, M., San Diego, CA 92121 (US); Soares, Christopher, J., San Diego, CA 92121 (US); Lin, Qing, San Diego, CA 92121 (US)
(74) Representative: Raynor, Stuart Andrew

(57) **Abstract**

Provided herein is the use of GLP-1 molecules or agonists and analogs thereof, and the use of exendin molecules or agonists and analogs thereof, including their derivatives and active fragments, for the prevention or treatment of congestive heart failure. Pharmaceutical compositions for use in the methods described herein are also disclosed. Further provided are compositions and methods for the treatment and/or prevention of diabetes mellitus, hyperglycemia, insulin resistance and obesity, and for the reduction of food intake and suppression of appetite of subjects.

SEQUENCE LISTING

<110> AMYLIN PHARMACEUTICALS, INC.

<120> COMPOSITIONS AND METHODS FOR TREATMENT OF CONGESTIVE
      HEART FAILURE

<130> 0233WO

<140>
<141>

<150> 60/808,810
<151> 2006-05-26

<160> 106

<170> PatentIn Ver. 3.3

<210> 1
<211> 30
<212> PRT
<213> Artificial Sequence

<220>
<223> Description of Artificial Sequence: Synthetic
      polypeptide

<400> 1
His Ala Glu Gly Thr Phe Thr Ser Asp Val Ser Ser Tyr Leu Glu Gly
  1               5                  10                  15

Gln Ala Ala Lys Glu Phe Ile Ala Trp Leu Val Lys Gly Arg
             20                  25                  30


<210> 2
<211> 39
<212> PRT
<213> Artificial Sequence

<220>
<223> Description of Artificial Sequence: Synthetic
      polypeptide

<400> 2
His Gly Glu Gly Thr Phe Thr Ser Asp Leu Ser Lys Gln Met Glu Glu
  1               5                  10                  15

Glu Ala Val Arg Leu Phe Ile Glu Trp Leu Lys Asn Gly Gly Pro Ser
             20                  25                  30

Ser Gly Ala Pro Pro Pro Ser
         35

<210> 3
<211> 39
<212> PRT
<213> Artificial Sequence

<220>
<223> Description of Artificial Sequence: Synthetic
      polypeptide

<400> 3
His Gly Glu Gly Thr Phe Thr Ser Asp Leu Ser Lys Gln Leu Glu Glu
  1               5                  10                  15

Glu Ala Val Arg Leu Phe Ile Glu Trp Leu Lys Asn Gly Gly Pro Ser
             20                  25                  30

Ser Gly Ala Pro Pro Pro Ser
         35

<210> 4
<211> 39
<212> PRT
<213> Artificial Sequence

<220>
<223> Description of Artificial Sequence: Synthetic
      polypeptide

<400> 4
His Gly Asp Gly Thr Phe Thr Ser Asp Leu Ser Lys Gln Met Glu Glu
  1               5                  10                  15

Glu Ala Val Arg Leu Phe Ile Glu Trp Leu Lys Asn Gly Gly Pro Ser
             20                  25                  30

Ser Gly Ala Pro Pro Pro Ser
         35

<210> 5
<211> 39
<212> PRT
<213> Artificial Sequence

<220>
<223> Description of Artificial Sequence: Synthetic
      polypeptide

<400> 5
His Gly Glu Gly Thr Phe Thr Ser Glu Leu Ser Lys Gln Met Glu Glu
  1               5                  10                  15

Glu Ala Val Arg Leu Phe Ile Glu Trp Leu Lys Asn Gly Gly Pro Ser
            20                  25                  30

Ser Gly Ala Pro Pro Pro Ser
        35

<210> 6
<211> 39
<212> PRT
<213> Artificial Sequence

<220>
<223> Description of Artificial Sequence: Synthetic
      polypeptide

<400> 6
His Gly Glu Gly Thr Phe Thr Ser Asp Leu Ser Lys Gln Met Glu Glu
  1               5                  10                  15

Glu Ala Val Arg Leu Phe Ile Glu Trp Leu Lys Gln Gly Gly Pro Ser
            20                  25                  30

Ser Gly Ala Pro Pro Pro Ser
        35

<210> 7
<211> 39
<212> PRT
<213> Artificial Sequence

<220>
<223> Description of Artificial Sequence: Synthetic
      polypeptide

<400> 7
His Gly Glu Gly Thr Phe Thr Ser Asp Leu Ser Lys Gln Ala Glu Glu
  1               5                  10                  15

Glu Ala Val Arg Leu Phe Ile Glu Trp Leu Lys Gln Gly Gly Pro Ser
            20                  25                  30

Ser Gly Ala Pro Pro Pro Ser
        35

<210> 8
<211> 39
<212> PRT
<213> Artificial Sequence

<220>
<223> Description of Artificial Sequence: Synthetic
      polypeptide

<400> 8
His Gly Glu Gly Thr Phe Thr Ser Asp Leu Ser Lys Gln Ile Glu Glu
  1               5                  10                  15

Glu Ala Val Arg Leu Phe Ile Glu Trp Leu Lys Gln Gly Gly Pro Ser
             20                  25                  30

Ser Gly Ala Pro Pro Pro Ser
        35

<210> 9
<211> 39
<212> PRT
<213> Artificial Sequence

<220>
<223> Description of Artificial Sequence: Synthetic
      polypeptide

<400> 9
His Gly Glu Gly Thr Phe Thr Ser Asp Leu Ser Lys Gln Leu Glu Glu
  1               5                  10                  15

Glu Ala Val Arg Leu Phe Ile Glu Trp Leu Lys Gln Gly Gly Pro Ser
             20                  25                  30

Ser Gly Ala Pro Pro Pro Ser
        35

<210> 10
<211> 39
<212> PRT
<213> Artificial Sequence

<220>
<223> Description of Artificial Sequence: Synthetic
      polypeptide

<400> 10
His Gly Glu Gly Thr Phe Thr Thr Asp Leu Ser Lys Gln Leu Glu Glu
  1               5                  10                  15

Glu Ala Val Arg Leu Phe Ile Glu Trp Leu Lys Gln Gly Gly Pro Ser
             20                  25                  30

Ser Gly Ala Pro Pro Pro Ser
        35

<210> 11
<211> 39
<212> PRT
<213> Artificial Sequence

<220>
<223> Description of Artificial Sequence: Synthetic
      polypeptide

<400> 11
His Gly Glu Gly Thr Phe Thr Ser Asp Phe Ser Lys Gln Leu Glu Glu
1               5                   10                  15

Glu Ala Val Arg Leu Phe Ile Glu Trp Leu Lys Gln Gly Gly Pro Ser
            20                  25                  30

Ser Gly Ala Pro Pro Pro Ser
        35


<210> 12
<211> 39
<212> PRT
<213> Artificial Sequence

<220>
<223> Description of Artificial Sequence: Synthetic
      polypeptide

<220>
<221> MOD_RES
<222> (9)
<223> D(OMe)

<400> 12
His Gly Glu Gly Thr Phe Thr Ser Asp Leu Ser Lys Gln Leu Glu Glu
1               5                   10                  15

Glu Ala Val Arg Leu Phe Ile Glu Trp Leu Lys Gln Gly Gly Pro Ser
            20                  25                  30

Ser Gly Ala Pro Pro Pro Ser
        35


<210> 13
<211> 39
<212> PRT
<213> Artificial Sequence

<220>
<223> Description of Artificial Sequence: Synthetic
      polypeptide

<220>
<221> MOD_RES
<222> (9)
<223> D(OMe)

<400> 13
His Gly Glu Gly Thr Phe Thr Ser Asp Leu Ser Lys Gln Ala Glu Glu
1               5                   10                  15

Glu Ala Val Arg Leu Phe Ile Glu Trp Leu Lys Gln Gly Gly Pro Ser
            20                  25                  30

Ser Gly Ala Pro Pro Pro Ser
        35

<210> 14
<211> 39
<212> PRT
<213> Artificial Sequence

<220>
<223> Description of Artificial Sequence: Synthetic
      polypeptide

<400> 14
His Gly Glu Gly Thr Phe Thr Ser Asp Leu Ser Lys Gln Leu Glu Glu
1               5                   10                  15

Glu Ala Val Arg Leu Phe Ile Glu Trp Leu Lys Gln Gly Gly Pro Ser
            20                  25                  30

Ser Gly Phe Pro Pro Pro Ser
        35

<210> 15
<211> 39
<212> PRT
<213> Artificial Sequence

<220>
<223> Description of Artificial Sequence: Synthetic
      polypeptide

<400> 15
His Gly Glu Gly Thr Phe Thr Ser Asp Leu Ser Lys Gln Leu Glu Glu
1               5                   10                  15

Glu Ala Val Arg Leu Phe Ile Glu Trp Leu Lys Asn Gly Gly Pro Ser
            20                  25                  30

Ser Gly Phe Pro Pro Pro Ser
        35

<210> 16
<211> 39
<212> PRT
<213> Artificial Sequence

<220>
<223> Description of Artificial Sequence: Synthetic
      polypeptide

<400> 16
His Gly Glu Gly Thr Phe Thr Ser Asp Leu Ser Lys Gln Leu Glu Glu
  1               5                  10                  15

Glu Ala Val Arg Leu Phe Ile Glu Trp Leu Lys Gln Gly Gly Pro Ser
             20                  25                  30

Ser Gly Leu Pro Pro Pro Ser
        35

<210> 17
<211> 39
<212> PRT
<213> Artificial Sequence

<220>
<223> Description of Artificial Sequence: Synthetic
      polypeptide

<400> 17
His Gly Glu Gly Thr Phe Thr Ser Asp Leu Ser Lys Gln Leu Glu Glu
  1               5                  10                  15

Glu Ala Val Arg Leu Phe Ile Glu Trp Leu Lys Gln Gly Gly Pro Ser
             20                  25                  30

Ser Gly Pro Pro Pro Pro Ser
        35

<210> 18
<211> 39
<212> PRT
<213> Artificial Sequence

<220>
<223> Description of Artificial Sequence: Synthetic
      polypeptide

<400> 18
His Gly Glu Gly Thr Phe Thr Ser Asp Leu Ser Lys Gln Leu Glu Glu
1               5                   10                  15

Glu Ala Val Arg Leu Phe Ile Glu Trp Leu Lys Gln Gly Gly Pro Ser
            20                  25                  30

Ser Gly Thr Pro Pro Pro Ser
        35


<210> 19
<211> 39
<212> PRT
<213> Artificial Sequence

<220>
<223> Description of Artificial Sequence: Synthetic
      polypeptide

<400> 19
His Gly Glu Gly Thr Phe Thr Ser Asp Leu Ser Lys Gln Leu Glu Glu
1               5                   10                  15

Glu Ala Val Arg Leu Phe Ile Glu Trp Leu Lys Gln Gly Gly Pro Ser
            20                  25                  30

Ser Gly Val Pro Pro Pro Ser
        35


<210> 20
<211> 39
<212> PRT
<213> Artificial Sequence

<220>
<223> Description of Artificial Sequence: Synthetic
      polypeptide

<400> 20
His Gly Glu Gly Thr Phe Thr Ser Asp Leu Ser Lys Gln Leu Glu Glu
1               5                   10                  15

Glu Ala Val Arg Leu Phe Ile Glu Trp Leu Lys Gln Gly Gly Pro Ser
            20                  25                  30

Ser Gly Arg Pro Pro Pro Ser
        35

<210> 21
<211> 39
<212> PRT
<213> Artificial Sequence

<220>
<223> Description of Artificial Sequence: Synthetic
      polypeptide

<400> 21
His Gly Glu Gly Thr Phe Thr Ser Asp Leu Ser Lys Gln Leu Glu Glu
  1               5                  10                  15

Glu Ala Val Arg Leu Phe Ile Glu Trp Leu Lys Gln Gly Gly Pro Ser
             20                  25                  30

Ser Gly Lys Pro Pro Pro Ser
         35


<210> 22
<211> 39
<212> PRT
<213> Artificial Sequence

<220>
<223> Description of Artificial Sequence: Synthetic
      polypeptide

<400> 22
His Gly Glu Gly Thr Phe Thr Ser Asn Leu Ser Lys Gln Leu Glu Glu
  1               5                  10                  15

Glu Ala Val Arg Leu Phe Ile Glu Trp Leu Lys Gln Gly Gly Pro Ser
             20                  25                  30

Ser Gly Ala Pro Pro Pro Ser
         35


<210> 23
<211> 39
<212> PRT
<213> Artificial Sequence

<220>
<223> Description of Artificial Sequence: Synthetic
      polypeptide

<400> 23
His Gly Glu Gly Thr Phe Thr Ser Asp Lys Ser Lys Gln Leu Glu Glu
  1               5                  10                  15

Glu Ala Val Arg Leu Phe Ile Glu Trp Leu Lys Gln Gly Gly Pro Ser
            20                  25                  30

Ser Gly Ala Pro Pro Pro Ser
        35

<210> 24
<211> 39
<212> PRT
<213> Artificial Sequence

<220>
<223> Description of Artificial Sequence: Synthetic
polypeptide

<400> 24
His Gly Glu Gly Thr Phe Thr Ser Asp Trp Ser Lys Gln Leu Glu Glu
1               5                   10                  15

Glu Ala Val Arg Leu Phe Ile Glu Trp Leu Lys Gln Gly Gly Pro Ser
            20                  25                  30

Ser Gly Ala Pro Pro Pro Ser
        35

<210> 25
<211> 39
<212> PRT
<213> Artificial Sequence

<220>
<223> Description of Artificial Sequence: Synthetic
polypeptide

<400> 25
His Gly Glu Gly Thr Phe Thr Ser Asp Glu Ser Lys Gln Leu Glu Glu
1               5                   10                  15

Glu Ala Val Arg Leu Phe Ile Glu Trp Leu Lys Gln Gly Gly Pro Ser
            20                  25                  30

Ser Gly Ala Pro Pro Pro Ser
        35

<210> 26
<211> 39
<212> PRT
<213> Artificial Sequence

<220>
<223> Description of Artificial Sequence: Synthetic
      polypeptide

<400> 26
His Gly Glu Gly Thr Phe Thr Ser Asp Val Thr Gln Gln Leu Glu Glu
1               5                   10                  15

Glu Ala Val Arg Leu Phe Ile Glu Trp Leu Lys Gln Gly Gly Pro Ser
            20                  25                  30

Ser Gly Ala Pro Pro Pro Ser
        35


<210> 27
<211> 39
<212> PRT
<213> Artificial Sequence

<220>
<223> Description of Artificial Sequence: Synthetic
      polypeptide

<400> 27
His Gly Glu Gly Thr Phe Thr Ser Asp Leu Ser Lys Gln Leu Glu Glu
1               5                   10                  15

Lys Ala Ala Lys Glu Phe Ile Glu Trp Leu Lys Gln Gly Gly Pro Ser
            20                  25                  30

Ser Gly Ala Pro Pro Pro Ser
        35


<210> 28
<211> 39
<212> PRT
<213> Artificial Sequence

<220>
<223> Description of Artificial Sequence: Synthetic
      polypeptide

<400> 28
His Gly Glu Gly Thr Phe Thr Ser Asp Leu Ser Lys Gln Leu Glu Glu
1               5                   10                  15

Lys Ala Val Arg Leu Phe Ile Glu Trp Leu Lys Gln Gly Gly Pro Ser
            20                  25                  30

Ser Gly Ala Pro Pro Pro Ser
        35

<210> 29
<211> 39
<212> PRT
<213> Artificial Sequence

<220>
<223> Description of Artificial Sequence: Synthetic
      polypeptide

<400> 29
His Gly Glu Gly Thr Phe Thr Ser Asp Leu Ser Lys Gln Leu Glu Glu
1               5                   10                  15

Lys Ala Val Arg Leu Phe Ile Glu Trp Leu Lys Asn Gly Gly Pro Ser
            20                  25                  30

Ser Gly Ala Pro Pro Pro Ser
        35


<210> 30
<211> 39
<212> PRT
<213> Artificial Sequence

<220>
<223> Description of Artificial Sequence: Synthetic
      polypeptide

<400> 30
His Gly Glu Gly Thr Tyr Thr Asn Asp Leu Ser Lys Gln Leu Glu Glu
1               5                   10                  15

Glu Ala Val Arg Leu Phe Ile Glu Trp Leu Lys Gln Gly Gly Pro Ser
            20                  25                  30

Ser Gly Ala Pro Pro Pro Ser
        35


<210> 31
<211> 39
<212> PRT
<213> Artificial Sequence

<220>
<223> Description of Artificial Sequence: Synthetic
      polypeptide

<400> 31
His Gly Glu Gly Thr Phe Thr Ser Asp Val Thr Glu Tyr Leu Glu Glu
1               5                   10                  15

Glu Ala Val Arg Leu Phe Ile Glu Trp Leu Lys Gln Gly Gly Pro Ser
            20                  25                  30

Ser Gly Ala Pro Pro Pro Ser
        35

<210> 32
<211> 39
<212> PRT
<213> Artificial Sequence

<220>
<223> Description of Artificial Sequence: Synthetic
      polypeptide

<400> 32
His Gly Glu Gly Thr Tyr Thr Asn Asp Val Thr Glu Tyr Leu Glu Glu
  1               5                  10                  15

Glu Ala Val Arg Leu Phe Ile Glu Trp Leu Lys Gln Gly Gly Pro Ser
            20                  25                  30

Ser Gly Ala Pro Pro Pro Ser
        35

<210> 33
<211> 39
<212> PRT
<213> Artificial Sequence

<220>
<223> Description of Artificial Sequence: Synthetic
      polypeptide

<400> 33
His Gly Glu Gly Thr Tyr Thr Asn Asp Val Thr Glu Tyr Leu Glu Glu
  1               5                  10                  15

Glu Ala Val Arg Leu Phe Ile Glu Trp Leu Lys Asn Gly Gly Pro Ser
            20                  25                  30

Ser Gly Ala Pro Pro Pro Ser
        35

<210> 34
<211> 39
<212> PRT
<213> Artificial Sequence

<220>
<223> Description of Artificial Sequence: Synthetic
      polypeptide

<400> 34
His Gly Glu Gly Thr Tyr Thr Asn Asp Val Ser Ser Tyr Leu Glu Glu
  1               5                  10                  15

Glu Ala Ala Arg Leu Phe Ile Glu Trp Leu Lys Gln Gly Gly Pro Ser
             20                  25                  30

Ser Gly Ala Pro Pro Pro Ser
         35


<210> 35
<211> 38
<212> PRT
<213> Artificial Sequence

<220>
<223> Description of Artificial Sequence: Synthetic
      polypeptide

<400> 35
His Gly Glu Gly Thr Tyr Thr Asn Asp Val Ser Ser Tyr Leu Glu Gly
  1               5                  10                  15

Gln Ala Ala Arg Leu Phe Ile Glu Trp Leu Gln Gly Gly Pro Ser Ser
             20                  25                  30

Gly Ala Pro Pro Pro Ser
         35


<210> 36
<211> 39
<212> PRT
<213> Artificial Sequence

<220>
<223> Description of Artificial Sequence: Synthetic
      polypeptide

<400> 36
His Gly Glu Gly Thr Phe Thr Ser Asp Leu Ser Lys Gln Leu Glu Glu
  1               5                  10                  15

Arg Ala Val Arg Leu Phe Ile Glu Trp Leu Lys Gln Gly Gly Pro Ser
             20                  25                  30

Ser Gly Ala Pro Pro Pro Ser
         35

<210> 37
<211> 39
<212> PRT
<213> Artificial Sequence

<220>
<223> Description of Artificial Sequence: Synthetic
      polypeptide

<400> 37
His Gly Glu Gly Thr Phe Thr Ser Asp Leu Ser Lys Gln Lys Glu Glu
1               5                   10                  15

Glu Ala Val Arg Leu Phe Ile Glu Trp Leu Lys Gln Gly Gly Pro Ser
            20                  25                  30

Ser Gly Ala Pro Pro Pro Ser
        35

<210> 38
<211> 39
<212> PRT
<213> Artificial Sequence

<220>
<223> Description of Artificial Sequence: Synthetic
      polypeptide

<400> 38
His Gly Glu Gly Thr Phe Thr Ser Asp Leu Ser Lys Gln Leu Glu Glu
1               5                   10                  15

Glu Ala Val Arg Leu Phe Ile Glu Tyr Leu Lys Asn Gly Gly Pro Ser
            20                  25                  30

Ser Gly Ala Pro Pro Pro Ser
        35

<210> 39
<211> 39
<212> PRT
<213> Artificial Sequence

<220>
<223> Description of Artificial Sequence: Synthetic
      polypeptide

<400> 39
His Gly Glu Gly Thr Tyr Thr Asn Asp Val Thr Glu Tyr Leu Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser
        35

<210> 40
<211> 39
<212> PRT
<213> Artificial Sequence

<220>
<223> Description of Artificial Sequence: Synthetic
polypeptide

<400> 40
His Gly Glu Gly Thr Phe Thr Ser Asp Leu Ser Lys Gln Leu Glu Glu
1               5                   10                  15

Glu Ala Val Arg Leu Phe Ile Glu Trp Leu Lys Asn Gly Gly Pro Ser
            20                  25                  30

Ser Gly Phe Pro Pro Pro Ser
        35

<210> 41
<211> 39
<212> PRT
<213> Artificial Sequence

<220>
<223> Description of Artificial Sequence: Synthetic
polypeptide

<400> 41
His Gly Glu Gly Thr Phe Thr Ser Asp Leu Ser Lys Gln Ser Glu Glu
1               5                   10                  15

Glu Ala Val Arg Leu Phe Ile Glu Trp Leu Lys Gln Gly Gly Pro Ser
            20                  25                  30

Ser Gly Ala Pro Pro Pro Ser
        35

<210> 42
<211> 39
<212> PRT
<213> Artificial Sequence

<220>
<223> Description of Artificial Sequence: Synthetic
      polypeptide

<400> 42
His Gly Glu Gly Thr Phe Thr Ser Asp Leu Ser Lys Gln Leu Glu Glu
1               5                   10                  15

Lys Ala Val Arg Leu Phe Ile Glu Trp Leu Lys Asn Gly Gly Pro Ser
            20                  25                  30

Ser Gly Ala Pro Pro Pro Ser
        35


<210> 43
<211> 32
<212> PRT
<213> Artificial Sequence

<220>
<223> Description of Artificial Sequence: Synthetic
      polypeptide

<400> 43
His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Ile Pro Ser
            20                  25                  30


<210> 44
<211> 39
<212> PRT
<213> Artificial Sequence

<220>
<223> Description of Artificial Sequence: Synthetic
      polypeptide

<400> 44
His Gly Glu Gly Thr Phe Thr Ser Asp Leu Val Lys Ile Leu Glu Ala
1               5                   10                  15

Glu Ala Val Arg Lys Phe Ile Glu Phe Leu Lys Asn Gly Gly Pro Ser
            20                  25                  30

Ser Gly Ala Pro Pro Pro Ser
        35

<210> 45
<211> 32
<212> PRT
<213> Artificial Sequence

<220>
<223> Description of Artificial Sequence: Synthetic
      polypeptide

<400> 45
His Gly Glu Gly Thr Phe Thr Ser Asp Leu Ser Lys Gln Met Glu Glu
  1               5                  10                  15

Glu Ala Val Arg Leu Phe Ile Glu Trp Gly Ser Trp Gly Ile Pro Ser
             20                  25                  30

<210> 46
<211> 39
<212> PRT
<213> Artificial Sequence

<220>
<223> Description of Artificial Sequence: Synthetic
      polypeptide

<220>
<221> MOD_RES
<222> (29)
<223> OctG

<400> 46
His Gly Glu Gly Thr Phe Thr Ser Asp Leu Ser Lys Gln Leu Glu Glu
  1               5                  10                  15

Glu Ala Val Arg Leu Phe Ile Glu Trp Leu Lys Gln Xaa Gly Pro Ser
             20                  25                  30

Ser Gly Ala Pro Pro Pro Ser
         35

<210> 47
<211> 39
<212> PRT
<213> Artificial Sequence

<220>
<223> Description of Artificial Sequence: Synthetic
      polypeptide

<220>
<221> MOD_RES
<222> (30)
<223> OctG

<400> 47
His Gly Glu Gly Thr Phe Thr Ser Asp Leu Ser Lys Gln Leu Glu Glu
1               5                   10                  15

Glu Ala Val Arg Leu Phe Ile Glu Trp Leu Lys Gln Gly Xaa Pro Ser
            20                  25                  30

Ser Gly Ala Pro Pro Pro Ser
        35


<210> 48
<211> 39
<212> PRT
<213> Artificial Sequence

<220>
<223> Description of Artificial Sequence: Synthetic
      polypeptide

<220>
<221> MOD_RES
<222> (34)
<223> OctG

<400> 48
His Gly Glu Gly Thr Phe Thr Ser Asp Leu Ser Lys Gln Leu Glu Glu
1               5                   10                  15

Glu Ala Val Arg Leu Phe Ile Glu Trp Leu Lys Gln Gly Gly Pro Ser
            20                  25                  30

Ser Xaa Ala Pro Pro Pro Ser
        35


<210> 49
<211> 39
<212> PRT
<213> Artificial Sequence

<220>
<223> Description of Artificial Sequence: Synthetic
      polypeptide

<220>
<221> MOD_RES
<222> (34)
<223> OctG

<400> 49
His Gly Glu Gly Thr Phe Thr Ser Asp Leu Ser Lys Gln Ala Glu Glu
1               5                   10                  15

Glu Ala Val Arg Leu Phe Ile Glu Trp Leu Lys Asn Gly Gly Pro Ser
            20                  25                  30

Ser Xaa Ala Pro Pro Pro Ser
        35


<210> 50
<211> 39
<212> PRT
<213> Artificial Sequence

<220>
<223> Description of Artificial Sequence: Synthetic
      polypeptide

<220>
<221> MOD_RES
<222> (34)
<223> OctG

<400> 50
His Gly Glu Gly Thr Phe Thr Ser Asp Leu Ser Lys Gln Ala Glu Glu
1               5                   10                  15

Glu Ala Val Arg Leu Phe Ile Glu Phe Leu Lys Asn Gly Gly Pro Ser
            20                  25                  30

Ser Xaa Ala Pro Pro Pro Ser
        35


<210> 51
<211> 37
<212> PRT
<213> Artificial Sequence

<220>
<223> Description of Artificial Sequence: Synthetic
      polypeptide

<220>
<221> MOD_RES
<222> (34)
<223> OctG

<400> 51
His Gly Glu Gly Thr Phe Thr Ser Asp Leu Ser Lys Gln Leu Glu Glu
1               5                   10                  15

Glu Ala Val Arg Leu Phe Ile Glu Trp Leu Lys Asn Gly Lys Pro Lys
            20                  25                  30

Lys Xaa Arg Tyr Ser
        35

<210> 52
<211> 37
<212> PRT
<213> Artificial Sequence

<220>
<223> Description of Artificial Sequence: Synthetic
      polypeptide

<220>
<221> MOD_RES
<222> (35)
<223> OctG

<400> 52
His Gly Glu Gly Thr Phe Thr Ser Asp Leu Ser Lys Gln Leu Glu Glu
1               5                   10                  15

Glu Ala Val Arg Leu Phe Ile Glu Trp Leu Lys Asn Gly Gly Pro Ser
            20                  25                  30

Lys Glu Xaa Ile Ser
        35

<210> 53
<211> 37
<212> PRT
<213> Artificial Sequence

<220>
<223> Description of Artificial Sequence: Synthetic
      polypeptide

<220>
<221> MOD_RES
<222> (34)
<223> OctG

<400> 53
His Gly Glu Gly Thr Phe Thr Ser Asp Leu Ser Lys Gln Ala Glu Glu
1               5                   10                  15

Glu Ala Val Arg Leu Phe Ile Glu Trp Leu Lys Asn Gly Lys Pro Lys
            20                  25                  30

Lys Xaa Arg Tyr Ser
        35

<210> 54
<211> 37
<212> PRT
<213> Artificial Sequence

<220>
<223> Description of Artificial Sequence: Synthetic
      polypeptide

<220>
<221> MOD_RES
<222> (34)
<223> OctG

<400> 54
His Gly Glu Gly Thr Phe Thr Ser Asp Leu Ser Lys Gln Leu Glu Glu
  1               5                  10                  15

Glu Ala Val Arg Leu Phe Ile Glu Phe Leu Lys Asn Gly Lys Pro Lys
            20                  25                  30

Lys Xaa Arg Tyr Ser
        35

<210> 55
<211> 39
<212> PRT
<213> Artificial Sequence

<220>
<223> Description of Artificial Sequence: Synthetic
      polypeptide

<220>
<221> MOD_RES
<222> (34)
<223> OctG

<400> 55
His Gly Glu Gly Thr Phe Thr Ser Asp Leu Ser Lys Gln Leu Glu Glu
  1               5                  10                  15

Glu Ala Val Arg Leu Phe Ile Glu Trp Leu Lys Asn Gly Gly Pro Ser
            20                  25                  30

Ser Xaa Ala Pro Pro Pro Ser
        35

<210> 56
<211> 37
<212> PRT
<213> Artificial Sequence

<220>
<223> Description of Artificial Sequence: Synthetic
      polypeptide

<400> 56
His Gly Glu Gly Thr Phe Thr Ser Asp Leu Ser Lys Gln Leu Glu Glu
  1               5                  10                  15

Glu Ala Val Arg Leu Phe Ile Glu Trp Leu Ile Gln Gly Gly Pro Ser
             20                  25                  30

Lys Glu Ile Ile Ser
        35

<210> 57
<211> 37
<212> PRT
<213> Artificial Sequence

<220>
<223> Description of Artificial Sequence: Synthetic
      polypeptide

<400> 57
His Gly Glu Gly Thr Phe Thr Ser Asp Val Thr Gln Gln Leu Glu Glu
  1               5                  10                  15

Glu Ala Val Arg Leu Phe Ile Glu Trp Leu Ile Gln Gly Gly Pro Ser
             20                  25                  30

Lys Glu Ile Ile Ser
        35

<210> 58
<211> 37
<212> PRT
<213> Artificial Sequence

<220>
<223> Description of Artificial Sequence: Synthetic
      polypeptide

<400> 58
His Gly Glu Gly Thr Phe Thr Ser Asp Leu Ser Lys Gln Leu Glu Glu
1               5                   10                  15

Lys Ala Ala Lys Glu Phe Ile Glu Trp Leu Ile Gln Gly Gly Pro Ser
            20                  25                  30

Lys Glu Ile Ile Ser
        35

<210> 59
<211> 37
<212> PRT
<213> Artificial Sequence

<220>
<223> Description of Artificial Sequence: Synthetic
      polypeptide

<400> 59
His Gly Glu Gly Thr Phe Thr Ser Asp Leu Ser Lys Gln Leu Glu Glu
1               5                   10                  15

Lys Ala Val Arg Leu Phe Ile Glu Trp Leu Ile Gln Gly Gly Pro Ser
            20                  25                  30

Lys Glu Ile Ile Ser
        35

<210> 60
<211> 37
<212> PRT
<213> Artificial Sequence

<220>
<223> Description of Artificial Sequence: Synthetic
      polypeptide

<400> 60
His Gly Glu Gly Thr Phe Thr Ser Asp Leu Ser Lys Gln Leu Glu Glu
1               5                   10                  15

Glu Ala Val Arg Leu Phe Ile Glu Trp Leu Lys Gln Gly Gly Pro Ser
            20                  25                  30

Lys Glu Ile Ile Ser
        35

<210> 61
<211> 37
<212> PRT
<213> Artificial Sequence

<220>
<223> Description of Artificial Sequence: Synthetic
      polypeptide

<400> 61
His Gly Glu Gly Thr Phe Thr Ser Asp Leu Ser Lys Gln Leu Glu Glu
  1               5                  10                  15

Glu Ala Val Arg Leu Phe Ile Glu Trp Leu Lys Gln Gly Gly Pro Ser
             20                  25                  30

Lys Glu Ile Ile Ser
         35


<210> 62
<211> 31
<212> PRT
<213> Artificial Sequence

<220>
<223> Description of Artificial Sequence: Synthetic
      polypeptide

<400> 62
His Gly Glu Gly Thr Phe Thr Ser Asp Leu Ser Lys Gln Leu Glu Glu
  1               5                  10                  15

Glu Ala Val Arg Leu Phe Ile Glu Trp Leu Ile Lys Gly Arg Pro
             20                  25                  30


<210> 63
<211> 31
<212> PRT
<213> Artificial Sequence

<220>
<223> Description of Artificial Sequence: Synthetic
      polypeptide

<400> 63
His Gly Glu Gly Thr Phe Thr Ser Asp Leu Ser Lys Gln Leu Glu Glu
  1               5                  10                  15

Glu Ala Val Arg Leu Phe Ile Glu Trp Leu Lys Gln Gly Lys Pro
             20                  25                  30

<210> 64
<211> 31
<212> PRT
<213> Artificial Sequence

<220>
<223> Description of Artificial Sequence: Synthetic
      polypeptide

<400> 64
His Gly Glu Gly Thr Phe Thr Ser Asp Leu Ser Lys Gln Leu Glu Glu
  1               5                  10                  15

Glu Ala Val Arg Leu Phe Ile Glu Trp Leu Ile Gln Gly Lys Pro
             20                  25                  30


<210> 65
<211> 31
<212> PRT
<213> Artificial Sequence

<220>
<223> Description of Artificial Sequence: Synthetic
      polypeptide

<400> 65
His Gly Glu Gly Thr Phe Thr Ser Asp Leu Ser Lys Gln Leu Glu Glu
  1               5                  10                  15

Glu Ala Val Arg Leu Phe Ile Glu Trp Leu Ile Lys Gly Lys Pro
             20                  25                  30


<210> 66
<211> 37
<212> PRT
<213> Artificial Sequence

<220>
<223> Description of Artificial Sequence: Synthetic
      polypeptide

<400> 66
His Gly Glu Gly Thr Phe Thr Ser Asp Leu Ser Lys Gln Leu Glu Glu
  1               5                  10                  15

Glu Ala Val Arg Leu Phe Ile Glu Trp Leu Lys Gln Gly Lys Pro Lys
             20                  25                  30

Lys Ile Arg Tyr Ser
         35

<210> 67
<211> 38
<212> PRT
<213> Artificial Sequence

<220>
<223> Description of Artificial Sequence: Synthetic
      polypeptide

<400> 67
His Gly Glu Gly Thr Phe Thr Ser Asp Leu Ser Lys Gln Leu Glu Glu
  1               5                  10                  15

Glu Ala Val Arg Leu Phe Ile Glu Trp Leu Lys Asn Gly Gly Lys Pro
             20                  25                  30

Lys Lys Ile Arg Tyr Ser
         35


<210> 68
<211> 39
<212> PRT
<213> Artificial Sequence

<220>
<223> Description of Artificial Sequence: Synthetic
      polypeptide

<400> 68
His Gly Glu Gly Thr Phe Thr Ser Asp Leu Ser Lys Gln Leu Glu Glu
  1               5                  10                  15

Glu Ala Val Arg Leu Phe Ile Glu Trp Leu Lys Asn Gly Lys Pro Gly
             20                  25                  30

Lys Gly Lys Ile Arg Tyr Ser
         35


<210> 69
<211> 36
<212> PRT
<213> Artificial Sequence

<220>
<223> Description of Artificial Sequence: Synthetic
      polypeptide

<400> 69
His Gly Glu Gly Thr Phe Thr Ser Asp Leu Ser Lys Gln Leu Glu Glu
  1               5                  10                  15

Glu Ala Val Arg Leu Phe Ile Glu Trp Leu Lys Asn Pro Gly Gly Lys
            20                  25                  30

Glu Ile Ile Ser
        35

<210> 70
<211> 38
<212> PRT
<213> Artificial Sequence

<220>
<223> Description of Artificial Sequence: Synthetic
      polypeptide

<400> 70
His Gly Glu Gly Thr Phe Thr Ser Asp Leu Ser Lys Gln Leu Glu Glu
  1               5                  10                  15

Glu Ala Val Arg Leu Phe Ile Glu Trp Leu Lys Asn Gly Gly Pro Gly
            20                  25                  30

Gly Lys Glu Ile Ile Ser
        35

<210> 71
<211> 37
<212> PRT
<213> Artificial Sequence

<220>
<223> Description of Artificial Sequence: Synthetic
      polypeptide

<400> 71
His Gly Glu Gly Thr Phe Thr Ser Asp Leu Ser Lys Gln Leu Glu Glu
  1               5                  10                  15

Glu Ala Val Arg Leu Phe Ile Glu Trp Leu Lys Asn Gly Lys Pro Lys
            20                  25                  30

Lys Ile Arg Tyr Ala
        35

<210> 72
<211> 37
<212> PRT
<213> Artificial Sequence

<220>
<223> Description of Artificial Sequence: Synthetic
      polypeptide

<400> 72
His Gly Glu Gly Thr Phe Thr Ser Asp Leu Ser Lys Gln Leu Glu Glu
  1               5                  10                  15

Glu Ala Val Arg Leu Phe Ile Glu Trp Leu Lys Asn Gly Lys Pro Lys
             20                  25                  30

Lys Ile Ala Tyr Ser
         35


<210> 73
<211> 37
<212> PRT
<213> Artificial Sequence

<220>
<223> Description of Artificial Sequence: Synthetic
      polypeptide

<400> 73
His Gly Glu Gly Thr Phe Thr Ser Asp Leu Ser Lys Gln Leu Glu Glu
  1               5                  10                  15

Glu Ala Val Arg Leu Phe Ile Glu Trp Leu Lys Asn Gly Lys Pro Lys
             20                  25                  30

Lys Ala Arg Tyr Ser
         35


<210> 74
<211> 37
<212> PRT
<213> Artificial Sequence

<220>
<223> Description of Artificial Sequence: Synthetic
      polypeptide

<400> 74
His Gly Glu Gly Thr Phe Thr Ser Asp Leu Ser Lys Gln Leu Glu Glu
  1               5                  10                  15

Glu Ala Val Arg Leu Phe Ile Glu Trp Leu Lys Asn Gly Lys Pro Lys
             20                  25                  30

Ala Ile Arg Tyr Ser
         35

<210> 75
<211> 37
<212> PRT
<213> Artificial Sequence

<220>
<223> Description of Artificial Sequence: Synthetic
      polypeptide

<400> 75
His Gly Glu Gly Thr Phe Thr Ser Asp Leu Ser Lys Gln Leu Glu Glu
1               5                   10                  15

Glu Ala Val Arg Leu Phe Ile Glu Trp Leu Lys Asn Gly Lys Pro Ala
            20                  25                  30

Lys Ile Arg Tyr Ser
        35


<210> 76
<211> 37
<212> PRT
<213> Artificial Sequence

<220>
<223> Description of Artificial Sequence: Synthetic
      polypeptide

<400> 76
His Gly Glu Gly Thr Phe Thr Ser Asp Leu Ser Lys Gln Leu Glu Glu
1               5                   10                  15

Glu Ala Val Arg Leu Phe Ile Glu Trp Leu Lys Asn Gly Gly Pro Ser
            20                  25                  30

Lys Glu Ile Ile Ala
        35


<210> 77
<211> 37
<212> PRT
<213> Artificial Sequence

<220>
<223> Description of Artificial Sequence: Synthetic
      polypeptide

<400> 77
His Gly Glu Gly Thr Phe Thr Ser Asp Leu Ser Lys Gln Leu Glu Glu
1               5                   10                  15

Glu Ala Val Arg Leu Phe Ile Glu Trp Leu Lys Asn Gly Gly Pro Ser
            20                  25                  30

Lys Glu Ile Ala Ser
        35

<210> 78
<211> 37
<212> PRT
<213> Artificial Sequence

<220>
<223> Description of Artificial Sequence: Synthetic
      polypeptide

<400> 78
His Gly Glu Gly Thr Phe Thr Ser Asp Leu Ser Lys Gln Leu Glu Glu
  1               5                  10                  15

Glu Ala Val Arg Leu Phe Ile Glu Trp Leu Lys Asn Gly Gly Pro Ser
            20                  25                  30

Lys Ala Ile Ile Ser
        35

<210> 79
<211> 39
<212> PRT
<213> Artificial Sequence

<220>
<223> Description of Artificial Sequence: Synthetic
      polypeptide

<400> 79
His Gly Glu Gly Thr Phe Thr Ser Asp Leu Ser Lys Gln Leu Glu Glu
  1               5                  10                  15

Glu Ala Val Arg Leu Phe Ile Glu Trp Leu Lys Asn Gly Lys Pro Gly
            20                  25                  30

Gly Lys Lys Ile Arg Tyr Ser
        35

<210> 80
<211> 33
<212> PRT
<213> Artificial Sequence

<220>
<223> Description of Artificial Sequence: Synthetic
      polypeptide

<400> 80
His Ala Glu Gly Thr Phe Thr Ser Asp Val Ser Ser Tyr Leu Glu Gly
  1               5                  10                  15

Gln Ala Ala Lys Glu Phe Ile Ala Trp Leu Val Lys Gly Arg Gly Gly
             20                  25                  30

Gly

<210> 81
<211> 33
<212> PRT
<213> Artificial Sequence

<220>
<223> Description of Artificial Sequence: Synthetic
      polypeptide

<400> 81
His Ala Glu Gly Thr Phe Thr Ser Asp Val Ser Ser Tyr Leu Glu Gly
  1               5                  10                  15

Gln Ala Ala Lys Glu Phe Ile Ala Trp Leu Val Lys Gly Arg Gly Gly
             20                  25                  30

Gly

<210> 82
<211> 37
<212> PRT
<213> Artificial Sequence

<220>
<223> Description of Artificial Sequence: Synthetic
      polypeptide

<400> 82
His Ala Glu Gly Thr Phe Thr Ser Asp Val Ser Ser Tyr Leu Glu Gly
  1               5                  10                  15

Gln Ala Ala Lys Glu Phe Ile Ala Trp Leu Val Lys Gly Arg Gly Gly
             20                  25                  30

Gly Gly Ile Pro Ile
         35

<210> 83
<211> 38
<212> PRT
<213> Artificial Sequence

<220>
<223> Description of Artificial Sequence: Synthetic
      polypeptide

<400> 83
His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Pro Ser
            20                  25                  30

Gly Gly Gly Ile Pro Ile
        35

<210> 84
<211> 36
<212> PRT
<213> Artificial Sequence

<220>
<223> Description of Artificial Sequence: Synthetic
      polypeptide

<400> 84
His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Gly
            20                  25                  30

Gly Ile Pro Ile
        35

<210> 85
<211> 30
<212> PRT
<213> Artificial Sequence

<220>
<223> Description of Artificial Sequence: Synthetic
      polypeptide

<400> 85
His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg
            20                  25                  30

<210> 86
<211> 30
<212> PRT
<213> Artificial Sequence

<220>
<223> Description of Artificial Sequence: Synthetic
polypeptide

<400> 86
His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg
            20                  25                  30

<210> 87
<211> 41
<212> PRT
<213> Artificial Sequence

<220>
<223> Description of Artificial Sequence: Synthetic
polypeptide

<400> 87
His Ala His Gly Glu Gly Thr Phe Thr Ser Asp Leu Ser Lys Gln Leu
1               5                   10                  15

Glu Glu Glu Ala Val Arg Leu Phe Ile Glu Trp Leu Lys Gln Gly Gly
            20                  25                  30

Pro Ser Ser Gly Ala Pro Pro Pro Ser
        35                  40

<210> 88
<211> 42
<212> PRT
<213> Artificial Sequence

<220>
<223> Description of Artificial Sequence: Synthetic
polypeptide

<400> 88
His Ala Glu His Gly Glu Gly Thr Phe Thr Ser Asp Leu Ser Lys Gln
1               5                   10                  15

Leu Glu Glu Glu Ala Val Arg Leu Phe Ile Glu Trp Leu Lys Gln Gly
            20                  25                  30

Gly Pro Ser Ser Gly Ala Pro Pro Pro Ser
        35                  40

<210> 89
<211> 43
<212> PRT
<213> Artificial Sequence

<220>
<223> Description of Artificial Sequence: Synthetic
      polypeptide

<400> 89
His Ala Glu Gly His Gly Glu Gly Thr Phe Thr Ser Asp Leu Ser Lys
  1               5                  10                  15

Gln Leu Glu Glu Glu Ala Val Arg Leu Phe Ile Glu Trp Leu Lys Gln
            20                  25                  30

Gly Gly Pro Ser Ser Gly Ala Pro Pro Pro Ser
        35                  40

<210> 90
<211> 41
<212> PRT
<213> Artificial Sequence

<220>
<223> Description of Artificial Sequence: Synthetic
      polypeptide

<400> 90
Tyr Ala His Gly Glu Gly Thr Phe Thr Ser Asp Leu Ser Lys Gln Leu
  1               5                  10                  15

Glu Glu Glu Ala Val Arg Leu Phe Ile Glu Trp Leu Lys Asn Gly Gly
            20                  25                  30

Pro Ser Ser Gly Ala Pro Pro Pro Ser
        35                  40

<210> 91
<211> 41
<212> PRT
<213> Artificial Sequence

<220>
<223> Description of Artificial Sequence: Synthetic
      polypeptide

<400> 91
His Ser His Gly Glu Gly Thr Phe Thr Ser Asp Leu Ser Lys Gln Leu
  1               5                  10                  15

Glu Glu Glu Ala Val Arg Leu Phe Ile Glu Trp Leu Lys Asn Gly Gly
             20                  25                  30

Pro Ser Ser Gly Ala Pro Pro Pro Ser
         35                  40


<210> 92
<211> 41
<212> PRT
<213> Artificial Sequence

<220>
<223> Description of Artificial Sequence: Synthetic
      polypeptide

<400> 92
Tyr Pro His Gly Glu Gly Thr Phe Thr Ser Asp Leu Ser Lys Gln Leu
  1               5                  10                  15

Glu Glu Glu Ala Val Arg Leu Phe Ile Glu Trp Leu Lys Asn Gly Gly
             20                  25                  30

Pro Ser Ser Gly Ala Pro Pro Pro Ser
         35                  40


<210> 93
<211> 30
<212> PRT
<213> Artificial Sequence

<220>
<223> Description of Artificial Sequence: Synthetic
      polypeptide

<400> 93
His Ala Glu Gly Thr Phe Thr Ser Asp Val Ser Ser Tyr Leu Glu Gly
  1               5                  10                  15

Gln Ala Ala Lys Glu Phe Ile Ala Trp Leu Val Lys Gly Arg
             20                  25                  30

<210> 94
<211> 41
<212> PRT
<213> Artificial Sequence

<220>
<223> Description of Artificial Sequence: Synthetic
      polypeptide

<220>
<221> MOD_RES
<222> (36)
<223> OctG

<400> 94
His Ala His Gly Glu Gly Thr Phe Thr Ser Asp Leu Ser Lys Gln Leu
  1               5                  10                  15

Glu Glu Glu Ala Val Arg Leu Phe Ile Glu Trp Leu Lys Asn Gly Gly
             20                  25                  30

Pro Ser Ser Xaa Ala Pro Pro Pro Ser
         35                  40


<210> 95
<211> 45
<212> PRT
<213> Artificial Sequence

<220>
<223> Description of Artificial Sequence: Synthetic
      polypeptide

<400> 95
His Ala His Ala His Ala His Gly Glu Gly Thr Phe Thr Ser Asp Leu
  1               5                  10                  15

Ser Lys Gln Met Glu Glu Glu Ala Val Arg Leu Phe Ile Glu Trp Leu
             20                  25                  30

Lys Asn Gly Gly Pro Ser Ser Gly Ala Pro Pro Pro Ser
         35                  40                  45


<210> 96
<211> 28
<212> PRT
<213> Artificial Sequence

<220>
<223> Description of Artificial Sequence: Synthetic
      polypeptide

<400> 96
His Gly Glu Gly Thr Phe Thr Ser Asp Leu Ser Lys Gln Leu Glu Glu
1               5                   10                  15

Glu Ala Val Arg Leu Phe Ile Glu Phe Leu Lys Asn
            20                  25

<210> 97
<211> 28
<212> PRT
<213> Artificial Sequence

<220>
<223> Description of Artificial Sequence: Synthetic
      polypeptide

<400> 97
His Gly Glu Gly Thr Phe Thr Ser Asp Leu Ser Lys Gln Leu Glu Glu
1               5                   10                  15

Glu Ala Val Arg Leu Phe Ile Glu Trp Leu Lys Asn
            20                  25

<210> 98
<211> 28
<212> PRT
<213> Artificial Sequence

<220>
<223> Description of Artificial Sequence: Synthetic
      polypeptide

<400> 98
His Gly Glu Gly Thr Phe Thr Ser Asp Leu Ser Lys Gln Val Gln Glu
1               5                   10                  15

Glu Ala Val Arg Leu Phe Val Glu Phe Leu Lys Asn
            20                  25

<210> 99
<211> 28
<212> PRT
<213> Artificial Sequence

<220>
<223> Description of Artificial Sequence: Synthetic
      polypeptide

<400> 99
His Gly Glu Gly Thr Phe Thr Ser Asp Leu Val Lys Ile Leu Glu Ala
1               5                   10                  15

Glu Ala Val Arg Lys Phe Ile Glu Phe Leu Lys Asn
            20                  25

<210> 100
<211> 39
<212> PRT
<213> Artificial Sequence

<220>
<223> Description of Artificial Sequence: Synthetic
      polypeptide

<220>
<221> MOD_RES
<222> (14)
<223> OctG

<400> 100
His Gly Glu Gly Thr Phe Thr Ser Asp Leu Ser Lys Gln Xaa Glu Glu
  1               5                  10                  15

Glu Ala Val Arg Leu Phe Ile Glu Trp Leu Lys Gln Gly Gly Pro Ser
            20                  25                  30

Ser Gly Ala Pro Pro Pro Ser
        35

<210> 101
<211> 28
<212> PRT
<213> Artificial Sequence

<220>
<223> Description of Artificial Sequence: Synthetic
      polypeptide

<400> 101
His Gly Glu Gly Thr Phe Thr Ser Asp Leu Ser Lys Gln Leu Leu Glu
  1               5                  10                  15

Glu Ala Val Arg Leu Lys Val Glu Phe Leu Lys Asn
            20                  25

<210> 102
<211> 28
<212> PRT
<213> Artificial Sequence

<220>
<223> Description of Artificial Sequence: Synthetic
      polypeptide

<400> 102
His Gly Glu Gly Thr Phe Thr Ser Asp Leu Ser Lys Gln Leu Gln Glu
  1               5                  10                  15

Glu Ala Val Arg Leu Leu Asn Glu Phe Leu Lys Asn
             20                  25


<210> 103
<211> 28
<212> PRT
<213> Artificial Sequence

<220>
<223> Description of Artificial Sequence: Synthetic
      polypeptide

<400> 103
His Gly Glu Gly Thr Phe Thr Ser Asp Leu Ser Lys Gln Val Glu Glu
  1               5                  10                  15

Glu Ala Val Arg Leu Lys Asn Glu Phe Leu Lys Asn
             20                  25


<210> 104
<211> 28
<212> PRT
<213> Artificial Sequence

<220>
<223> Description of Artificial Sequence: Synthetic
      polypeptide

<400> 104
His Gly Glu Gly Thr Phe Thr Ser Asp Leu Ser Lys Gln Val Leu Glu
  1               5                  10                  15

Glu Ala Val Arg Leu Leu Ile Glu Phe Leu Lys Asn
             20                  25


<210> 105
<211> 28
<212> PRT
<213> Artificial Sequence

<220>
<223> Description of Artificial Sequence: Synthetic
      polypeptide

<400> 105
His Gly Glu Gly Thr Phe Thr Ser Asp Leu Ser Lys Gln Gln Glu Glu
1               5                   10                  15

Glu Ala Val Arg Leu Leu Val Glu Phe Leu Lys Asn
            20                  25

<210> 106
<211> 28
<212> PRT
<213> Artificial Sequence

<220>
<223> Description of Artificial Sequence: Synthetic
      polypeptide

<400> 106
His Gly Glu Gly Thr Phe Thr Ser Asp Leu Ser Lys Gln Gln Leu Glu
1               5                   10                  15

Glu Ala Val Arg Leu Phe Asn Glu Phe Leu Lys Asn
            20                  25

## Description

### FIELD OF THE INVENTION

The present invention relates generally to the use of GLP-1 molecules or agonists and analogs thereof, and to the use of exendin molecules or agonists and analogs thereof, and more particularly to the use of these molecules for the prevention or treatment of congestive heart failure. Pharmaceutical compositions for use in the methods of the invention are also disclosed. The present invention further relates to compositions and methods for the treatment of diabetes mellitus, for the prevention of hyperglycemia and for the reduction of food intake of subjects.

### BACKGROUND

Glucagon-like peptide-1[7-36] amide (also referred to as GLP-[7-36]NH₂ or GLP-1) is a product of the proglucagon gene. It is secreted into plasma mainly from the gut and produces a variety of biological effects related to pancreatic and gastrointestinal function. The parent peptide, proglucagon (PG), has numerous cleavage sites that produce other peptide products dependent on the tissue of origin including glucagon (PG[32-62]) and GLP-1[7-36]NH₂ (PG[78-107]) in the pancreas, and GLP-1[7-37] (PG[78-108]) and GLP-1[7-36]NH₂ (PG[78-107]) in the L cells of the intestine where GLP-1[7-36]NH₂ (78-107 PG) is the major product.

GLP-1[7-36]NH₂, or commonly, just "GLP-1," as used herein, has an insulinotropic effect, stimulating insulin secretion from pancreatic beta-cells; GLP-1 also inhibits glucagon secretion from pancreatic alpha-cells (Orskov, et al., Diabetes, 42:658-61, 1993; D'Alessio, et al., J. Clin. Invest., 97:133-38, 1996). GLP-1 is reported to inhibit gastric emptying (Williams B, et al., J. Clin. Endocrinol. Metab., 81 (1): 327-32, 1996; Wettergren A, et al., Dig. Dis. Sci., 38 (4): 665-73, 1993), and gastric acid secretion. (Schjoldager BT, et al., Dig. Dis. Sci., 34 (5): 703-8, 1989; O'Halloran DJ, et al., J. Endocrinol., 126 (1): 169-73, 1990; Wettergren A, et al., Dig. Dis. Sci., 38 (4): 665-73, 1993). A diuretic, antidypsogenic effect of intracerebroventricular administration of GLP-1 has been reported, however, this report claims that a peripheral, intraperitoneal injection of GLP-1 did not have this effect. (Tand-Christensen et al., Am. J. Physiol., 271:R848-56, 1996). GLP-1[7-37], which has an additional glycine residue at its carboxy terminus, also stimulates insulin secretion in humans (Orskov, et al., Diabetes, 42:658-61, 1993). A transmembrane G-protein adenylate-cyclase-coupled receptor believed to be responsible for the insulinotropic effect of GLP-1 has been cloned from a rat pancreatic islet cDNA library (Thorens, Proc. Natl. Acad. Sci. USA 89:8641-45, 1992).

Glucagon and glucagon-like peptides have been found to have different cardiovascular effects. Glucagon has been reported to have positive inotropic and chronotropic effects, produce a slight increase in arterial blood pressure in normal individuals, and affect regional blood circulation. A high dose of GLP-1 has been found to produce a moderate increase in both systolic and diastolic blood pressure, while GLP-2 has no effect on those parameters. An extremely high dose of GLP-1, administered through the jugular vein, has been reported to induce an increase in systolic and diastolic blood pressure and heart rate. This effect is mediated by GLP-1 receptors in the CNS. (Reviewed in Barragan, J. M., et al., Regulatory Peptides, 67:63-68, 1996).

Exendins are peptides that are found in the saliva of the Gila-monster, a lizard endogenous to Arizona, and the Mexican Beaded Lizard. Exendin-3 is present in the saliva of *Heloderma horridum,* and exendin-4 is present in the saliva of *Heloderma suspectum* (Eng, J., et al., J. Biol. Chem., 265:20259-62, 1990; Eng., J., et al., J. Biol. Chem., 267:7402-05, 1992). The exendins have some sequence similarity to several members of the glucagon-like peptide family, with the highest identity, 53%, being to GLP-1 (Goke, et al., J. Biol. Chem., 268:19650-55, 1993).

Exendin-4 is a potent GLP-1 receptor agonist. The peptide also stimulates somatostatin release and inhibits gastrin release in isolated stomachs (Goke, et al., J. Biol. Chem., 268:19650-55, 1993; Schepp, et al., Eur. J. Pharmacol., 69:183-91, 1994; Eissele, et al., Life Sci., 55:629-34, 1994). Exendin-3 and exendin-4 were found to be GLP-1 receptor agonists in stimulating cAMP production in, and amylase release from, pancreatic acinar cells (Malhotra, R., et al., Regulatory Peptides, 41:149-56, 1992; Raufinan, et al., J. Biol. Chem., 267:21432-37, 1992; Singh, et al., Regulatory Peptides., 53:47-59, 1994). The use of the insulinotropic activities of exendin-3 and exendin-4 for the treatment of diabetes mellitus and the prevention of hyperglycemia has been proposed (Eng, U.S. Pat. No. 5,424,286). The US Food and Drug Administration (FDA) has approved BYETTA^{®} (exenatide) injection as adjunctive therapy to improve glycemic control in subjects with type 2 diabetes mellitus who are taking metformin, a sulfonylurea, or a combination of metformin and a sulfonylurea but have not achieved adequate glycemic control.

Truncated exendin peptides such as exendin-[9-39], a carboxyamidated molecule, and fragments 3-39 through 9-39 have been reported to be potent and selective antagonists of GLP-1 (Goke, et al., J. Biol. Chem., 268:19650-55, 1993; Raufman, J. P., et al., J. Biol. Chem., 266:2897-902, 1991; Schepp, W., et al., Bur. J. Pharm., 269:183-91, 1994; Montrose-Rafizadeh, et al., Diabetes, 45(Suppl. 2):152A, 1996). Exendin-[9-39] blocks endogenous GLP-1 *in vivo,* resulting in reduced insulin secretion. Wang, et al., J. Clin. Invest., 95:417-21, 1995; D'Alessio, et al., J. Clin. Invest., 97:133-38, 1996). The receptor apparently responsible for the insulinotropic effect of GLP-1 has been cloned from rat pancreatic islet cells (Thorens, B., Proc. Natl. Acad. Sci. USA 89:8641-8645, 1992). Exendins and exendin-[9-39] bind to the cloned GLP-1 receptor (rat pancreatic -cell GLP-1 receptor: Fehmann HC, et al., Peptides, 15 (3): 453-6, 1994; human GLP-1 receptor: Thorens B, et al., Diabetes, 42 (11): 1678-82, 1993). In cells transfected with the cloned GLP-1 receptor, exendin-4 is an agonist, *i.e.,* it increases cAMP, while exendin-[9-39] is an antagonist, i.e., it blocks the stimulatory actions of exendin-4 and GLP-1. Id.

Exendin-[9-39] also acts as an antagonist of the full length exendins, inhibiting stimulation of pancreatic acinar cells by exendin-3 and exendin-4 (Raufman, et al., J. Biol. Chem., 266:2897-902, 1991; Raufinan, et al., J. Biol. Chem., 266:21432-37, 1992). Exendin-[9-39] inhibits the stimulation of plasma insulin levels by exendin-4, and inhibits the somatostatin release-stimulating and gastrin release-inhibiting activities of exendin-4 and GLP-1 (Kolligs, F., et al., Diabetes, 44:16-19, 1995; Eissele, et al., Life Sciences, 55:629-34, 1994). Exendin-4, administered through the jugular vein, has been reported to induce an increase in systolic, diastolic and mean arterial blood pressure, and in heart rate (Barragan, et al., Regulatory Peptides, 67:63-68, 1996).

Congestive heart failure ("CHF") is one of the most common causes of death and disability in industrialized nations and has a mortality rate of about 50% at five years (Goodman and Gilman's The Pharmacological Basis of Therapeutics, 9th Ed. McGraw Hill, N.Y., pp. 809-838). Congestive heart failure may be caused by the occurrence of an index event such as a myocardial infarction or may be secondary to other causes such as hypertension, ischemic heart disease, cardiac malformations such as valvular disease, or exposure to cardiotoxic compounds such as the anthracycline antibiotics. Without being limited by theory, it has been reported that the increased workload that results from high blood pressure or the loss of contractile tissue induces compensatory cardiomyocyte hypertrophy and thickening of the left ventricular wall, thereby enhancing contractility and maintaining cardiac function. However, over time, the left ventricular chamber dilates, systolic and diastolic function deteriorates, cardiomyocytes undergo apoptotic cell death, and myocardial function progressively deteriorates.

Congestive heart failure can develop slowly. The initial decline in pumping capacity may not be immediately noticeable due to the activation of one or more compensatory mechanisms. In addition, the progression of CHF has been found to be independent of the patient's hemodynamic status. Therefore, the damaging changes caused by the disease may be present and ongoing even while the patient remains asymptomatic. In fact, the compensatory mechanisms which maintain normal cardiovascular function during the early phases of CHF may actually contribute to progression of the disease, for example by exerting deleterious effects on the heart and circulation.

The myocardium predominantly uses free fatty acids as its major energy source. However, glucose remains the most efficient source of myocardial ATP production in situations of myocardial ischemia or injury due to the relative economy of O₂ consumption. A major problem in congestive heart failure is stress hyperglycemia and insulin resistance. As a result of a combination of high circulating levels of free fatty acids and reduced glucose uptake, there is a shift toward fatty acid oxidation, depletion of Krebs cycle intermediates and diminished glucose oxidation. These changes ultimately lead to reduced levels of creatine phosphate, loss of energy reserve and low efficiency of energy utilization.

Agents currently used for treatment of congestive heart failure include angiotensin converting enzyme (ACE) inhibitors, beta-blockers, compounds that induce inotropic effects (e.g., increase of force of contraction of the heart) and compounds that increase urine flow, or diuretics. Among other drawbacks associated with the use of these agents for treatment of congestive heart failure, these agents do not adequately address the problems associated with stress hyperglycemia and insulin resistance.

Diuretics have several properties that make them suboptimal agents for treatment of congestive heart failure. One difficulty encountered with many diuretics such as thiazides, loop diuretics, carbonic anhydrase inhibitors, and osmotic diuretics, is that although they may be employed to increase sodium excretion, they also result in an increase of urinary potassium loss. Examples of the effects of potassium loss include muscular weakness, paralysis (including the paralysis of respiratory muscles), electrocardiographic abnormalities, cardiac dysrhythmia, and cardiac arrest. Another difficulty encountered with some diuretics is their slow rate of action, which is not conducive to their use in an emergency setting.

Inotropic agents currently in clinical use include digitalis, sympathomimetic amines and amrinone (Harrison's Principles of Internal Medicine, 12th Edition, 1991, McGraw Hill, N.Y., pp. 894-899). Digotoxin, a cardiac glycoside, an ancient but effective therapy for cardiac failure, was initially derived from the foxglove leaf, Digitalis purpurea and Digitalis lanata. Cardiac glycosides are potent and highly selective inhibitors of the active transport of sodium and potassium ions across cell membranes (Goodman and Gilman, *supra*). Cardiac glycosides have been reported to increase the velocity of shortening of cardiac muscle, resulting in an improvement in ventricular function; this effect has been reported to be due to an increase in the availability during systole of cytosolic Ca²⁺ to interact with contractile proteins in increase the velocity and extent of sarcomere shortening (Goodman and Gilman, *supra*).

Digotoxin and related cardiac glycosides (e.g. digitoxin) have useful durations of action because their excretion, mainly via the kidneys, results in plasma t_{1/2} of 1.5-5 days. But the therapeutic index of these drugs is very low with the mildly toxic:minimally-effective dose ratio being 2:1 and the lethal:minimally-effective dose ratio being between 5:1 and 10:1. Urinary potassium loss due to use of thiazide and loop diuretics may seriously enhance the dangers of digitalis intoxication, including susceptibility to cardiac arrhythmia, and potassium-sparing diuretics are often necessary. Slow elimination of cardiac glycosides can prolong the period of jeopardy during digitalis intoxication, which has been reported to occur in 20% of hospital subjects on these drugs. Absorption and onset of action for all cardiac glycosides except ouabain is somewhat prolonged, and this may be a disadvantage in emergency cardiac conditions.

Sympathomimetic amines, which generally include epinephrine, isoproterenol, dopamine and dobutamine, can be useful in an acute setting to stimulate myocardial contractility, but they usually require constant intravenous infusion and continuous intensive monitoring of the subject. They typically lose their effectiveness after 8 hours, apparently due to receptor downregulation.

Thus, there is a need for improved agents for treatment of congestive heart failure. Such methods, and compounds and compositions which are useful therefore, have been invented and are described and claimed herein.

The use of GLP-1, exendins, and exendin or GLP-1 agonists for treatment of congestive heart failure has been proposed. *See, e.g.,* U.S. 6,703,359, filed February 5, 1999, which enjoys common ownership with the present invention and is hereby incorporated by reference. Recent studies have demonstrated that acute treatment with GLP-1 (48-72 hours infusion) can improve cardiac function in humans and animals post-infarction, by improving myocardial glucose utilization. *(See, e.g.,* Nikolaidis, L.A. et al., Circulation, 110: 955-961, 2004; Nikolaidis, L.A. et al., Circulation, 109:962-965, 2004; Bose, A.K. et al., Diabetes, 54:146-151, 2005). However, the efficacy of long-term or chronic treatment with GLP-1 or exenatide on cardiac function and remodeling in congestive heart failure was previously unknown. The present application concerns the surprising discovery that treatment, and in particular chronic treatment, with GLP-1, an exendin, or an exendin or GLP-1 agonist or analog can improve cardiac function, attenuate cardiac remodeling and enhance exercise capacity in a congestive heart failure animal model. Chronic treatment with GLP-1, an exendin, or an exendin or GLP-1 agonist also improves exercise performance and improves insulin sensitivity. Chronic administration of GLP-1 or incretin mimetics represents a potentially novel therapeutic approach for the treatment of congestive heart failure.

The compounds and compositions disclosed herein are also useful in the reduction of food intake and the treatment of obesity. Exendins have been found to inhibit gastric emptying (U.S. patent application Ser. No. 08/694,954, filed Aug. 8, 1996, which enjoys common ownership with the present invention and is hereby incorporated by reference). Exendin-[9-39] has been used to investigate the physiological relevance of central GLP-1 in control of food intake (Turton, M. D. et al., Nature, 379:69-72, 1996). GLP-1 administered by intracerebroventricular (ICV) injection inhibits food intake in rats. This satiety-inducing effect of GLP-1 delivered by intracerebroventricular injection is reported to be inhibited by ICV injection of exendin-[9-39] (Turton, *supra*). However, it has been reported that GLP-1 does not inhibit food intake in mice when administered by peripheral injection (Turton, M. D., Nature 379:69-72, 1996; Bhavsar, S. P., Soc. Neurosci. Abstr. 21:460 (188.8), 1995). Administration of exendins and exendin analogs has also been found to reduce food intake (U.S. Patent 6,956,026, filed Jan. 7, 1998, which enjoys common ownership with the present invention and is hereby incorporated by reference).

Obesity, excess adipose tissue, is becoming increasingly prevalent in developed societies. For example, approximately 30% of adults in the U.S. were estimated to be 20 percent above desirable body weight-an accepted measure of obesity sufficient to impact a health risk (Harrison's Principles of Internal Medicine 12th Edition, McGraw Hill, Inc. (1991) p. 411). The pathogenesis of obesity is believed to be multifactorial but the basic problem is that in obese subjects food intake and energy expenditure do not come into balance until there is excess adipose tissue. Attempts to reduce food intake, or hypernutrition, are usually fruitless in the medium term because the weight loss induced by dieting results in both increased appetite and decreased energy expenditure *(*Leibel et al., (1995) New England Journal of Medicine, 322: 621-628). The intensity of physical exercise required to expend enough energy to materially lose adipose mass is too great for most people to undertake on a sufficiently frequent basis. Thus, obesity is currently a poorly treatable, chronic, essentially intractable metabolic disorder. Not only is obesity itself believed by some to be undesirable for cosmetic reasons, but obesity also carries serious risk of co-morbidities including, Type 2 diabetes, degenerative arthritis, and increased incidence of complications of surgery involving general anesthesia. Overweight and obesity are associated with numerous cardiac complications such as increased cardiac risk, hypertension, atherosclerosis, congestive heart failure, and sudden death. Obesity due to hypernutrition is also a risk factor for the group of conditions called insulin resistance syndrome, or "syndrome X." In syndrome X, it has been reported that there is a linkage between insulin resistance and hypertension. (Watson N. and Sandler M., Curr. Med. Res. Opin., 12(6):374-378 (7991); Kodama J. et al., Diabetes Care, 13(11):1109-1111 (1990); Lithell et al., J. Cardiovasc. Pharmacol., 15 Suppl. 5:846-852 (1990)).

In those few subjects who do succeed in losing weight, by about 10 percent of body weight, there can be striking improvements in co-morbid conditions, most especially Type 2 diabetes in which dieting and weight loss are the primary therapeutic modality, albeit relatively ineffective in many subjects for the reasons stated above. Reducing food intake in obese subjects would decrease the plasma glucose level, the plasma lipid level, and the cardiac risk in these subjects. Hypernutrition is also the result of, and the psychological cause of, many eating disorders. Reducing food intake would also be beneficial in the treatment of such disorders.

Thus, it can be appreciated that an effective means to reduce food intake is a major challenge and a superior method of treatment would be of great utility. Such a method, and compounds and compositions which are useful thereof, have been invented and are described and claimed herein. Moreoever, because overweight and obesity are risk factors for cardiac disease, the methods described herein for treatment of obesity and reduction of food intake may also delay the onset or reduce the severity of congestive heart failure or other cardiac disease that is secondary to obesity and overweight.

### SUMMARY

Provided herein is the use of GLP-1 molecules or agonists and analogs thereof, and the use of exendin molecules or agonists and analogs thereof, including their derivatives and active fragments, for the prevention or treatment of congestive heart failure. Pharmaceutical compositions for use in the methods described herein are also disclosed. Further provided are compositions and methods for the treatment and/or prevention of diabetes mellitus, hyperglycemia, insulin resistance and obesity, and for the reduction of food intake and suppression of appetite of subjects.

GLP-1, exendins, and exendin or GLP-1 agonists and analogs of the present application include those shown in Table 1 (SEQ ID NOS 1-99, respectively in order of appearance).

In one embodiment is provided a polypeptide comprising the amino acid sequence HGEGTFTSDLSKQLEEKAAKEFIEWLKQGGPSSGAPPPS (SEQ ID NO: 27), or its C-terminal amide (-NH2) form. The polypeptides herein can optionally comprise a C-terminal amide, which is denoted as "-NH2." This terminal amide can be included during peptide chemical synthesis, added in vivo or added post-synthesis via chemical or enzymatic means. In another embodiment is provided a polypeptide comprising the amino acid sequence HGEGTFTSDLSKQLEEEAVRLFIEWLKQGGPSKEIIIS-OH (SEQ ID NO: 60).

Further embodiments relate to GLP-1 analogs and exendin analogs comprising one or more substitutions with a modified amino acid comprising a C₁-C₂₀ alkyl side chain (e.g., an octyl chain). One embodiment relates to GLP-1 analogs and exendin analogs comprising one or more octylglycine residues. Another embodiment relates to GLP-1 analogs and exendin analogs comprising an octylglycine residue at amino acid position 14.

In one embodiment is provided a polypeptide comprising the amino acid sequence HGEGTFTSDLSKQ[OctG]EEEAVRLFIEWLKQGGPSSGAPPPS (SEQ ID NO: 100).

In yet another embodiment, is provided a polypeptide comprising the amino acid sequence HGEGTFTSDLSKQLEEEAVRLFIEWLKQGGPSS[OctG]APPPS (SEQ ID NO: 48).

In one embodiment, a method for treating congestive heart failure is provided. The method generally comprises administering to a subject in need thereof an amount of GLP-1, an exendin, or an exendin or GLP-1 agonist or analog effective to treat congestive heart failure. In one embodiment the GLP-1, exendin or GLP-1 or exendin agonist or analog is any of the novel peptides disclosed herein, for example those contained in Table 1. In one aspect, the GLP-1, exendin, or exendin or GLP-1 agonist or analog is chronically administered. In another embodiment, the method comprises administering to a subject in need thereof an amount of a GLP-1 agonist or analog or exendin agonist or analog effective to treat congestive heart failure. In one aspect, the GLP-1 agonist or analog or exendin agonist or analog is chronically administered. In another aspect, the GLP-1 agonist or analog or exendin agonist or analog is acutely administered.

In another embodiment, a method for preventing congestive heart failure is provided. The method generally comprises administering to a subject in need thereof an amount of GLP-1, an exendin, or an exendin or GLP-1 agonist or analog effective to prevent congestive heart failure. In one embodiment the GLP-1, exendin or GLP-1 or exendin agonist or analog is any of the novel peptides disclosed herein, for example those contained in Table 1. In one aspect, the GLP-1, exendin, or exendin or GLP-1 agonist or analog is chronically administered. In another embodiment, the method comprises administering to a subject in need thereof an amount of a GLP-1 agonist or analog or exendin agonist or analog effective to prevent congestive heart failure. In one aspect, the GLP-1 agonist or analog or exendin agonist or analog is chronically administered. In another aspect, the GLP-1 agonist or analog or exendin agonist or analog is acutely administered.

In another embodiment, a method for improving cardiac function associated with congestive heart failure is provided. The method generally comprises administering to a subject in need thereof an amount of GLP-1, an exendin, or an exendin or GLP-1 agonist or analog effective to improve cardiac function associated with congestive heart failure. In one embodiment the GLP-1, exendin or GLP-1 or exendin agonist or analog is any of the novel peptides disclosed herein, for example those contained in Table 1. In another embodiment, the method comprises administering to a subject in need thereof an amount of a GLP-1 agonist or analog or exendin agonist or analog to improve cardiac function associated with congestive heart failure. In one aspect, the GLP-1 agonist or analog or exendin agonist or analog is chronically administered. In another aspect, the GLP-1 agonist or analog or exendin agonist or analog is acutely administered.

In another embodiment, a method for attenuating cardiac remodeling is provided. The method generally comprises administering to a subject in need thereof an amount of GLP-1, an exendin, or an exendin or GLP-1 agonist or analog effective to attenuate cardiac remodeling. In one embodiment the GLP-1, exendin or GLP-1 or exendin agonist or analog is any of the novel peptides disclosed herein, for example those contained in Table 1. In one embodiment, the method comprises administering to a subject in need thereof an amount of a GLP-1 agonist or analog or exendin agonist or analog effective to attenuate cardiac remodeling. In one aspect, the cardiac remodeling occurs prior to diagnosis of, or prior to onset of congestive heart failure. In another aspect, the cardiac remodeling occurs after diagnosis of, or after onset of congestive heart failure. In another aspect, the cardiac remodeling is associated with congestive heart failure. In yet another aspect, the cardiac remodeling occurs after myocardial infarction. In one aspect, the GLP-1 agonist or analog or exendin agonist or analog is chronically administered. In another aspect, the GLP-1 agonist or analog or exendin agonist or analog is acutely administered.

In another embodiment, a method for limiting infarct size is provided. The method generally comprises administering to a subject in need thereof an amount of GLP-1, an exendin, or an exendin or GLP-1 agonist or analog effective to limit infarct size. In one embodiment the GLP-1, exendin or GLP-1 or exendin agonist or analog is any of the novel peptides disclosed herein, for example those contained in Table 1. In an aspect, the GLP-1, exendin, or exendin or GLP-1 agonist or analog is chronically administered. In an embodiment, the method comprises administering to a subject in need thereof an amount of a GLP-1 agonist or analog or exendin agonist or analog effective to limit infarct size. In an aspect, the subject in need thereof has experienced or is experiencing a myocardial infarction. In another aspect, the GLP-1 agonist or analog or exendin agonist or analog is chronically administered.

In another embodiment, a method for attenuating insulin resistance associated with congestive heart failure is provided. The method generally comprises administering to a subject in need thereof an amount of GLP-1, an exendin, or an exendin or GLP-1 agonist or analog effective to attenuate insulin resistance associated with congestive heart failure. In one embodiment the GLP-1, exendin or GLP-1 or exendin agonist or analog is any of the novel peptides disclosed herein, for example those contained in Table 1. In an embodiment, the method comprises administering to a subject in need thereof an amount of a GLP-1 agonist or analog or exendin agonist or analog effective to attenuate insulin resistance associated with congestive heart failure. In one aspect, the GLP-1 agonist or analog or exendin agonist or analog is chronically administered. In another aspect, the GLP-1 agonist or analog or exendin agonist or analog is acutely administered.

In another embodiment, a method for improving exercise capacity in a subject having congestive heart failure is provided. The method generally comprises administering to a subject in need thereof an amount of GLP-1, an exendin, or an exendin or GLP-1 agonist or analog effective to improve exercise capacity. In one embodiment the GLP-1, exendin or GLP-1 or exendin agonist or analog is any of the novel peptides disclosed herein, for example those contained in Table 1. In an embodiment, the method comprises administering to a subject in need thereof an amount of a GLP-1 agonist or analog or exendin agonist or analog effective to improve exercise capacity. In one aspect, the GLP-1 agonist or analog or exendin agonist or analog is chronically administered. In another aspect, the GLP-1 agonist or analog or exendin agonist or analog is acutely administered.

In another embodiment, a method for treating diabetes mellitus is provided. The method generally comprises administering to a subject in need thereof an amount of a GLP-1 agonist or analog or exendin agonist or analog effective to treat diabetes mellitus. In one embodiment the GLP-1, exendin or GLP-1 or exendin agonist or analog is any of the novel peptides disclosed herein, for example those contained in Table 1.

In another embodiment, a method for treating insulin resistance is provided. The method generally comprises administering to a subject in need thereof an amount of a GLP-1 agonist or analog or exendin agonist or analog effective to treat insulin resistance. In one embodiment the GLP-1, exendin or GLP-1 or exendin agonist or analog is any of the novel peptides disclosed herein, for example those contained in Table 1.

In another embodiment, a method for treating postprandial hyperglycemia is provided. The method generally comprises administering to a subject in need thereof an amount of a GLP-1 agonist or analog or exendin agonist or analog effective to treat postprandial hyperglycemia. In one embodiment the GLP-1, exendin or GLP-1 or exendin agonist or analog is any of the novel peptides disclosed herein, for example those contained in Table 1.

In another embodiment, a method for lowering blood glucose is provided. The method generally comprises administering to a subject in need thereof an amount of a GLP-1 agonist or analog or exendin agonist or analog effective to lower blood glucose. In one embodiment the GLP-1, exendin or GLP-1 or exendin agonist or analog is any of the novel peptides disclosed herein, for example those contained in Table 1.

In another embodiment, a method for stimulating insulin release is provided. The method generally comprises administering to a subject in need thereof an amount of a GLP-1 agonist or analog or exendin agonist or analog effective to stimulate insulin release. In one embodiment the GLP-1, exendin or GLP-1 or exendin agonist or analog is any of the novel peptides disclosed herein, for example those contained in Table 1.

In another embodiment, a method for reducing food intake in a subject desirous or in need of reducing food intake is provided. The method generally comprises peripherally administering to the subject an amount of a GLP-1 agonist or analog or exendin agonist or analog effective to reduce food intake. In one embodiment the GLP-1, exendin or GLF-1 or exendin agonist or analog is any of the novel peptides disclosed herein, for example those contained in Table 1.

In another embodiment, a method for reducing appetite in a subject desirous or in need of reducing appetite is provided. The method generally comprises peripherally administering to the subject an amount of a GLP-1 agonist or analog or exendin agonist or analog effective to reduce appetite. In one embodiment the GLP-1, exendin or GLP-1 or exendin agonist or analog is any of the novel peptides disclosed herein, for example those contained in Table 1.

In another embodiment, a method for reducing food intake in a subject desirous or in need of reducing body weight is provided. The method generally comprises peripherally administering to the subject an amount of a GLP-1 agonist or analog or exendin agonist or analog effective to reduce body weight. In one embodiment the GLP-1, exendin or GLP-1 or exendin agonist or analog is any of the novel peptides disclosed herein, for example those contained in Table 1.

In another embodiment, a method for treating obesity is provided. The method generally comprises administering to a subject in need thereof an amount of a GLP-1 agonist or analog or exendin agonist or analog effective to treat obesity. In one embodiment the GLP-1, exendin or GLP-1 or exendin agonist or analog is any of the novel peptides disclosed herein, for example those contained in Table 1.

In another embodiment, a method for treating obesity-related cardiac disease is provided. The method generally comprises administering to a subject in need thereof an amount of a GLP-1 agonist or analog or exendin agonist or analog effective to treat obesity-related cardiac disease. In one embodiment the GLP-1, exendin or GLP-1 or exendin agonist or analog is any of the novel peptides disclosed herein, for example those contained in Table 1. In one aspect, the GLP-1 agonist or analog or exendin agonist or analog is chronically administered.

In another embodiment, a method for treating obesity-related congestive heart failure is provided. The method generally comprises administering to a subject in need thereof an amount of a GLP-1 agonist or analog or exendin agonist or analog effective to treat obesity-related congestive heart failure. In one embodiment the GLP-1, exendin or GLP-1 or exendin agonist or analog is any of the novel peptides disclosed herein, for example those contained in Table 1. In one aspect, the GLP-1 agonist or analog or exendin agonist or analog is chronically administered.

### BRIEF DESCRIPTION OF THE DRAWINGS

FIG. 1(A-B) is a graphical representation of the response of blood glucose levels to intraperitoneal injection of exendin agonists. Points represent mean ± standard deviation.
FIG. 2(A-C) is a graphical representation of the response of cardiac diastolic and systolic function following myocardial infarction (MI)-induced congestive heart failure (CHF) to chronic treatment with GLP-1 or an exendin agonist. The ratio of peak of early (E) vs late (A) filling waves (E/A) ratio and left atrial volume (LAV) represent cardiac diastolic function; left ventricular ejection fraction (LVEF) represents cardiac systolic function.
FIG. 3(A-B) is a graphical representation of the response of left ventricle chamber size following MI-induced CHF to chronic treatment with GLP-1 or an exendin agonist. Left ventricle chamber size is represented by left ventricle end diastolic dimension (LVEDD) and left ventricle end systolic dimension (LVESD).
FIG. 4(A-C) is a graphical representation of the response of fasting plasma insulin and glucose levels and insulin resistance following MI-induced CHF to chronic treatment with GLP-1 or an exendin agonist. Homeostasis Model Assessment (HOMA) is a major index for insulin resistance.
FIG. 5(A-C) is a graphical representation of the response of exercise capacity and efficiency following MI-induced CHF to chronic treatment with GLP-1 or an exendin agonist.
FIG. 6(A-C) is a graphical representation of the response of exercise capacity-to-peak lactate ratio, and baseline plasma lactate, following MI-induced CHF to chronic treatment with GLP-1 or an exendin agonist.
FIG. 7(A-B) is a graphical representation of the response of diastolic function following MI-induced CHF to treatment with GLP-1, captopril, or combination therapy with GLP-1 and captopril. E/A ratio is a measure of cardiac diastolic function.
FIG. 8(A-B) is a graphical representation of the response of cardiac contractility following MI-induced CHF to treatment with GLP-1, captopril, or combination therapy with GLP-1 and captopril. Fractional shortening percentage is a measure of cardiac contractility.
FIG. 9(A-D) is a graphical representation of the response of left ventricle chamber size following MI-induced CHF to treatment with GLP-1, captopril, or combination therapy with GLP-1, captopril, or combination therapy with GLP-1 and captopril. Left ventricle chamber size is represented by left ventricle end diastolic dimension (LVEDD) and left ventricle end systolic dimension (LVESD).
FIG. 10(A-B) is a graphical representation of the response of exercise capacity and efficiency following MI-induced CHF to treatment with GLP-1, captopril, or combination therapy with GLP-1 and captopril.
FIG. 11(A-B) is a graphical representation of the response of exercise capacity-to-peak lactate ratio, and baseline plasma lactate, following MI-induced CHF to treatment with GLP-1, captopril, or combination therapy with GLP-1 and captopril.
FIG. 12(A-B) is a graphical representation of the response of cardiac function and insulin resistance following MI-induced CHF to treatment with exendin agonists.

### DETAILED DESCRIPTION

The GLP-1 molecules or agonists and analogs thereof, and exendin molecules or agonists and analogs thereof, including their derivatives and active fragments, provided herein are useful in view of their pharmacological properties. Particular GLP-1 molecules or agonists and analogs thereof, and exendin molecules or agonists and analogs thereof are shown in Table 1. Activity as GLP-1 or exendin analogs or agonists can be indicated by activity in the assays described below. For example, effects of GLP-1 or exendin 1 agonists or analogs thereof on glucose lowering and reducing food intake can be identified, evaluated, or screened for, using the methods described in the examples below, or other methods known in the art. In Table 1, the double asterisks "**" indicate testing using GLP-1 CYCLASE(6-23).

| **Cmpd** | **SEQ ID NO.** | **SEQUENCE (amide or acid form as tested)** | **RBA GLP (RIN) IC₅₀ (nM)** | **CYCLASE GLP/GIP/C T (RIN) EC₅₀ (nM)** | **GLU LOWERING AUC240 @2nmol/kg** | **FOOD INTAKE at 60min** | **FOOD INTAKE dose** |
|---|---|---|---|---|---|---|---|
| 3521 | 1 | HAEGTFTSDVSSYLEGQAAKEFIAWLVKGR-NH2 | 0.034 | 0.162 | -9% @ 80nmol/kg | -27% | 1mg/kg |
| 3922 | 2 | HGEGTFTSDLSKQMEEEAVRLFIEWLKNGGPSSGAPPPS-NH2 | 0.5 | 0.2 | -22% | 0.2 | ED50nmol/kg |
| 4103 | 3 | HGEGTFTSDLSKQLEEEAVRLFIEWLKNGGPSSGAPPPS-NH2 | 0.6 | 0.3 | -13% @1nmol/kg, -11 to - 25% @2nmol/kg | 2 | ED50nmol/ kg |
| 4016 | 4 | HGDGTFTSDLSKQMEEEAVRLFIEWLKNGGPSSGAPPPS-NH2 | 0.65 | 0.54 | | 1 | ED50nmol/ kg |
| 4596 | 5 | HGEGTFTSELSKQMEEEAVRLFIEWLKNGGPSSGAPPPS-NH2 | 0.98 | 1000 | | | |
| 4597 | 6 | HGEGTFTSDLSKQMEEEAVRLFIEWLKQGGPSSGAPPPS-NH2 | 0.78 | 0.18 | | | |
| 4784 | 7 | HGEGTFTSDLSKQAEEEAVRLFIEWLKQGGPSSGAPPPS-NH2 | 0.91 | 0.49 | | -34% | |
| 4792 | 8 | HGEGTFTFDLSKQIEEEAVRLFIEWLKQGGPSSGAPPPS-NH2 | 0.67 | 0.74 | | | |
| 4793 | 9 | HGEGTFTSDLSKQLEEEAVRLFIEWLKQGGPSSGAPPPS-NH2 | 0.6 | 0.27 | | -51% | 10nmol/kg |
| 4855 | 10 | HGEGTFTTDLSKQLEEEAVRLFIEWLKQGGPSSGAPPPS-NH2 | 1.1 | 0.6 | | -46% | 10nmol/kg |
| 4856 | 11 | HGEGTFTSDFSKQLEEEAVRLFIEWLKQGGPSSGAPPPS-NH2 | 0.52 | 0.2 | | -52% | 10nmol/kg |
| 4958 | 12 | HGEGTPTSDLSKQLEEEAVRLFIEWLKQGGPSSGAPPPS-NH2 **[D (OMe)** | 0.27 | 0.18 | | -36% | 10nmol/kg |
| 4959 | 13 | HGEGTFTSDLSKQAEEEAVRLFIEWLKQGGPSSGAPPPS-NH2 **[D(QMe)]** | 0.83 | 6.9 | | | |
| 5084 | 14 | HGEGTFTSDLSKQLEEEAVRLFIEWLKQGGPSSGFPPPS-NH2 | 2.5 | 0.76 | | | |
| 5272 | 15 | HGEGTFTSDLSKQLEEBAVRLFIEWLKNGGPSSGFPPPS-NH2 | 0.46 | 0.107 | -21% | | |
| 5085 | 16 | HGEGTFTSDLSKQLEEEAVRLFIEWLKQGGPSSGLPPPS-NH2 | 5.5 | 2.04 | | | |
| 5086 | 17 | HGEGTFTSDLSKQLEEEAVRLFIEWLKQGGPSSGPPPPS-NH2 | 1.9 | 0.66/0.16 | -1% | -77% | 10nmol/kg |
| 5087 | 18 | HGEGTFTSDLSKQLEEEAVRLFIEMLKQGGPSSGTPPPS-NH2 | 6.5 | 3.53 | | | |
| 5088 | 19 | HGEGTFTSDLSKQLEEEAVRLFIEWLKQGGPSSGVPPPS-NH2 | 3 | 1.03 | | | |
| 5194 | 20 | HGEGTFTSDLSKQLEEEAVRLFIEWLKQGGPSRGRPPPS-NH2 | 0.28 | 0.1 | | | |
| 5112 | 21 | HGEGTFTSDLSKQLEEEAVRLFIEWLKQGGPSSGKPPPS-NH2 | 1.1 | 0.48/0.09 ** | -2% @1nmol/kg | -41% | 10nmol/kg |
| 5090 | 22 | HGEGTFTSNLSKQLEEEAVRLFIEWKQGGPSSGAPPPS-NH2 | 0.83 | 3.34 | | | |
| 5091 | 23 | HGEGTFTSDKSKQLEEEAVRLFIEWLKQGGPSSGAPPPS-NH2 | 0.93 | 0.32 | -12% @1nmol/kg | -31% | 10nmol/kg |
| 5092 | 24 | HGEGTFTSDWSKQLEEEAVRLFIEWLKQGGPSSGAPPPS-NH2 | 0.97 | 0.25 | -1% @1nmol/kg | -47% | 10nmol/kg |
| 5096 | 25 | HGEGTFTSDESKQLEEEAVRLFIEWLKQGGPSSGAPPPS-NH2 | 8.5 | 1.52 | | | |
| 5099 | 26 | HGEGTFTSDVTQQLEEEAVRLFIEWLKQGGPSSGAPPPS-NH2 | 5 | 1.29 | | | |
| 5100 | 27 | HGEGTFTSDLSKQLEEKAAKEFIEWLKQGGPSSGAPPPS-NH2 | 1.8 | 0.89/0.09/0.49 | -16% @1nmol/kg | -66% | 10nmol/kg |
| 5102 | 28 | HGEGTFTSDLSKQLEEKAVRLFIEWLKQGGPSSGAPPPS-NH2 | 0.45 | 0.42/0.24 | -11% | -53% | 10nmol/kg |
| 5452 | 29 | HGEGTFTSDLSKQLEEKAVRLFIEMLKNGGPSSGAPPPS-NH2 | | 0.062 | | | |
| 5128 | 30 | HGEGTYTNDLSKQLEEEAVRLFIEWLKQGGPSSGAPPPS-NH2 | 1.4 | 0.72/0.18 | -10% @1nmol/kg | -46% | 10nmol/kg |
| 5129 | 31 | HGEGTFTSDVTEYLEEEAVRLFIEWLKQGGPSSGAPPPS-NH2 | 3 | 1.09 | | | |
| 5130 | 32 | HGEGTYTNDVTEYLEEEAVRLFIEWLKQGGPSSGAPPPS-NH2 | 2.6 | 0.89/0.05 44** | -22% | | |
| 5271 | 33 | HGEGTYTNDVTEYLEEEAVRLFIENLKNGGPSSGAPPPS-NH2 | 1.5 | 0.138 | -17% | | |
| 5182 | 34 | HGEGTYTNDVSSYLEEEAARLFIEMLKQGGPSSGAPPPS-NH2 | 1.5 | 0.24 | -16% | | |
| 5183 | 35 | HGEGTYTNDVSSYLEGQAARLFIEWL_QGGPSSGAPPPS-NH2 | 0.31 | 0.21 | | | |
| 5195 | 36 | HGEGTFTSDLSKQLEERAVRLFIEWLKQGGPSSGAPPPS-NH2 | 0.14 | 0.1 | | | |
| 5196 | 37 | HGEGTFTSDLSKQKEEEEAVRLFTEWLKQGGPSSGAPPPS-NH2 | 0.43 | 0.1 | | | |
| 5270 | 38 | HGEGTFTSDLSKQLEEEAVRLFIEYLKNGGPSSGAPPPS-NH2 | 1.1 | 0.104 | | | |
| 5271 | 39 | HGEGTYTNDVTEYLEEEAVRLFIEWLKNGGPSSGAPPPS-NH2 | 1.5 | 0.138 | -17% | | |
| 5272 | 40 | HGEGTFTSDLSKQLEEEAVRLFIEWLKNGGPSSGFPPPS-NH2 | 0.46 | 0.107 | -21% | | |
| 5197 | 41 | HGEGTFTSDLSKQSEEEAVRLFIEWLKQGGPSSGAPPPS-NH2 | 1.3 | 0.14 | | | |
| 5452 | 42 | HGEGTFTSDLSKQLEEKAVRLFIEWLKNGGPSSGAPPPS-NH2 | | 0.062 | | | |
| 5450 | 43 | HGEGTFTSDLSKQMEEEVRLFIEWLKNGIPS-NH2 | | 0.133 | | | |
| 5529 | 44 | HGEGTFTSDLVKILEAEAVRKFIEFLKNGGP SSGAPPPS-NH2 | | 0.3877** | | | |
| 5530 | 45 | HGEGTFTSDLSKQMEEEAVRLFIEWGSWGIPS-NH2 | | 622.811** | | | |
| 5198 | 46 | HGEGTFTSDLSKQLEEEAVRLFIEWLKQ(OctG)GPSSGAPPPS-NH2 | 1.2 | 1.5/0.58 | -14% | | |
| 5199 | 47 | HGEGTFTSDLSKQLEEEAVRLFIEWLKQG(OctG)PSSGAPPPS-NH2 | 1.8 | 1/0.18 | -19% | | |
| 5200 | 48 | HGEGTFTSDLSKQLEEEAVRLFIEWLKQGGPSS(OctG)APPPS-NH2 | 0.53 | 0.33/0.15 | -22 and - 33% | | |
| 5264 | 49 | HGEGTFTSDLSKQAEEEAVRLFIEWLKNGGPSS(OctG)APPPS-NH2 | 0.58 | 0.171 | -24% | | |
| 5265 | 50 | HGEGTFTSDLSKQAEEEAVRLFIEFLKNGGPSS(OctG)APPPS-NH2 | 0.69 | 0.201 | | | |
| 5266 | 51 | HGEGTFTSDLSKQLEEEAVRLFIEWLKNGRPKK(OctG)RYS-OH | 0.14 | 0.829 | | | |
| 5267 | 52 | HGEGTFTSDLSKQLEEEAVRLFIEMLKNGGPSKE(OctG)IS-OH | 0.69 | 0.175 | -11% | | |
| 5268 | 53 | HGEGTFTSDLSKQAEEEAVRLFIEWLKNGKPKK(OctG)RYS-OH | 0.11 | 0.489 | -8% | | |
| 5269 | 54 | HGEGTFTSDLSKQLEEEAVRLFIEFLKNGKPKK(OctG)RYS-OH | 0.088 | 1.312 | -14% | | |
| 5391 | 55 | HGEGTFTSDLSKQLEEEAVRLFIENLKNGGPSS(OctG)APPPS-NH2 | 0.65 | 0.290 | -20% | | |
| 5097 | 56 | HGEGTFTSDLSKQLEEEAVRLFIEWLIQGGPSKEIIS-OH | 2.3 | 0.74/0.19 1** | | | |
| 5098 | 57 | HGEGTFTSDVTQQLEEEAVRLFIENLIQGGPSKEIIS-OH | 7.1 | 0.987/0.2 29** | | | |
| 5101 | 58 | HGEGTFTSDLSKQLEEKAAKEFIEWLIQGGPSKEIIS-OH | 2.9 | 0.8632 | -10% | | |
| 5103 | 59 | HGEGTFTSDLSKQLEEKAVRLFIEWLIQGGPSKEIIS-OH | 0.78 | 0.65/0.20 3 | -17% | -42% | 10nmol/kg |
| 5131 | 60 | HGEGTFTSDLSKQLEEEAVRLFIEWLKQGGPSKEIIS-OH | 1.1 | 0.31/0.09 4 | -29% | -49% | 10nmol/kg |
| 5526 | 61 | HGEGTFTSDLSKQLEEEAVRLFIEWLKQGGPSKEIIS-NN2 | | 0.0842** | | | |
| 5132 | 62 | HGEGTFTSDLSKQLEEEAVRLFIEWLIKGRP-OH | 0.58 | 1.295 | -6% | -51% | 10nmol/kg |
| 5185 | 63 | HGEGTFTSDLSKQLEEEAVRLFIEWLKQGKP-OH | 0.47 | 0.17 | | | |
| 5186 | 64 | HGEGTFTSDLSKQLEEEAVRLFIEWLIQGKP-OH | 0.3 | 0.26 | | | |
| 5227 | 65 | HGEGTFTSDLSKQLEEEAVRLFIEWLIKGKP-OH | | 0.629** | | | |
| 5184 | 66 | HGEGTFTSDLSKQLEEEAVRLFIEWLKQGKPKKIRYS-OH | 0.045 | 0.14 | -12% | | |
| 5294 | 67 | HGEGTFTSDLSKQLEEEAVRLFIEWLKNGGKPKKIRYS-OH | 0.08 | 0.09** | 8% | | |
| 5295 | 68 | HGEGTFTSDLSKQLEEEAVRLFIEWLKNGKPGKGKIRYS-OH | 0.068 | 0.089** | 7% | | |
| 5296 | 69 | HGEGTFTSDLSKQLEEEAVRLFIEWLKNPGGKEIIS-OH | 4 | 0.145 | | | |
| 5297 | 70 | HGEGTFTSDLSKQLEEEAVRLFIEWLKNGGPGGKEIIS-OH | 2.5 | 0.097 | -19% | | |
| 5439 | 71 | HGEGTFTSDLSKQLEEEAVRLFIEWLKNGKPKKIRYA-OH | | 0.17** | | | |
| 5440 | 72 | HGEGTFTSDLSKQLESFAVRLFIEWLKNGKPKKIAYS-OH | | 0.13** | | | |
| 5441 | 73 | HGEGTFTSDLSKQLEEEAVRLFIEWLKNGKPKKARYS-OH | | 0.128** | | | |
| 5442 | 74 | HGEGTFTSDLSKQLEEEAVRLFIEWLKNGKPKAIRYS-OH | | 0.1** | | | |
| 5443 | 75 | HGEGTFTSDLSKQLEEEAVRLFIEWLKNGKPAKIRYS-OH | | 0.08** | | | |
| 5444 | 76 | HGEGTFTSDLSKQLEEEAVRLFIEWLKNGGPSKEIIA-OH | | 0.09** | | | |
| 5445 | 77 | HGEGTFTSDLSKQLEEEAVRLFIEWLKNGGPSKEIAS-OH | | 0.09** | | | |
| 5446 | 78 | HGEGTFTSDLSKQLEEEAVRLFIEWLKNGGPSKAIIS-OH | | 0.1** | | | |
| 5451 | 79 | HGEGTFTSDLSKQLEEEAVRLFIEWLKNGKPGGKKIRYS-OH | | 0.205 | | | |
| 4844 | 80 | HaEGTFTSDVSSYLEGQAAKEFIAWLVKGRGGG(DPP4I1)-NH2 GLP-1-(7-37)-GG-(S)-2,6-diamino-1-(thiazolidin-3-yl)hexan-1-one amide | 0.087 | 0.13 | | | |
| 4845 | 81 | HAEGTFTSDVSSYLEGQAAKEFIAWLVKGRGGG(DPP4I2)-NH2 GLP-1-(7-37)-GG-(S)-2,6-diamino-1-(thiazolidin-2-cyano-3-yl)hexan-1-one amide | 0.1 | 0.4 | | | |
| 4887 | 82 | HAEGTFTSDVSSYLEGQAAKEFIAWLVKGRGGGGIPI-NH2 | 0.17 | 0.22 | | | |
| 4888 | 83 | HAEGTFTSDVSSYLEGQAAKEFIAWLVKGRPSGGGIPI-NH2 | 0.13 | 0.17 | | | |
| 4957 | 84 | HAEGTFTSDVSSYLEGQAAKEFIAWLVKGRGGGIPI-NH2 | 0.1 | 0.51 | | | |
| 4983 | 85 | (VPI1)-HAEGTFTSDVSSYLEGQAAKEFIAWLVKGR-NH2 (R,S)-1-[N2-(1-carboxy-3-phenylpropyl)-L-ala]-L-pro-ado-GLP-1-(7-36) amide | 13 | 239 | | | |
| 4984 | 86 | (VPI1)-HAEGTFTSDVSSYLEGQAAKEFIAWLVKGR-NH2 (R,S)-1-[N2-(1-carboxy-3-phenylpropyl)-L-ala]-L-pro-ado-ado-GLP-1-(7-36) amide | 8.1 | 135 | | | |
| 5201 | 87 | HAHGEGTFTSDLSKQLEEEAVRLFIEWLKQGGPSSGAPPPS-NH2 | 6.1 | 10000/8.6 9** | | | |
| 5202 | 88 | HAEHGEGTFTSDLSKQLEEEAVRLFIEMLKQGGPSSGAPPPS-NH2 | 7.9 | 10000/10 67** | | | |
| 5203 | 89 | HAEGHGEGTFTSDLSKQLEEEAVRLFIEWLKQGGPSSGAPPPS-NH2 | 8.8 | 10000/5.7 1** | | | |
| 5292 | 90 | YAHGEGTFTSDLSKQLEEEAVRLFIEGVLKNGGPSSGAPPPS-NH2 | 3.6 | 2.45/7.4* * | | | |
| 5293 | 91 | HSHGEGTFTSDLSKQLEEEAVRLFIEWLKNGGPSSGAPPPS-NH2 | 10 | 1.42**,8. 13** | | | |
| 5337 | 92 | YPHGEGTFTSDLSKQLEEEAVRLFIEWLKNGGPSSGAPPPS-NH2 | 2.6 | 0.79/0.92 ** | | | |
| 4992 | 93 | Ado-HAEGTFTSDVSSYLEGQAAKEFIAWLVKGR-NH2 | 13 | 15 | | | |
| 5447 | 94 | HAHGEGTFTSDLSKQLEEEAVRLFIEWLKNGGPSS(OctG)APPPS -NH2 | | 0.64**/8. 83** | | | |
| 5540 | 95 | HAHAHAHGEGTFTSDLSKQMEEEAVRLFIEWLKNGGPSSGAPPPS-NH2 | | | | | |
| 4106 | 96 | HGEGTFTSDLSKQLEEEAVRLFTEFLKN-NH2 | 0.6 | 1.4 | | -47% | 63µg/kg |
| 4828 | 97 | HGEGTFTSDLSKQLEEEAVRLFIEWLKN-NH2 | 0.47 | 1.1 | | -63% | 10nmol/kg |
| 5035 | 98 | HGEGTFTSDLSKQVQEEAVRLFVEFLKN-NH2 | 181 | 628 | | | |
| 5052 | 99 | HGEGTFTSDLVKILEAEAVRKFIEFLKN-NH2 | 0.56 | 4.5045 | | | |

In accordance with the present disclosure and as used herein, the following terms are defined to have the following meanings, unless explicitly stated otherwise.

The term "amino acid" refers to natural amino acids, unnatural amino acids, and amino acid analogs, all in their D and L stereoisomers if their structures allow such stereoisomeric forms. Natural amino acids include alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or 1), leucine (Leu or L), Lysine (Lys or K), methionine (Met or M), phenylalanine (Phe or F), proline (Pro or P), serine (Ser or S), threonine (Thr or T), tryptophan (Trp or W), tyrosine (Tyr or Y) and valine (Val or V). Unnatural amino acids include, but are not limited to azetidinecarboxylic acid, 2-aminoadipic acid, 3-aminoadipic acid, beta-alanine, aminopropionic acid, 2-aminobutyric acid, 4-aminobutyric acid, 6-aminocaproic acid, 2-aminoheptanoic acid, 2-aminoisobutyric acid, 3-aminoisbutyric acid, 2-aminopimelic acid, tertiary-butylglycine, 2,4-diaminoisobutyric acid, desmosine, 2,2'-diaminopimelic acid, 2,3-diaminopropionic acid, N-ethylglycine, N-ethylasparagine, homoproline, hydroxylysine, allo-hydroxylysine, 3-hydroxyproline, 4-hydroxyproline, isodesmosine, allo-isoleucine, N-methylalanine, N-methylglycine, N-methylisoleucine, N-methylpentylglycine. N-methylvaline, naphthalanine, norvaline, norleucine, octylglycine, ornithine, pentylglycine, pipecolic acid and thioproline. Amino acid analogs include the natural and unnatural amino acids which are chemically blocked, reversibly or irreversibly, or modified on their N-termninal amino group or their side-chain groups, as for example, methionine sulfoxide, methionine sulfone, S-(carboxymethyl)-cysteine, S-(carboxymethyl)-cysteine sulfoxide and S-(carboxymethyl)-eysteine sulfone.

The term "amino acid analog" refers to an amino acid where either the C-terminal carboxy group, the N-terminal amino group or side-chain functional group has been chemically modified to another functional group. For example, aspartic acid-(beta-methyl ester) is an amino acid analog of aspartic acid. N-ethylglycine is an amino acid analog of glycine; or alanine carboxamide is an amino acid analog of alanine.

"Alkyl" as used herein means a straight or branched aliphatic hydrocarbon group. Non limiting examples of substituents are straight or branched alkyl such as methyl, ethyl, propyl, isopropyl, butyl, isobutyl, sec-butyl, pentyl, hexyl, and alkenyl, hydroxyl, alkoxy, amino, halo.

The term "lower" referred to herein in connection with organic radicals such as alkyl groups defines such groups with up to and including about 6, up to and including 4 or one or two carbon atoms. Such groups may be straight chain or branched chain.

"Pharmaceutically acceptable salt" includes salts of the compounds described herein derived from the combination of such compounds and an organic or inorganic acid. In practice the use of the salt form amounts to use of the base form. The compounds are useful in both free base and salt form.

In addition, the following abbreviations stand for the following:
"CAN" or "CH₃CN" refers to acetonitrile.
"Ado" refers to 8-amino 3,6 dioxaoctanoic acid.
"Boc", "tBoc" or "Tboc" refers to t-butoxy carbonyl.
"DCC" refers to N,N'-dicyclohexylcarbodiimide.
"D(OMe)" or "Asp(OMe)" refers to the *O*⁴-methyl ester of aspartate.
"Fmoc" refers to fluorenylmethoxycarbonyl.
"HBTU" refers to 2-(1H-benzotriazol-1-yl)-1,1,3,3,-tetramethyluronium hexaflurophosphate.
"HOBt" refers to 1-hydroxybenzotriazole monohydrate.
"homoP" or "hPro" refers to homoproline.
"MeAla" or "Nime" refers to N-methylalanine.
"naph" refers to naphthylalanine.
"pG" or "pGly" refers to pentylglycine.
"tBuG" refers to tertiary-butylglycine.
"ThioP" or "tPro" refers to thioproline.
"3Hyp" refers to 3-hydroxyproline.
"4Hyp" refers to 4-hydroxyproline.
"NAG" refers to N-alkylglycine.
"NAPG" refers to N-alkylpentylglycine.
"Norval" refers to norvaline.
"Norleu" refers to norleucine.
"OctG" refers to octylglycine.

The analog polypeptides disclosed herein may also be further derivatized by chemical alterations such as amidation, glycosylation, acylation, sulfation, phosphorylation, acetylation, and cyclization. Such chemical alterations may be obtained through chemical or biochemical methodologies, as well as through in-vivo processes, or any combination thereof. Derivatives of the analog polypeptides of the invention may also include conjugation to one or more polymers or small molecule substituents. One type of polymer conjugation is linkage or attachment of polyamino acids (e.g., poly-his, poly-arg, poly-lys, etc.) and/or fatty acid chains of various lengths to the N- or C-terminus or amino acid residue side chains of an exendin or GLP-1 analog. Small molecule substituents include short alkyls and constrained alkyls (e.g., branched, cyclic, fused, adamantyl), and aromatic groups.

Agonist refers to a molecule that has an affinity for a receptor associated with the reference molecule and stimulates at least one activity associated with the reference molecule binding to that receptor. For example, and without limitation, a GLP-1 agonist binds to a receptor that also binds GLP-1 and as a result of the agonist binding stimulates at least one activity associated with the binding of CrLP-1 to the same receptor.

GLP-1 molecules or agonists and analogs thereof, and exendin molecules or agonists and analogs thereof may be coupled to polyethylene glycol (PEG) by one of several strategies. *See,* Int. J. Hematology, 68:1 (1998); Bioconjugate Chem., 6:150 (1995); and Crit. Rev. Therap. Drug Carrier Sys., 9:249 (1992) all of which are incorporated herein by reference in their entirety. Those skilled in the art, therefore, will be able to utilize such well-known techniques for linking one or more polyethylene glycol polymers to the exendins and exendin agonists or analogs, or GLP-1 and GLP-1 agonists or analogs, described herein. Suitable polyethylene glycol polymers typically are commercially available or may be made by techniques well know to those skilled in the art. The polyethylene glycol polymers preferably have molecular weights between 500 and 20,000 and may be branched or straight chain polymers. In other embodiments, the GLP-1 molecules or agonists or analogs thereof, and exendin molecules or agonists or analogs thereof are modified by the addition of polyamide chains of precise lengths as described in U.S. Patent No. 6,552,167 which is incorporated by reference in its entirety. In yet other embodiments, the GLP-1 molecules or agonists or analogs thereof, and exendin molecules or agonists or analogs thereof are modified by the addition of alkylPEG moieties as described in U.S. Patent Nos. 5,359,030 and 5,681,811 and which are incorporated by reference in its entirety.

The attachment of a PEG on an intact peptide or protein can be accomplished by coupling to amino, carboxyl or thiol groups. These groups will typically be the N and C termini and on the side chains of such naturally occurring amino acids as lysine, aspartic acid, glutamic acid and cysteine. Since the compounds of the present disclosure can be prepared by solid phase peptide chemistry techniques, a variety of moieties containing diamino and dicarboxylic groups with orthogonal protecting groups can be introduced for conjugation to PEG.

The present disclosure also provides for conjugation of a GLP-1 molecule or agonist or analog thereof, or exendin molecule or agonist or analog thereof, to one or more polymers other than polyethylene glycol which can regulate kidney clearance in a manner similar to polyethylene glycol. Examples of such polymers include albumin and gelatin. *See,* Gombotz and Pettit, Bioconjugate Chem., 6:332-351, 1995, which is incorporated herein by reference in its entirety. In one aspect, conjugates to immunoglobulins are encompassed within the scope of the invention, , e.g., monoclonal antibody, catalytic antibody such as aldolase catalytic antibody human MAb 38C2, or their fragments, e.g. Fc. Polymers and methods can be found for example in United States Published Application US20030175921A1 and publication WO2002/046227, which are incorporated by reference in their entirety.

One embodiment relates to exendin and GLP-1 analogs comprising one or more substitutions with a modified amino acid comprising a C₁-C₂₀ alkyl side chain, which, in an embodiment is an octyl chain. In one embodiment, the amino acid that is modified with an octyl chain is a glycine. In another embodiment, a leucine residue is replaced by an octylglycine residue. In another embodiment, an alanine residue is replaced by an octylglycine residue. Without being limited by theory, it is believed that incorporation of one or more octyl chains increases the half life of an analog, perhaps because the analog binds more efficiently to circulating plasma proteins. Again not limited by theory, incorporation of one or more octyl chains may slow clearance of the analog by the kidney. It has been reported that GLP-1 analogs having octyglycine substitutions at certain positions may possess an extended duration of action. *See, e.g.,* WO 2005/066207.

In one embodiment, a GLP-1 molecule or agonist or analog thereof, or exendin molecule or agonist or analog thereof of the present disclosure comprises an alkylglycine comprising a C₅₋₉ straight or branched alkyl side chain, or a cycloalkyl group. In one embodiment, a GLP-1 molecule or agonist or analog thereof, or exendin molecule or agonist or analog thereof comprises an alkylglycine comprising a C₆₋₈ straight or branched alkyl side chain. In another embodiment, a GLP-1 molecule or agonist or analog thereof, or exendin molecule or agonist or analog thereof comprises an octyl glycine comprising a C₈ straight alkyl side chain.

In one embodiment, a GLP-1 molecule or agonist or analog thereof, or exendin molecule or agonist or analog thereof of the present disclosure comprises an octylglycine at position 14. In one embodiment is provided a polypeptide comprising the amino acid sequence HGEGTFTSDLSKQ[OctG]EEEAVRLFIEWLKQGGPSSGAPPPS (SEQ ID NO: 100) or its amide form.

In other embodiments, a GLP-1 molecule or agonist or analog thereof, or exendin molecule or agonist or analog thereof comprises an octylglycine at position 29, 30, 34, or 35.

Compounds such as the GLP-1 molecules or agonists and analogs thereof, and exendin molecules or agonists and analogs thereof described herein may be prepared using standard solid-phase peptide synthesis techniques, for example, an automated or semiautomated peptide synthesizer. Typically, using such techniques, an α-N-carbamoyl protected amino acid and an amino acid attached to the growing peptide chain on a resin are coupled at room temperature in an inert solvent such as dimethylformamide, N-methylpyrrolidinone or methylene chloride in the presence of coupling agents such as dicyclohexylcarbodiimide and 1-hydroxybenzotriazole in the presence of a base such as diisopropylethylamine. The α-N-carbamoyl protecting group is removed from the resulting peptide-resin using a reagent such as trifluoroacetic acid or piperidine, and the coupling reaction repeated with the next desired N-protected amino acid to be added to the peptide chain. Suitable N-protecting groups are well known in the art, with t-butyloxycarbonyl (tBoc) and fluorenylmethoxycarbonyl (Fmoc) being typically used.

The solvents, amino acid derivatives and 4-methylbenzhydryl-amine resin used in the peptide synthesizer may be purchased from Applied Biosystems Inc. (Foster City, Calif.). The following side-chain protected amino acids may be purchased from Applied Biosystems, Inc.: Boc-Arg(Mts), Fmoc-Arg(Pmc), Boc-Thr(Bzl), Fmoc-Thr(t-Bu), Boc-Ser(Bzl), Fmoc-Ser(t-Bu), Boc-Tyr(BrZ), Fmoc-Tyr(t-Bu), Boc-Lys(Cl-Z), Fmoc-Lys(Boc), Boc-Glu(Bzl), Fmoc-Glu(t-Bu), Fmoc-His(Trt), Fmoc-Asn(Trt), and Fmoc-Gln(Trt). Boc-His(BOM) may be purchased from Applied Biosystems, Inc. or Bachem Inc. (Torrance, Calif.). Anisole, dimethylsulfide, phenol, ethanedithiol, and thioanisole may be obtained from Aldrich Chemical Company (Milwaukee, Wis.). Air Products and Chemicals (Allentown, Pa.) supplies HF. Ethyl ether, acetic acid and methanol may be purchased from Fisher Scientific (Pittsburgh, Pa.).

Solid phase peptide synthesis may be carried out with an automatic peptide synthesizer (Model 430A, Applied Biosystems Inc., Foster City. Calif.) using the NMP/HOBt (Option 1) system and tBoc or Fmoc chemistry (see, Applied Biosystems User's Manual for the ABI 430A Peptide Synthesizer, Version 1.3B Jul. 1, 1988, section 6, pp. 49-70, Applied Biosystems, Inc., Foster City, Calif.) with capping. Boc-peptide-resins may be cleaved with HF (-5°C. to 0°C., 1 hour). The peptide may be extracted from the resin with alternating water and acetic acid, and the filtrates lyophilized. The Fmoc-peptide resins may be cleaved according to standard methods (Introduction to Cleavage Techniques, Applied Biosystems, Inc., 1990, pp. 6-12). Peptides may be also be assembled using an Advanced Chem Tech Synthesizer (Model MPS 350, Louisville. Ky.).

Peptides may be purified by RP-HPLC (preparative and analytical) using a Waters Delta Prep 3000 system. A C4, C8 or C18 preparative column (10 *µ*, 2.2x25 cm; Vydac, Hesperia, Calif.) may be used to isolate peptides, and purity may be determined using a C4, C8 or C18 analytical column (5*µ*, 0.46x25 cm; Vydac). Solvents (A=0.1% TFA/water and B=0.1% TFA/CH₃CN) may be delivered to the analytical column at a flowrate of 1.0 ml/min and to the preparative column at 15 ml/min. Amino acid analyses may be performed on the Waters Pico Tag system and processed using the Maxima program. Peptides may be hydrolyzed by vapor-phase acid hydrolysis (115°C., 20-24 h). Hydrolysates may be derivatized and analyzed by standard methods (Cohen, et al., The Pico Tag Method: A Manual of Advanced Techniques for Amino Acid Analysis, pp. 11-52, Millipore Corporation, Milford, Mass. (1989)). Fast atom bombardment analysis may be carried out by M-Scan, Incorporated (West Chester, Pa.). Mass calibration may be performed using cesium iodide or cesium iodide/glycerol. Plasma desorption ionization analysis using time of flight detection may be carried out on an Applied Biosystems Bio-Ion 20 mass spectrometer. Electrospray mass spectroscopy may be carried out on a VG-Trio machine.

Peptide compounds may also be prepared using recombinant DNA techniques, using methods now known in the art. *See, e.g.,* Sambrook et al., Molecular Cloning: A Laboratory Manual, 2d Ed., Cold Spring Harbor (1989) and United States 'Patent Application Publication 20050260701 filed November 24, 2004 which is incorporated by reference. Other compounds useful in the present disclosure may be prepared by art-known methods. For example, phosphate-containing amino acids and peptides containing such amino acids, may be prepared using methods known in the art. *See,* e.g., Bartlett and Landen, Biorg. Chem. 14:356-377 (1986).

Exendin or GLP-1 agonists, analogs or derivatives, and in particular, those contained in Table 1, are included within the methods described herein. Analogs or derivatives are functional variants of an exendin or to GLP-1 having similar amino acid sequence and retaining, to some extent, the receptor binding, glucose lowering or other activities of the related exendin or GLP-1 or agonists thereto. By a functional variant is meant the derivative has an activity that can be substituted for one or more activities of a particular exendin or GLP-1 or an agonist thereto. In one embodiment, functional variants retain all of the activities of a particular exendin or GLP-1 or an agonist thereto, however, the functional variant may have an activity that, when measured quantitatively, is stronger or weaker, as measured in functional assays, for example, such as those disclosed herein. Exemplary functional variants have activities that are within about 1% to about 10,000% of the activity of the related exendin, GLP-1, or agonist or analog thereof, between about 10% to about 1000%, and within about 50% to about 500%. Functional variants, such as derivatives or analogs, have at least about 50% sequence similarity, about 70%, about 90%, or about 95% sequence identity to the related exendin or GLP-1, or agonist or analog thereto. In some embodiments the functional variants have not more that 10 amino acid substitutions, deletions or additions as compared to the reference molecule. In other embodiments, the functional variants have not more than 5 amino acid substitutions, deletions or additions. In one embodiment the analog, derivative or functional variant is any of the novel peptides disclosed herein, for example those contained in Table 1.

Sequence similarity or identity can be readily calculated by known methods including, but not limited to, those described in Computational Molecular Biology, Lesk, ed., Oxford University Press, New York 1988; Biocomputing: Informatics and Genome Projects, Smith, ed., Academic Press, New York 1993; Computer Analysis of Sequence Data, Part I, Griffin and Griffin, eds., Humana Press, New Jersey 1994; Sequence Analysis in Molecular Biology, von Heinje, Academic Press 1987; Sequence Analysis Primer, Gribskov and Devereux, eds., Stockton Press, New York 1991; and Carillo and Lipman, STAM J. Applied Math, 48:1073 1988.

Publicly available computer programs which can be used to determine sequence similarity or identity between two sequences include, but are not limited to, GCG; a suite of five BLAST programs, three designed for nucleotide sequences queries (BLASTN, BLASTX, and TBLASTX) and two designed for protein sequence queries (BLASTP and TBLASTN). The BLASTX program is publicly available from NCBI and other sources, *e.g.,* BLAST Manual, Altschul et al., NCBI NLM NIH, Bethesda, MD 20894; Altschul et al., J. Mol. Biol. 215:403-410 (1990). The well-known Smith Waterman algorithm can also be used to determine identity.

Parameters for polypeptide sequence comparison typically include the following: Algorithm: Needleman and Wunsch, J. Mol. Biol. 48:443-453 (1970); Comparison matrix: BLOSSUM62 from Hentikoff and Hentikoff, Proc. Natl. Acad. Sci. USA 89:10915-10919 (1992); Gap Penalty: 12; Gap Length Penalty: 4. A program that can be used with these parameters is publicly available as the "gap" program from Genetics Computer Group ("GCG"), Madison, Wisconsin. The above parameters along with no penalty for end gap are the default parameters for peptide comparisons.

Parameters for nucleic acid molecule sequence comparison include the following: Algorithm: Needleman and Wunsch, J. Mol. Bio. 48:443-453 (1970); Comparison matrix: matches - +10; mismatches = 0; Gap Penalty: 50; Gap Length Penalty: 3. As used herein, "percent identity" is determined using the above parameters as the default parameters for nucleic acid molecule sequence comparisons and the "gap" program from GCG, version 10.2.

The ability of the derivative to retain some activity can be measured using techniques described herein. Derivatives include modification occurring during or after translation, for example, by phosphorylation, glycosylation, crosslinking, acylation, proteolytic cleavage, linkage to an antibody molecule, membrane molecule or other ligand (*see* Ferguson et al., Annu. Rev. Biochem., 57:285-320, 1988).

Derivatives can be produced using standard chemical techniques and recombinant nucleic acid molecule techniques. Modifications to a specific polypeptide may be deliberate, as through site-directed mutagenesis and amino acid substitution during solid-phase synthesis, or may be accidental such as through mutations in hosts which produce the polypeptide. Polypeptides including derivatives can be obtained using standard techniques such as those described in Sambrook, et al., Molecular Cloning, Cold Spring Harbor Laboratory Press (1989).

In an embodiment, a GLP-1 molecule or agonist or analog thereof may have a length of 27, 28, 29, 30, 31, 32, 33, 34, 35, 36, 37, 38, 39, 40, 41, 42, 43, 44, or 45 amino acid residues. In an embodiment, an exendin molecule or agonist or analog thereof may have a length of 20, 21, 22, 23, 24, 25, 26, 27, 28, 29, 30, 31, 32, 33, 34, 35, 36, 37, 38, 39, 40, 41, 42, 43, 44, or 45 amino acid residues. Accordingly, exendin analogs or their active fragments can have, for example, amino acids 1-27, 1-28, 1-29 or 1-30 (in which the 9 amino acid C-terminal "tail" found in exendin-4 is absent). For example, polypeptides useful in the compositions and methods herein can comprise the 1-30 fragment of compound 4016: HGDGTFTSDLSKQMEEEAVRLFIEWLKNGG or its amide form compound 4016(1-30)-NH2. In an embodiment, the nine amino acid C-terminal tail is truncated, substituted or derivatized, which can improve peptide solubility.

In yet another embodiment, a GLP-1 molecule agonist may be a small molecule which binds or activates a GLP-1 receptor, and may be synthesized in any manner known in the art.

In another embodiment, the use of DPP IV inhibitors to decrease or eliminate the inactivation of a GLP-1 molecule or agonist or analog thereof, or exendin molecule or agonist or analog thereof is also contemplated. DPP IV inhibitors can be administered alone or in combination with a GLP-1 molecule or agonist or analog thereof, or exendin molecule or agonist or analog thereof. As such, it is contemplated that active GLP-1 molecules or exendin molecules may be increased by the inhibition of DPP IV. Inhibitors of DPP IV are known to the skilled artisan and include, by way of non-limiting example, 2-cyanopyrrolidines, tetrahydroisoquinoline 3-carboxamide derivatives, fluorinated cyclic amides, adamantylglycine-based inhibitors, and glycinenitrile-based inhibitors. *See e.g.,* Fukushima, H., et al., Bioorg. Med. Chem. Lett., 14(22): 6053-6061 (2004). Non-limiting exemplary DPP IV inhibitors include valine-pyrrolidide (Marguet, D., et al., Proc. Natl. Acad. Sci. USA, 97(12): 6874-6879 (2000)), isoleucine thiazolidide (Pederson, R. A., et al., Diabetes, 47: 1253-1258 (1998), and NVP-DPP728 (Balkan, B., et al., Diabetologia, 42(11): 1324-1331 (1999)). DPP IV inhibitors including ketopyrrolidines and ketoazetidines have been discussed in the literature (Ferraris, D., et al., Bioorg. Med. Chem. Lett., 14(22): 5579-5583 (2004)). Examples of DPP-IV inhibitors suitable for use herein include those disclosed in U.S. Pat. Nos. 6,011,155, 6,124,305, 6,166,063, 6,432,969, 6,172,081, 6,710,040, 6,869,947, 6,995,183 and 6,995,180. Metformin and pioglitazone have been proposed to reduce DPP IV activity *in vivo.* (Kenhard, J.M., et al., Biochem. Biophys. Res. Commun., 324(1):92-97 (2004). Literature reports further describe optimization of a proline-derived homophenylalanine 3 to produce a potent DPP IV inhibitor. *See* Edmondson, S.D., et al, , Bioorg. Med. Chem. Lett., 14(20): 5151-5155 (2004).

The compounds described herein may form salts with various inorganic and organic acids and bases. Such salts include salts prepared with organic and inorganic acids, for example, HCl, HBr, H₂SO₄, H₃PO₄, trifluoroacetic acid, acetic acid, formic acid, methanesulfonic acid, toluenesulfonic acid, maleic acid, fumaric acid and camphorsulfonic acid. Salts prepared with bases include ammonium salts, alkali metal salts, *e.g.* sodium and potassium salts, alkali earth salts, e.g. calcium and magnesium salts, and zinc salts. The salts may be formed by conventional means, as by reacting the free acid or base forms of the product with one or more equivalents of the appropriate base or acid in a solvent or medium in which the salt is insoluble, or in a solvent such as water which is then removed in vacuo or by freeze-drying or by exchanging the ions of an existing salt for another ion on a suitable ion exchange resin.

The claimed compositions can also be formulated as pharmaceutically acceptable salts (e.g., acid addition salts) and/or complexes thereof. Pharmaceutically acceptable salts are non-toxic salts at the concentration at which they are administered. The preparation of such salts can facilitate the pharmacological use by altering the physical-chemical characteristics of the composition without preventing the composition from exerting its physiological effect. Examples of useful alterations in physical properties include lowering the melting point to facilitate transmucosal administration and increasing the solubility to facilitate the administration of higher concentrations of the drug.

Pharmaceutically acceptable salts include acid addition salts such as those containing sulfate, hydrochloride, phosphate, sulfamate, acetate, citrate, lactate, tartrate, succinate, oxalate, methanesulfonate, ethanesulfonate, benzenesulfonate, p-toluenesulfonate, cyclohexylsulfamate and quinate. Pharmaceutically acceptable salts can be obtained from acids such as hydrochloric acid, sulfuric acid, phosphoric acid, sulfamic acid, acetic acid, citric acid, lactic acid, tartaric acid, malonic acid, methanesulfonic acid, ethanesulfonic acid, benzenesulfonic acid, p-toluenesulfonic acid, cyclohexylsulfamic acid, and quinic acid. Such salts may be prepared by, for example, reacting the free acid or base forms of the product with one or more equivalents of the appropriate base or acid in a solvent or medium in which the salt is insoluble, or in a solvent such as water which is then removed *in vacuo* or by freeze-drying or by exchanging the ions of an existing salt for another ion on a suitable ion exchange resin.

Although the compounds are described herein in their acid or amide form, it should be appreciated that both the acid and amide forms of each molecule is contemplated.

The compounds described herein are useful in view of their pharmacological properties. In particular, the compounds possess activity as agents to treat congestive heart failure. The compounds also possess activity as agents for the treatment of diabetes mellitus, including Type I and II diabetes, in the treatment of disorders which would be benefited by agents which lower plasma glucose levels, for the prevention of hyperglycemia, for the prevention of hypertension, and for the treatment of disorders which would be benefited by the administration of agents useful in delaying and/or slowing gastric emptying. The compounds of the invention also possess activity as agents for the reduction of food intake, the suppression of appetite and the treatment of obesity.

The term "congestive heart failure" means an impaired cardiac function that renders the heart unable to maintain the normal blood output at rest or with exercise, or to maintain a normal cardiac output in the setting of normal cardiac filling pressure. A left ventricular ejection fraction of about 40% or less is indicative of congestive heart failure (by way of comparison, an ejection fraction of about 60% percent is normal). Patients in congestive heart failure display well-known clinical symptoms and signs, such as tachypnea, pleural effusions, fatigue at rest or with exercise, contractile dysfunction, and edema. Congestive heart failure is readily diagnosed by well known methods (*see, e.g.,* "Consensus recommendations for the management of chronic heart failure," Am. J. Cardiol., 83(2A):1A-38-A, 1999). A subject may be at risk for congestive heart failure if that individual smokes, is obese, has been or will be exposed to a cardiotoxic compound such as an anthracycline antibiotic, or has or had hypertension, ischemic heart disease, a myocardial infarct, a genetic defect known to increase the risk of heart failure, a family history of heart failure, myocardial hypertrophy, hypertrophic cardiomyopathy, left ventricular systolic dysfunction, coronary bypass surgery, diabetes, vascular disease, atherosclerosis, alcoholism, periocarditis, a viral infection, gingivitis, or an eating disorder (e.g., anorexia nervosa or bulimia), or is an alcoholic or cocaine addict.

While "obesity" is generally defined as a body mass index over 3 0, for purposes of this disclosure, any subject, including those with a body mass index of less than 30, who needs or wishes to reduce body weight is included in the scope of "obese."

In accordance with the methods of the present disclosure, the GLP-1 molecules or agonists or analogs thereof, or exendin molecules or agonists or analogs thereof, may be administered in any manner known in the art that renders such molecules biologically available to the subject, cell, population of cells or tissue in an effective amount. For example, the GLP-1 molecule or agonist or analog thereof, or exendin molecule or agonist or analogs thereof, may be administered to a subject *via* any central or peripheral route known in the art including, but not limited to: oral, parenteral, transdermal, transmucosal, or pulmonary routes. In one embodiment, administration is parenteral. In one embodiment, the mode of administration of said GLP-1 molecule or agonist or analog thereof, or exendin molecule or agonist or analog thereof, is by peripheral (subcutaneous or intravenous) administration. A particular route of administration is subcutaneous. In other aspects, said peripheral administration is selected from the group consisting of buccal, nasal, pulmonary, oral, intraocular, rectal, and transdermal administration. Further, the GLP-1 molecules or agonists or analogs thereof, or exendin molecules or agonists or analogs thereof, can be administered to a cell, group of cells, or tissue *via* pouring, pipetting, immersing, injecting, infusing, perfusing, or any other means known in the art. Determination of the appropriate administration method is usually made upon consideration of the condition (*e.g.,* disease or disorder) to be treated, the stage of the condition (*e.g.,* disease or disorder), the comfort of the subject, and other factors known to those of skill in the art.

Administration by the methods disclosed herein can be intermittent or continuous, both on an acute and/or chronic basis. In one embodiment, administration of a GLP-1 molecule or agonist or analog thereof, or exendin molecule or agonist or analog thereof, is continuous. Continuous intravenous or subcutaneous infusion, and continuous transcutaneous infusion are exemplary embodiments of administration for use in the methods disclosure. Subcutaneous infusions, both acute and chronic, are particularly preferred embodiments of administration.

In one aspect, an exendin or exendin agonist or analog is administered subcutaneously. In one embodiment, about 1 microgram to about 20 mg of the exendin or exendin agonist or analog is administered per dose. In another embodiment, about 30 micrograms to about 10 mg, or about 300 micrograms to about 5 mg of the exendin or exendin agonist or analog is administered per dose. In still another embodiment, about 30 micrograms to about 1 mg of the exendin or exendin agonist or analog is administered per dose.

In one aspect, GLP-1 or a GLP-1 agonist or analog is administered subcutaneously or intravenously, for example, at about 1 microgram to about 20 mg of GLP-1 or GLP-1 agonist or analog per dose. In one embodiment, about 30 micrograms to about 10 mg, or about 300 micrograms to about 5 mg of GLP-1 or GLP-1 agonist or analog is administered per dose. In another embodiment, about 30 microgram to about 1 mg of GLP-1 or GLP-1 agonist or analog is administered per dose.

As mentioned above, the GLP-1 molecule or agonist or analog thereof, or exendin molecule or agonist or analog thereof, may be administered on an acute or chronic basis. An acute administration includes a temporary administration for a period of time before, during and/or after the occurrence of a transient event. An acute administration generally entails an administration that is indicated by a transient event or condition. For example, acute administration may be implicated during an evolving myocardial infarction or during unstable angina. Administration before, during, and/or after a percutaneous cardiac intervention ("PCI") also constitutes an example of an acute administration. In addition, a GLP-1 molecule or agonist or analog thereof, or exendin molecule or agonist or analog thereof, may be administered acutely before, during and/or after any cardiac surgery, such as open-heart surgery, coronary bypass, minimally invasive cardiac surgery, valvuloplasty, or cardiac transplantation. Alternatively, a GLP-1 molecule or agonist or analog thereof, or exendin molecule or agonist or analog thereof, may also be administered acutely on the basis of congestive heart failure following myocardial infarction or surgery.

Acute administration before, during, and/or after a particular event may begin at any time before the happening of the event (*e.g.,* such as surgery or transplant) and may continue for any length of time, including for an extended period of time after the event, that is useful to prevent or ameliorate cardiac myocyte injury or death associated with the event. The duration of an acute administration can be determined by a clinician in light of the risk of cardiac myocyte injury or death related to the event or condition. In an embodiment, the duration of an acute administration is 1 hour, 2 hours, 4 hours, 8 hours, 12 hours, 18 hours, 24 hours, 36 hours, 48 hours, or 72 hours.

Chronic administration of a GLP-1 molecule or agonist or analog thereof, or exendin molecule or agonist or analog thereof, for the prevention or treatment of congestive heart failure may be warranted where no particular transient event or transient condition associated with congestive heart failure is identified. Chronic administration includes administration of a GLP-1 molecule or agonist or analog thereof, or exendin molecule or agonist or analog thereof, for an indefinite period of time on the basis of a general predisposition to congestive heart failure or on the basis of a predisposing condition that is non-transient (e.g., a condition that is non-transient may be unidentified or unamenable to elimination, such as hypertension or ischemic heart disease). A GLP-1 molecule or agonist or analog thereof, or exendin molecule or agonist or analog thereof, may be administered chronically in the methods of the disclosure in order to prevent congestive heart failure, regardless of etiology. Chronic administration of a GLP-1 molecule or agonist or analog thereof, or exendin molecule or agonist or analog thereof, for the prevention congestive heart failure may also be implicated in diabetics at risk for this condition. A GLP-1 molecule or agonist or analog thereof, or exendin molecule or agonist or analog thereof, may also be administered on a chronic basis in order to preserve a transplanted organ in individuals who have received a heart transplant. When a GLP-1 molecule or agonist or analog thereof, or exendin molecule or agonist or analog thereof, is administered chronically, administration may continue for any length of time. However, chronic administration often occurs for an extended period of time. For example, in an embodiment, chronic administration continues for greater than 72 hours. In another embodiment, chronic administration continues for 96 hours, 120 hours, 144 hours, 1 week, 2 weeks, 3 weeks, 4 weeks, 5 weeks, 6 weeks, 7 weeks, 8 weeks, 9 weeks, 10 weeks, 11 weeks, 12 weeks, 4 months, 5 months, 6 months, 9 months, 1 year, 2 years or longer.

In one embodiment, administration of a GLP-1 molecule or agonist or analog thereof, or exendin molecule or agonist or analog thereof, to prevent congestive heart failure can be a prophylactic treatment, beginning concurrently with the diagnosis of conditions (*e.g.,* disease or disorder) which places a subject at risk of congestive heart failure, such as for example upon a diagnosis of myocardial infarction (MI). In the alternative, administration of a GLP-1 molecule or agonist or analog thereof, or exendin molecule or agonist or analog thereof, to prevent congestive heart failure can occur subsequent to occurrence of symptoms associated with congestive heart failure.

The term "effective amount" refers to an amount of a pharmaceutical agent used to treat, ameliorate, prevent, or eliminate the identified condition (e.g., disease or disorder), or to exhibit a detectable therapeutic or preventative effect. The effect can be detected by, for example, chemical markers, biomarkers, antigen levels, or time to a measurable event, such as morbidity or mortality. Therapeutic effects include preventing further loss of cardiac function, or attenuating cardiac remodeling, or both. Therapeutic effects also include a reduced rate of enlargement of the left ventricle chamber. Further therapeutic effects include reduction in physical symptoms of a subject, such as, for example, an increased capacity for physical activity prior to breathlessness. The precise effective amount for a subject will depend upon the subject's body weight, size, and health; the nature and extent of the condition; and the therapeutic or combination of therapeutics selected for administration. Effective amounts for a given situation can be determined by routine experimentation that is within the skill and judgment of the clinician.

For any GLP-1 molecule or agonist or analog thereof, or exendin molecule or agonist or analog thereof, the effective amount can be estimated initially either in cell culture assays, e.g., or in animal models, such as rat or mouse models. An animal model may also be used to determine the appropriate concentration range and route of administration. Such information can then be used to determine useful doses and routes for administration in humans.

Efficacy and toxicity may be determined by standard pharmaceutical procedures in cell cultures or experimental animals, *e.g*., ED₅₀ (the dose therapeutically effective in 50% of the population) and LD₅₀ (the dose lethal to 50% of the population). The dose ratio between toxic and therapeutic effects is the therapeutic index, and it can be expressed as the ratio, LD₅₀/ED₅₀. Pharmaceutical compositions that exhibit large therapeutic indices are preferred. The data obtained from cell culture assays and animal studies may be used in formulating a range of dosage for human use. The dosage contained in such compositions is preferably within a range of circulating concentrations that include an ED₅₀ with little or no toxicity. The dosage may vary within this range depending upon the dosage form employed, sensitivity of the patient, and the route of administration.

In one embodiment, the methods also include administration of GLP-1 molecule or agonist or analog thereof, or exendin molecule or agonist or analog thereof, to improve cardiac function associated with congestive heart failure. Improving cardiac function may include an improvement in cardiac systolic function or cardiac diastolic function, or both. Improving cardiac function may also include an improvement in E/A ratio, left ventricular ejection fraction (LVEF), or left atrial volume (LAV). Exemplary molecules include, but are not limited to, the C-terminal amide form of HGEGTFTSDLSKQLEEEAVRLFIEWLKNGGPSSGAPPPS (SEQ ID NO: 3) and the C-terminal acid peptide HGEGTFTSDLSKQLEEEAVRLFIEWLKQGGPSKEIIS-OH (SEQ ID NO: 60). Improved cardiac function may be measured by any method known in the art, including the methods described in Examples 5-7.

In evaluating improved cardiac function associated with congestive heart failure, the improved function may be an improvement of any amount as compared with the cardiac functioning prior to administration of a GLP-1 molecule or agonist or analog thereof, or exendin molecule or agonist or analog thereof. Alternatively, the improved function may be an improvement of any amount as compared to the cardiac function of a matched control subject receiving vehicle only. For example, the improvement (i.e., increase) in LVEF after treatment may be about 10%, 20%, 30%, 40%, 50%, 60%, 70%, 80%, 90%, 100%, 150%, 200% or more than about 200%. In another example, the improvement in E/A ratio after treatment may be about 10%, 20%, 30%, 40%, 50%, 60%, 70%, 80%, 90%, 100%, 150%, 200% or more than about 200%. In yet another example, the improvement in LAV may be about 10%, 20%, 30%, 40%, 50%, 60%, 70%, 80%, 90%, 100%, 150%, 200% or more than about 200%.

In one embodiment, the methods also include administration of a GLP-1 molecule or agonist or analog thereof, or an exendin molecule or agonist or analog thereof, to attenuate cardiac remodeling. Cardiac remodeling may be measured by any method known in the art, including the methods described in Examples 5-7, such as echocardiography. As an example, left ventricle chamber size may be used as a measure for cardiac remodeling. In evaluating attenuation of cardiac remodeling, an attenuation of the increase in size of the left ventricle may be an attenuation of any amount as compared with the left ventricle size before administration of a GLP-1 molecule or agonist or analog thereof, or exendin molecule or agonist or analog thereof. Alternatively, an attenuation of the increase in size of the left ventricle may be an attenuation of any amount as compared with the left ventricle size of a matched control subject receiving vehicle only. Left ventricle chamber size may be measured, for example, by assaying left ventricle end diastolic dimension (LVEDD) or left ventricle end systolic dimension (LVESD). In an example, the change in LVEDD after treatment with a GLP-1 molecule or agonist or analog thereof, or exendin molecule or agonist or analog thereof may be 10%, 20%, 30%, 40%, 50%, 60%, 70%, 80%, 90%, 100%, 150%, 200% or more than about 200%. In another example, the change in LVESD after treatment with a GLP-1 molecule or agonist or analog thereof, or exendin molecule or agonist or analog thereof may be 10%, 20%, 30%, 40%, 50%, 60%, 70%, 80%, 90%, 100%, 150%, 200% or more than about 200%. Specifically excluded for this use are the specific GLP-1 agonists and exendin agonists described in publication WO2006110887.

In one embodiment, the methods also include administration of GLP-1 molecule or agonist or analog thereof, or exendin molecule or agonist or analog thereof, to attenuate insulin resistance associated with congestive heart failure. Insulin resistance associated with congestive heart failure may be measured by any method known in the art, including the methods described in Examples 5-7. For example, insulin resistance may be measured by assaying plasma insulin levels, plasma glucose levels or Homeostasis Model Assessment (HOMA). In an example, the reduction in plasma insulin or plasma glucose levels after treatment with a GLP-1 molecule or agonist or analog thereof, or exendin molecule or agonist or analog thereof may be 10%, 20%, 30%, 40%, 50%, 60%, 70%, 80%, 90%, 100%, 150%, 200% or more than about 200%. In another example, the change (reduction) in HOMA after treatment with a GLP-1 molecule or agonist or analog thereof, or exendin molecule or agonist or analog thereof may be 10%, 20%, 30%, 40%, 50%, 60%, 70%, 80%, 90%, 100%, 150%, 200% or more than about 200%.

In one embodiment, the methods also include administration of GLP-1 molecule or agonist or analog thereof, or exendin molecule or agonist or analog thereof, to improve exercise capacity in a subject having congestive heart failure. The improvement in exercise capacity may be measured by any method known in the art, including the methods described in Examples 5-7. For example, the improvement in exercise capacity may be measured by assaying peak VO₂ uptake or exercise capacity to peak lactate ratio. Peak oxygen uptake during exercise may be measured, for example, by indirect calorimetry. In an example, the change in exercise capacity to peak lactate ratio after treatment with a GLP-1 molecule or agonist or analog thereof, or exendin molecule or agonist or analog thereof may be 10%, 20%, 30%, 40%, 50%, 60%, 70%, 80%, 90%, 100%, 150%, 200% or more than about 200%.

In one embodiment, the methods also include administration of a GLP-1 molecule or agonist or analog thereof, or an exendin molecule or agonist or analog thereof, to improve cardiac contractility. Improving cardiac contractility may include any increase in the number of cardiac myocytes available for contraction, the ability of cardiac myocytes to contract, or both. In order to evaluate the improvement of cardiac contractility, any mode of assessment may be used. For example, clinical observation, such as an increase in cardiac output or a decrease in cardiac rate or both, may lead to a determination of increased cardiac contractility. Alternatively, *in vivo* an increased contractility of the heart may be assessed by a determination of an increased fractional shortening of the left ventricle. Fractional shortening of the left ventricle may be observed by any available means such as echocardiograph.

In evaluating increased cardiac contractility, the increase in fractional shortening of the left ventricle may be an increase of any amount as compared with the fractional shortening before administration of a GLP-1 molecule or agonist or analog thereof, or exendin molecule or agonist or analog thereof. For example, the increase in shortening may be about 10%, 20%, 30%, 40%, 50%, 60%, 70%, 80%, 90%, 100%, 150%, 200% or more than about 200%. In a further aspect, prophylactic and therapeutic methods are provided. Treatment on an acute or chronic basis is contemplated. In addition, treatment on an acute basis may be extended to chronic treatment, if so indicated. In one aspect is provided a method for the treatment or prevention of a condition associated with congestive heart failure in a subject in need thereof. The method generally comprises administering to the subject an amount of a GLP-1 molecule or agonist or analog thereof, or exendin molecule or agonist or analog thereof, effective to prevent or ameliorate congestive heart failure, wherein the condition associated with congestive heart failure is thereby improved. As described herein, administration of any GLP-1 molecule or agonist or analog thereof, or exendin molecule or agonist or analog thereof, may be done in any manner.

In yet another embodiment, the methods further comprise the identification of a subject in need of treatment. Any effective criteria may be used to determine that a subject may benefit from administration of a GLP-1 molecule or agonist or analog thereof, or exendin molecule or agonist or analog thereof. Methods for the diagnosis of heart disease and diabetes, for example, as well as procedures for the identification of individuals at risk for development of these conditions, are well known to those in the art. Such procedures may include clinical tests, physical examination, personal interviews and assessment of family history.

In one embodiment, the GLP-1 agonists or analogs and the exendin molecules and agonists or analogs thereof disclosed herein have increased stability in plasma as compared to native GLP-1. In another embodiment, greater than 90% of any one of the GLP-1 agonists or analogs and the exendin molecules and agonists or analogs thereof disclosed herein resists degradation, that is has increased plasma stability as shown by 90% of the peptide molecules being intact, after incubation in plasma for 5 hours. In still another embodiment, greater than 75% of any one of the GLP-1 agonists or analogs and the exendin molecules and agonists or analogs thereof disclosed herein resist degradation, that is have increased plasma stability as shown by 75% of the peptide molecules being intact, after incubation in plasma for 2 hours. Examples of peptides having increased stability can be found in Table 1. In one embodiment, examples of the GLP-1 agonists or analogs and the exendin molecules and agonists or analogs thereof having increased plasma stability include at least one of compounds 3922, 4103, 4596, 4597, 4784, 4792, 4793, 4855, 4856, 5272, 5194, 5112, 5090, 5091, 5092, 5096, 5099, 5100, 5102, 5452, 5128, 5129, 5130, 5271, 5182, 5196, 5270, 5271, 5272, 5197, 5452, 5450, 5198, 5199, 5200, 5264, 5265, 5266, 5267, 5268, 5269, 5391, 5097, 5098, 5101, 5103, 5131, 5526, 5132, 5185, 5186, 5294, 5296, 5297, 5440, 5441, 5442, 5443, 5444, 5445, 5446, 5451, 4983, 4984, 5201, 5202, 5203, 5293, 4992, 5447, 5540, or 5052 or any subgroup of such compounds. In another embodiment, examples of the GLP-1 agonists or analogs and the exendin molecules and agonists or analogs thereof having increased plasma stability include at least one of compounds 3922, 4103, 4596, 4597, 4855, 4856, 5194, 5112, 5090, 5091, 5092, 5096, 5099, 5102, 5452, 5129, 5182, 5452, 5200, 5267, 5268, 5269, 5391, 5101, 5103, 5131, 5132, 5185, 5186, 5294, 5297, 5440, 5441, 5442, 5443, 5451, 4983, 4992, or 5052 or any subgroup of such compounds.

To assess stability, the compound of interest is spiked into plasma at a concentration of 20µg/mL. It is incubated at 37°C for 0, 1, 2 3, 4, 5 hours in triplicate. At each time point, MeOH is added to a 96 well microtiter plate and then sample is added in a ratio of 1 part sample to 4 parts MeOH to quench the digestion reaction. Samples are mixed with multi-channel pipette or liquid handler. The plate is then centrifuged for 10 minutes and placed in an autosampler kept at 10°C. Samples are then injected into a mass spectrometer (API 3000, Applied Biosystems) equipped with an autosampler (Leap HTC Pal) and HPLC pumps (Shimadzu LC-10ADVP).

The GLP-1 molecules or agonists or analogs thereof, or exendin molecules or agonists or analogs thereof, may be formulated as pharmaceutical compositions for use in conjunction with the methods of the present disclosure. Compositions disclosed herein may conveniently be provided in the form of formulations suitable for parenteral (including intravenous, intramuscular and subcutaneous) or nasal or oral administration. In some cases, it will be convenient to provide a GLP-1 molecule or agonist or analog thereof, or an exendin or exendin agonist or analog thereof, and another active agent, such as another food-intake-reducing, plasma glucose-lowering or plasma lipid-lowering agent, such as amylin, an amylin agonist, a CCK, or a leptin, or another cardiac treatment agent such as angiotensin converting enzyme (ACE) inhibitors, in a single composition or solution for administration together. In other cases, it may be more advantageous to administer the additional agent separately from said GLP-1 or agonist or analog thereof, or exendin or exendin agonist or analog thereof. A suitable administration format may best be determined by a medical practitioner for each subject individually. Suitable pharmaceutically acceptable carriers and their formulation are described in standard formulation treatises, *e.g*., Remington's Pharmaceutical Sciences by E. W. Martin. See also Wang, Y. J. and Hanson, M. A. "Parenteral Formulations of Proteins and Peptides: Stability and Stabilizers," Journal of Parenteral Science and Technology, Technical Report No. 10, Supp. 42:2S (1988).

Compounds can be provided as parenteral compositions for injection or infusion. They can, for example, be suspended in inert oil, suitably a vegetable oil such as sesame, peanut, olive oil, or other acceptable carrier. In one embodiment, they are suspended in an aqueous carrier, for example, in an isotonic buffer solution at a pH of about 3.0 to 8.0, or at a pH of about 3.5 to 5.0. In alternative embodiments, the pH may be adjusted to a pH range from about 5.0 to about 8.0. These compositions may be sterilized by conventional sterilization techniques, or may be sterile filtered. The compositions may contain pharmaceutically acceptable auxiliary substances as required to approximate physiological conditions, such as pH buffering agents. Useful buffers include for example, sodium acetate/acetic acid buffers. A form of repository or "depot" slow release preparation may be used so that therapeutically effective amounts of the preparation are delivered into the bloodstream over many hours, days or weeks following transdermal injection or delivery. Examples of sustained release matrices, e.g., PLGA, and their formulations can be found in publication WO2005/102293A1, for example, which is incorporated by reference in its entirety.

The desired isotonicity may be accomplished using sodium chloride or other pharmaceutically acceptable agents such as dextrose, boric acid, sodium tartrate, propylene glycol, polyols (such as mannitol and sorbitol), or other inorganic or organic solutes. Sodium chloride is particularly useful for buffers containing sodium ions.

The term "pharmaceutically acceptable excipient" refers to an excipient for administration of a pharmaceutical agent, such as a GLP-1 molecule or agonist or analog thereof or an exendin or agonist or analog thereof. The term refers to any pharmaceutical excipient that may be administered without undue toxicity. Pharmaceutically acceptable excipients are determined in part by the particular composition being administered, as well as by the particular method used to administer the composition. Accordingly, there exists a wide variety of suitable formulations of pharmaceutical compositions for use in the methods of the present disclosure (*see, e.g*., *Remington's Pharmaceutical Sciences).*

Suitable excipients may be carrier molecules that include large, slowly metabolized macromolecules such as proteins, polysaccharides, polylactic acids, polyglycolic acids, polymeric amino acids, amino acid copolymers, and inactive virus particles. Other exemplary excipients include antioxidants such as ascorbic acid; chelating agents such as EDTA; carbohydrates such as dextrin, cyclodextrin, hydroxyalkylcellulose, hydroxyalkylmethylcellulose, stearic acid; liquids such as oils, water, saline, glycerol and ethanol; wetting or emulsifying agents; pH buffering substances; and the like. Liposomes are also included within the definition of pharmaceutically acceptable excipients. Other examples of carriers and excipients include calcium carbonate, calcium phosphate, various sugars such as lactose, glucose, or sucrose, or types of starch, cellulose derivatives, gelatin, vegetable oils, polyethylene glycols and physiologically compatible solvents.

Certain GLP-1 molecules or agonists or analogs thereof, or exendin molecules or agonists or analogs thereof, may be substantially insoluble in water and sparingly soluble in most pharmaceutically acceptable protic solvents and in vegetable oils. However, the compounds may be soluble in medium chain fatty acids (*e.g*., caprylic and capric acids) or triglycerides and have high solubility in propylene glycol esters of medium chain fatty acids. Also contemplated for use in the methods of the disclosure are compositions, which have been modified by substitutions or additions of chemical or biochemical moieties which make them more suitable for delivery (e.g., increase solubility, bioactivity, palatability, decrease adverse reactions, etc.), for example by esterification, glycation, PEGylation, etc.

A GLP-1 molecule or agonist or analog thereof, or an exendin molecule or agonist or analog thereof, may also be formulated for oral administration in a self-emulsifying drug delivery system (SEDDS). Lipid-based formulations such as SEDDS are particularly suitable for low solubility compounds, and can generally enhance the oral bioavailability of such compounds.

In an alternative embodiment, cyclodextrins may be added as aqueous solubility enhancers. Cyclodextrins include methyl, dimethyl, hydroxypropyl, hydroxyethyl, glucosyl, maltosyl and maltotriosyl derivatives of α-, β-, and γ-cyclodextrin. An exemplary cyclodextrin solubility enhancer is hydroxypropyl-β-cyclodextrin (HPBC), which may be added to any of the above-described compositions to further improve the aqueous solubility characteristics of a GLP-1 molecule or agonist or analog thereof, or exendin molecules or agonist or analog thereof. In one embodiment, the composition comprises 0.1 % to 20% hydroxypropyl-β-cyclodextrin, 1% to 15% hydroxypropyl-β-cyclodextrin, or from 2.5% to 10% hydroxypropyl-β-cyclodextrin. The amount of solubility enhancer employed will depend on the amount of GLP-1 molecule or agonist thereof, or exendin molecule or agonist thereof, in the composition.

In other embodiments, absorption enhancers may be added including, but not limited to, cationic polyamino acids, such as poly-arginine, poly-histidine and poly-lysine. Other suitable absorption enhancing agents include chitosan and phospholipids such as didecanoyl phosphatidylcholine (DDPC).

If desired, solutions of the compositions described herein may be thickened with a thickening agent such as methyl-cellulose. They may be prepared in emulsified form, either water in oil or oil in water. Any of a wide variety of pharmaceutically acceptable emulsifying agents may be employed including, for example, acacia powder, a nonionic surfactant (such as a Tween), or an ionic surfactant (such as alkali polyether alcohol sulfates or sulfonates, *e.g.,* a Triton).

Compositions may be prepared by mixing the ingredients following generally accepted procedures. For example, the selected components may be simply mixed in a blender or other standard device to produce a concentrated mixture which may then be adjusted to the final concentration and viscosity by the addition of water or thickening agent and possibly a buffer to control pH or an additional solute to control tonicity.

For use by the physician, the compositions may be provided in dosage unit form containing an amount of a GLP-1 or an agonist or analog thereof or an exendin or exendin agonist or analog, for example, exendin-3, and/or exendin-4. As will be recognized by those in the field, an effective amount of therapeutic agent will vary with many factors including the age and weight of the subject, the subject's physical condition and other factors.

The exact dose to be administered is determined by the attending clinician and is dependent, for example, upon where the particular compound lies within the ranges quoted herein. Administration should begin whenever a therapeutic effect is desired, for example, at the first sign of symptoms or shortly after diagnosis of congestive heart failure or diabetes. Administration may be by injection, for example, subcutaneous or intramuscular. Orally active compounds may be taken orally, however dosages should be increased 5-20 fold.

The optimal formulation and mode of administration of compounds of the present application to a subject depend on factors known in the art such as the particular disease or disorder, the desired effect, and the type of subject. While the compounds will typically be used to treat human subjects they may also be used to treat similar or identical diseases in other vertebrates such as other primates, farm animals such as swine, cattle and poultry, and sports animals and pets such as horses, dogs and cats.

In another aspect, it is also possible to combine a GLP-1 molecule or agonist or analog thereof, or exendin molecule or agonist or analog thereof, useful in the methods disclosed herein with one or more other active ingredients useful in the prevention of congestive heart failure. For example, a GLP-1 molecule or agonist or analog thereof, or exendin molecule or agonist or analog thereof, may be combined with one or more other compounds, e.g., for congestive heart failure, for obesity treatment, for glucose lowering, etc. as described herein, in a unitary dosage form, or in separate dosage forms intended for simultaneous or sequential administration to a subject in need of treatment. When administered sequentially, the combination may be administered in two or more administrations. In an alternative embodiment, it is possible to administer one or more GLP-1 molecules or agonists or analogs thereof, or exendin molecules or agonists or analogs thereof, and one or more additional active ingredients by different routes. The skilled artisan will also recognize that a variety of active ingredients may be administered in combination with GLP-1 molecules or agonists or analogs thereof, or exendin molecules or agonists or analogs thereof, that may act to augment or synergistically enhance the prevention or treatment of the condition of interest, e.g., congestive heart failure.

According to the methods disclosed herein, a GLP-1 molecule or agonist or analog thereof, or exendin molecule or agonist or analog thereof, may be: (1) co-formulated and administered or delivered simultaneously in a combined formulation; (2) delivered by alternation or in parallel as separate formulations; or (3) by any other combination therapy regimen known in the art. When delivered in alternation therapy, the methods may comprise administering or delivering the active ingredients sequentially, e.g., in separate solution, emulsion, suspension, tablets, pills or capsules, or by different injections in separate syringes. In general, during alternation therapy, an effective dosage of each active ingredient is administered sequentially, *i.e.,* serially, whereas in simultaneous therapy, effective dosages of two or more active ingredients are administered together. Various sequences of intermittent combination therapy may also be used.

In one embodiment, a GLP-1 molecule or agonist or analog thereof, or an exendin molecule or agonist or analog thereof may be used for treatment of congestive heart failure in combination with angiotensin converting enzyme (ACE) inhibitors. In one embodiment, a GLP-1 molecule or agonist or analog thereof, or an exendin molecule or agonist or analog thereof is used in combination with captopril (CAPOTEN®). In other embodiments, the agents of the disclosure may be used in combination with one or more additional ACE inhibitors, such as benazepril (LOTENSIN^{®}), enalapril (VASOTEC^{®}), lisinopril (PRINTVIL^{®}, ZESTRIL^{®}), fosinopril (MONOPRIL^{®}), ramipril (ALTACE^{®}), perindopril (ACEON^{®}), quinapril (ACCUPRIL^{®}), moexipril (UNIVASC^{®}), and trandolapril (MAVIK^{®}).

In another embodiment, a GLP-1 molecule or agonist or analog thereof, or an exendin molecule or agonist or analog thereof may be used for treatment of congestive heart failure in combination with one or more beta blockers, such as sotalol (BETAPACE^{®}), timolol (BLOCADREN^{®}), esmolol (BREVIBLOC^{®}), carteolol (CARTROL^{®}), carvedilol (COREG^{®}), nadolol (CORGARD^{®}), propranolol (INDERAL^{®}), propranolol (INDERAL-LA^{®}), betaxolol (KERLONE^{®}), penbutolol (LEVATOL^{®}), metoprolol (LOPRESSOR^{®}), labetalol (NORMODYNE^{®}), acebutolol (SECTRAL^{®}), atenolol (TENORMIN^{®}), metoprolol (TOPROL-XL^{®}), labetalol (TRANDATE^{®}), pindolol (VISKEN^{®}), and bisoprolol (ZEBETA^{®}).

In another embodiment, a GLP-1 molecule or agonist or analog thereof, or an exendin molecule or agonist or analog thereof may be used for treatment of congestive heart failure in combination with one or more angiotensin II receptor blockers (ARB), such as candesartan cilexetil (ATACAND^{®}), irbesartan (AVAPRO^{®}), losartan (COZAAR^{®}), valsartan (DIOVAN^{®}), telmisartan (MICARDIS^{®}), and eprosartan mesylate (TEVETEN^{®}).

In another embodiment, a GLP-1 molecule or agonist or analog thereof, or an exendin molecule or agonist or analog thereof may be used for treatment of congestive heart failure in combination with one or more aldosterone antagonists, such as spironolactone (ALDACTAZIDE®) and eplerenone (INSPRA^{®}).

In another embodiment, a GLP-1 molecule or agonist or analog thereof, or an exendin molecule or agonist or analog thereof may be used for treatment of congestive heart failure in combination with one or more vasopeptidase inhibitors. Vasopeptidase inhibitors include NEP/ACE inhibitors that possess natural endopeptidase (NEP) and ACE inhibitory activity. Examples of NEP/ACE inhibitors include, but are not limited to, tricyclic benzazepinone thiols, omapatrilat, gemopatrilat, mixanpril, racecadotril, fasidotril, sampatrilat, MDL 100.240 Z13752A, BMS189921, BMS182657, and CGS 30008. Examples of NEP/ACE inhibitors suitable for use herein include those disclosed in U.S. Pat. Nos. 5,362,727, 5,366,973, 5,225,401, 4,722,810, 5,223,516, 4,749,688, and 5,552,397.

A GLP-1 molecule or agonist or analog thereof, or an exendin molecule or agonist or analog thereof may be used for treatment of congestive heart failure in combination with any therapy for congestive heart failure that is known in the art. For example, in one embodiment, a GLP-1 molecule or agonist or analog thereof, or an exendin molecule or agonist or analog thereof may be used for treatment of congestive heart failure in combination with therapeutic devices such as cardiac resynchronization.

To assist in understanding the present invention, the following Examples are included. The experiments relating to this invention should not, of course, be construed as specifically limiting the invention and such variations of the invention, now known or later developed, which would be within the purview of one skilled in the art are considered to fall within the scope of the invention as described herein and hereinafter claimed. Each reference cited herein is incorporated by reference in its entirety.

### EXAMPLES

### Example 1. Receptor Binding Assay

Membranes are prepared from confluent cultures of RINm5f cells expressing endogenous GLP-1 receptors. Membranes are incubated with [¹²⁵I] human GLP-1 (2000 Ci/mmol) and with unlabeled peptides for 60 minutes at ambient temperature in 96 well polystyrene plates. The well contents are harvested onto 96 well glass fiber plates using a Perkin Elmer plate harvestor. Dried glass fiber plates are combined with scintillant and counted on a Perkin Elmer scintillation counter.

### Example 2. Cyclase Assay

### GLP/GIP/CT Cyclase (RIN) assay

Five µl of serially diluted peptides are transferred to the 384-well assay plate. Rat pancreas insulinoma cells (RIN-m5F) are detached from a tissue culture flask with Versene and washed once in buffer. Then RIN-m5F cells are resusupended in buffer at 2.5 x 10⁶ cells/ml. Then 10 µl cells (2.5 x 10⁴ cells) are added to all wells of the assay plate and cells are stimulated at room temperature in the dark for 30 minutes. The reaction is terminated with 10 µl of lysis buffer and incubated at room temperature for 4 hrs in the dark. cAMP content is measured using Perkin Elmer AlphaScreen™ cAMP assay kit and results are read on a Perkin Elmer Fusion fluorometer. The assay is completed in 384-well plates at 25 microliter volumes.

### GLP-1 Cyclase (6-23)

Five µl of serially diluted peptides are transferred to the 384-well assay plate. Rat thyroid carcinoma cells (6-23) are detached from a tissue culture flask with Versene and washed once in buffer. Then 6-23 cells are resusupended in buffer at 3.0 x 10⁶ cells/ml. Then 10 µl cells (3.0 x 10⁴ cells) are added to all wells of the assay plate and cells are stimulated at room temperature in the dark for 30 minutes. The reaction is terminated with 10 µl of lysis buffer and incubated at room temperature for 4 hrs in the dark. cAMP content is measured using Perkin Elmer AlphaScreen™ cAMP assay kit and results are read on a Perkin Elmer Fusion fluorometer. The assay is completed in 384-well plates at 25 µl volumes.

### Example 3. Glucose Lowering Assay

Female NIH/Swiss mice (-8-20 weeks of age), (Harlan, Indianapolis, IN, USA), housed 3 mice per cage, are allowed *ad libitum* access to food and water until the start of the experiment. At two hours prior to treatment, access to food is restricted.

At the time of treatment, the tip of the tail is pricked with a needle to obtain 1µl blood as a "pre-treatment" control blood sample. Immediately thereafter, each mouse is injected intraperitoneally (IP) with a test sample (1 nmol/kg or 2 nmol/kg) or 200 µl vehicle (10%DMSO saline). Additional blood samples are collected at 30, 60, 120, 180, and 240 minutes after injection.

Blood glucose is measured with a glucose oxidase biosensor (OneTouch® Ultra® (LifeScan, Inc., a Johnson & Johnson Company, Milpitas, CA)). At each time point, the effect of the test sample is expressed as the percent change in blood glucose relative to mice injected with vehicle only. Test samples and vehicle controls are also compared to their respective "pre-treatment" controls.

Significant test sample effects are identified by ANOVA (p<0.05). Where a significant difference existed, test means are compared to the control mean with Dunnett's post test using GraphPad Prism version 4.00 for Windows, GraphPad Software, San Diego California USA, www.graphpad.com).

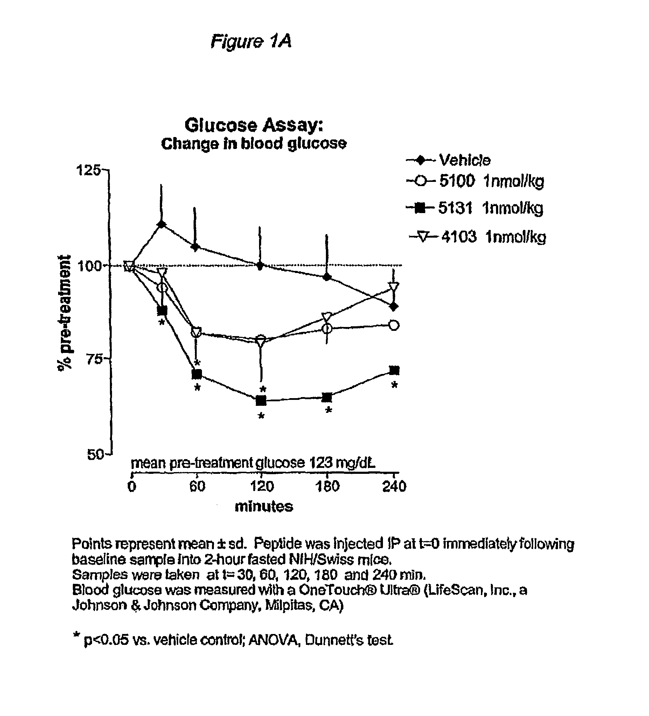

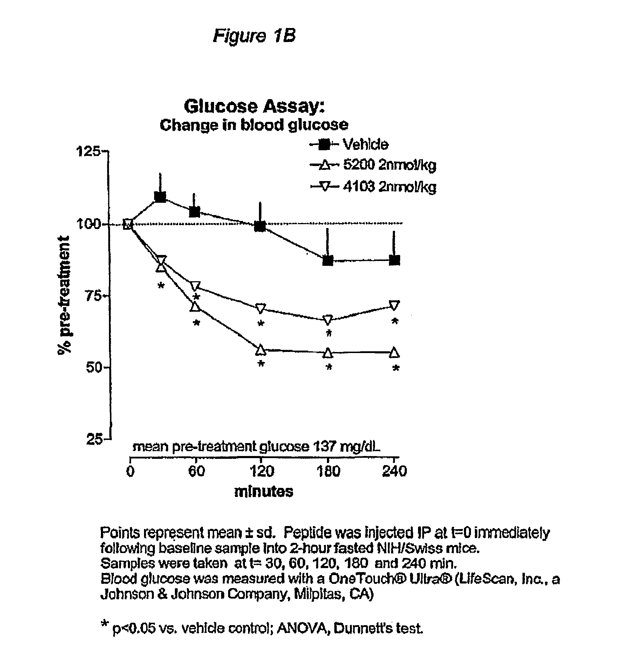

Exendin analogs disclosed herein have been shown to effectively lower blood glucose relative to vehicle control. *See, e.g.,* Table 1 and Figures 1A-B.

### Example 4. Food Intake Assay

All mice (NIH:Swiss mice) are housed in a stable environment of 22 (±2)° C., 60 (±10)% humidity and a 12:12 light:dark cycle; with lights on at 0300. Mice are housed in groups of three in standard cages with *ad libitum* access to food (Teklad: LM 485; Madison, Wis.) and water except as noted, for at least two weeks before the experiments.

All experiments are conducted between the hours of 0700 and 0900. The mice are food deprived (food removed at 1530 hr from all animals on day prior to experiment). All mice receive an intraperitoneal injection (200 µl) of either vehicle (10% DMSO saline) or test compound at 1 mg/kg and are immediately presented with a pre-weighed food pellet (Teklad LM 485). The food pellet is weighed at 30-minute, 1-hr, and 2-hr intervals to determine the amount of food eaten.

Significant test sample effects are identified by ANOVA (p<0.05). Where a signficicant difference exists, test means are compared to the control mean using Dunnett's test. One-way ANOVA with Dunnett's post test is performed using GraphPad Prism version 3.01 for Windows, GraphPad Software, San Diego California. *See, e.g*., Table 1.

### Example 5. MI-induced CHF Animal Model

Sprague Dawley rats undergo left coronary artery ligation to induce myocardial infarction (MI) and subsequently congestive heart failure. Some rats undergo sham surgery. Starting two weeks after coronary ligation, rats are treated with GLP-1 (2.5 or 25 pmol/kg/min), [Leu¹⁴]-Exendin-4 amide (1.67 or 5 pmol/kg/min) or vehicle for 11 weeks via subcutaneous infusion. Cardiac function and remodeling are assessed by echocardiography. At the end of study, rats undergo treadmill testing, hemodynamic measurements and fasting (12 hour fasting) insulin and glucose concentration were measured and the Homeostasis Model Assessment (HOMA), a major index for insulin resistance, is calculated. Peak Oxygen uptake during exercise is measured by indirect calorimetry.

Transthoracic Doppler echocardiography is performed. Briefly, short-axis images are obtained at the papillary muscle level and 2D guided M-mode tracing are recorded at a speed of 100 mm/s. Left ventricular end-diastolic (LVEDD) and end-systolic dimensions (LVESD) are measured according the American Society for Echocardiography leading-edge method. Left atrial volume (LAV) and ejection fraction (LVEF) are measured and calculated from the long-axis view.

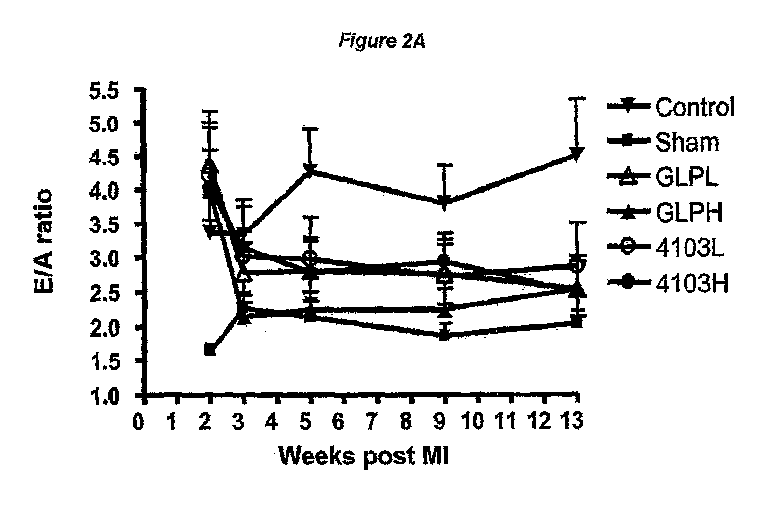

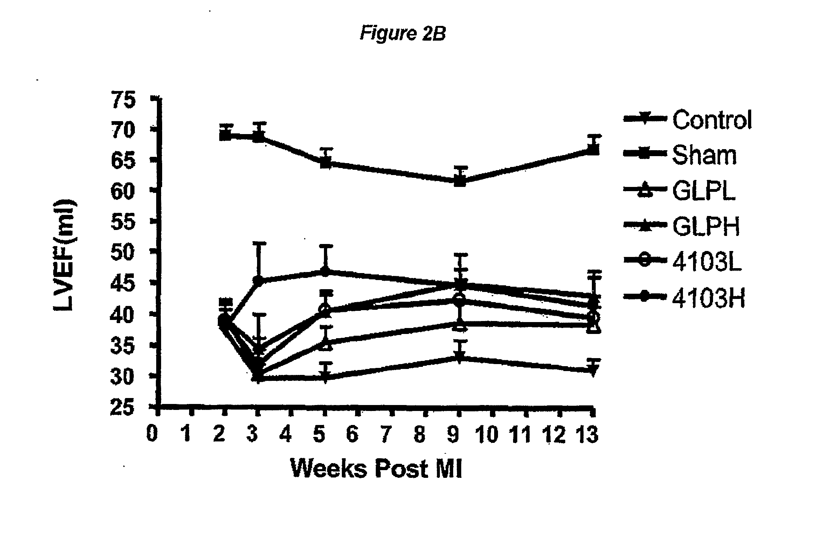

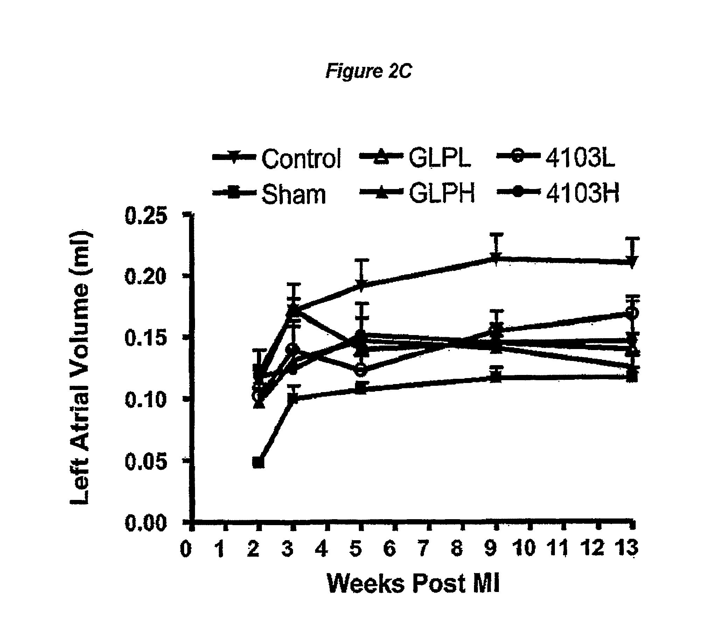

Pulsed-wave Doppler spectra of mitral inflow are obtained from apical 5-chamber view. The sample volume is placed at the tip of the mitral leaflets and adjusted to the position of maximal velocity. The peak of early (E) and late filling waves (A) are measured and E/A ratio are calculated. Chronic treatment with GLP-1 or compound 4103 improves cardiac diastolic and systolic function following MI-induced CHF. Fig. 2A-C. Designations "L" and "H" indicate low and high dose of drug, respectively. (E/A ratio and left atrial volume (LAV) represent cardiac diastolic function; left ventricular ejection fraction (LVEF) represents cardiac systolic function.)

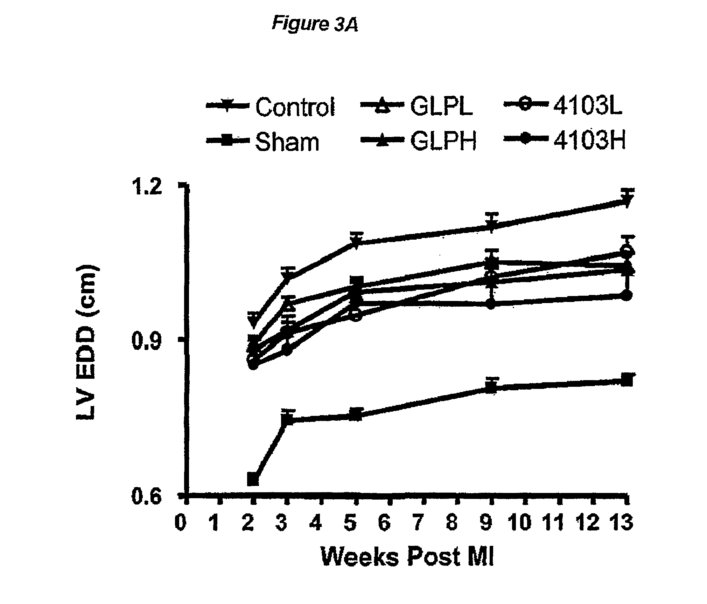

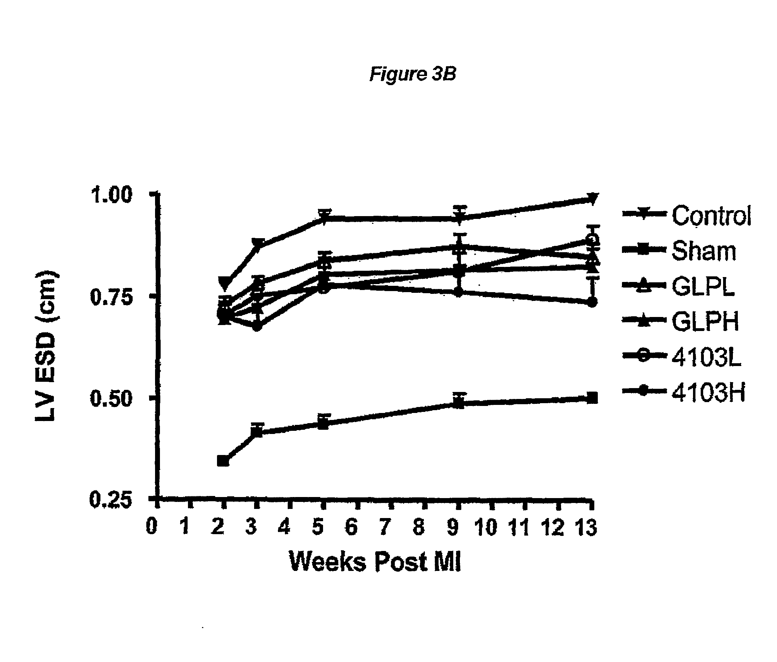

Chronic treatment with GLP-1 or compound 4103 attenuates enlargement of left ventricle chamber size following MI-induced CHF. Fig. 3A-B. Designations "L" and "H" indicate low and high dose of drug, respectively. Left ventricle chamber size is represented by left ventricle end diastolic dimension (LVEDD) and left ventricle end systolic dimension (LVESD). LVEDD and LVESD are measured according the American Society for Echocardiography leading-edge method.

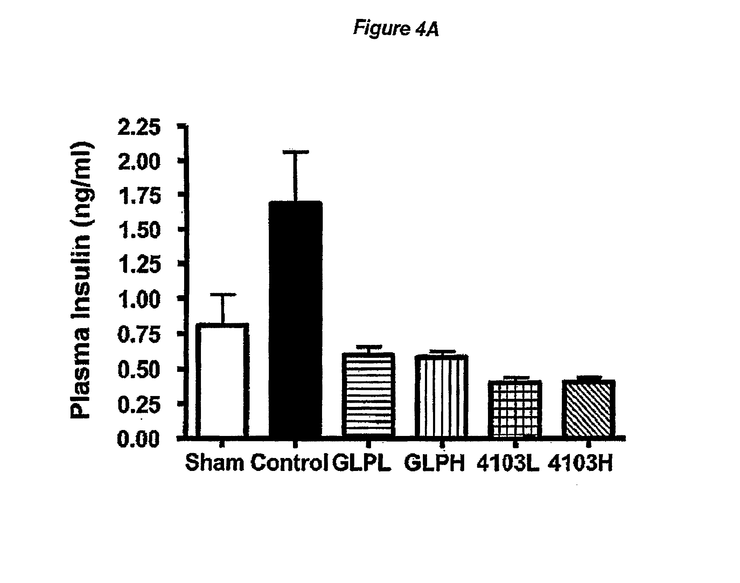

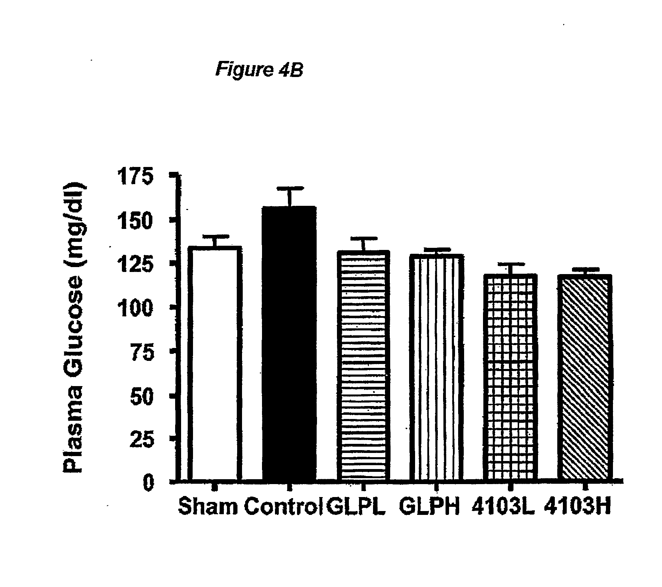

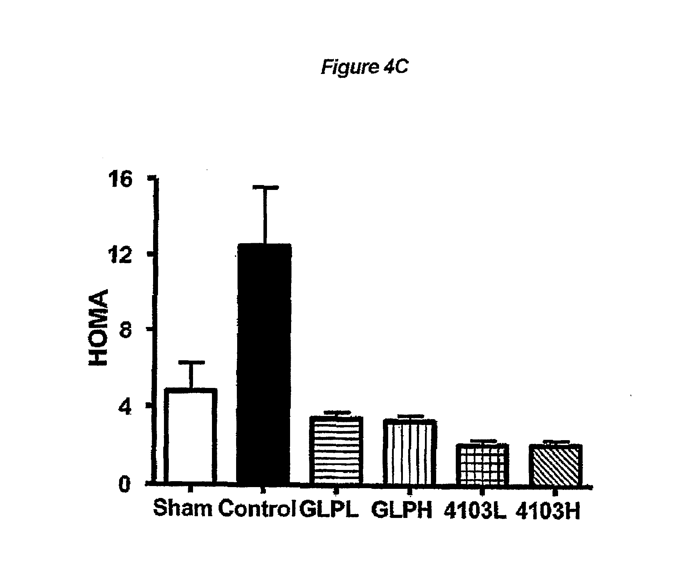

Chronic treatment with GLP-1 or compound 4103 attenuates insulin resistance and improves insulin sensitivity following MI-induced CHF. Fig. 4A-C. Designations "L" and "H" indicate low and high dose of drug, respectively. Hyperinsulinemia and hyperglycemia occurr at 13 week post MI in untreated control groups. As shown, chronic treatment can normalize fasting plasma insulin and glucose level and improve insulin sensitivity (as measured by Homeostasis Model Assessment (HOMA), a major index for insulin resistance).

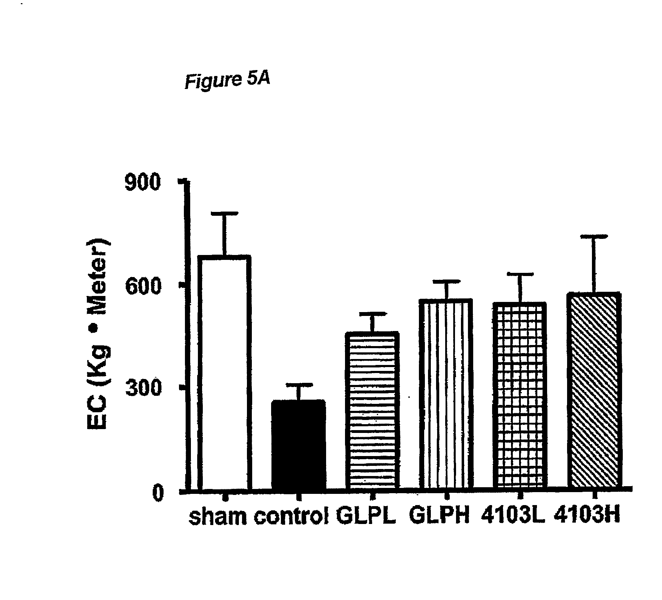

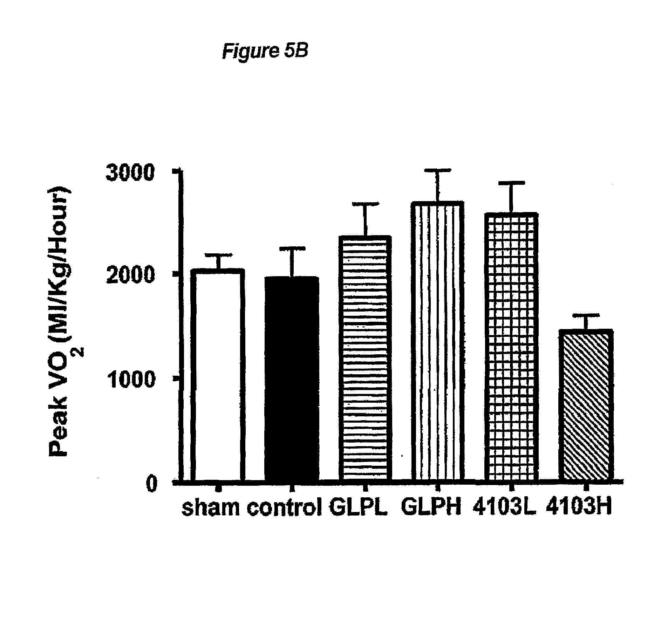

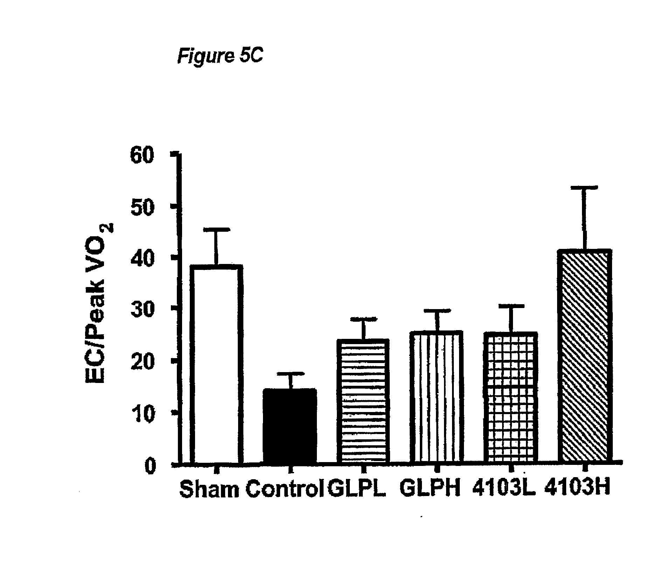

Fig. 5A-C demonstrates that chronic treatment with GLP-1 or compound 4103 improves exercise capacity and efficiency following MI-induced CHF. Designations "L" and "H" indicate low and high dose of drug, respectively. At the time of treadmill test, two rats are simultaneously placed on a two-track treadmill (Columbus Instruments, Columbus, OH) at a constant 5% grade enclosed by a metabolic chamber (Oxymax Deluxe, Columbus Instruments) through which airflow passes at a constant speed. Basal measurements are obtained over a period of 8-10 minutes. The treadmill is then started at 8 m/min for 3 minutes, followed by 12m/min for 3 minutes and then kept at 18m/min until rats reach exhaustion. The end point for treadmill test is determined by rat's inability to keep the pace of treadmill and land on the electric shock grid for over 6 seconds. The exercise capacity (EC) is calculated as EC (kgm)=Body weight (kg) x degree of grade x running distance. Oxygen consumption (VO₂), carbon dioxide production (VCO₂) are measured as described. Within 1 minute after the treadmill test, plasma lactate and glucose are measured. The treadmill test is run and analyzed by one investigator who is blinded to the study.

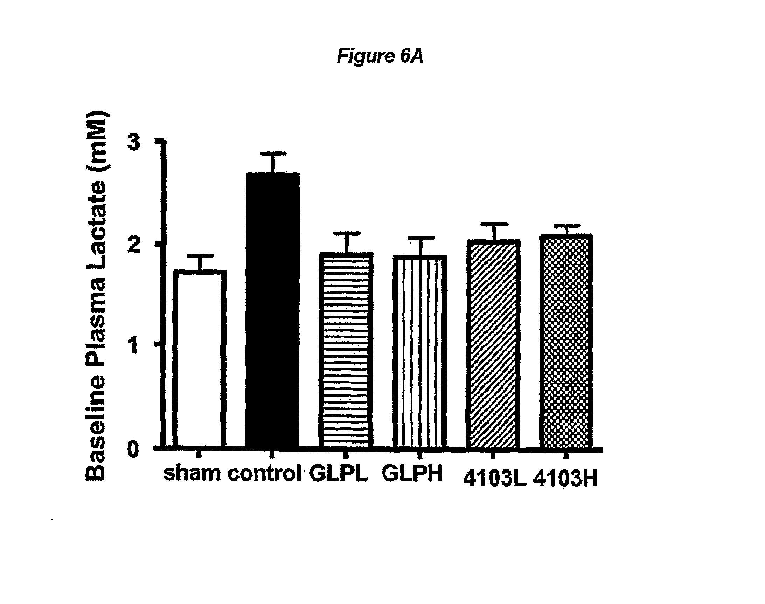

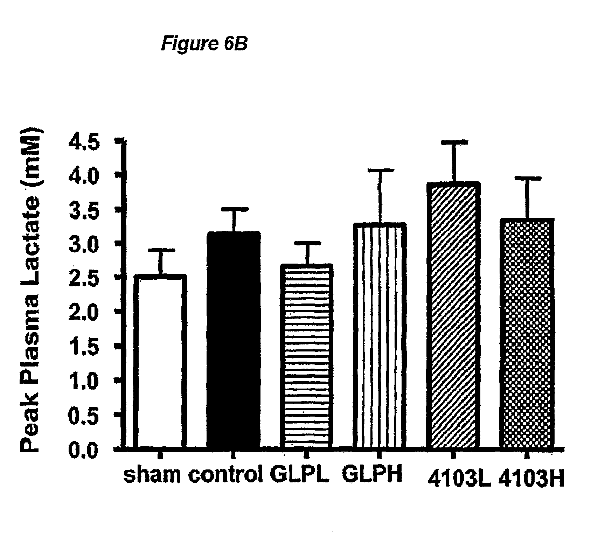

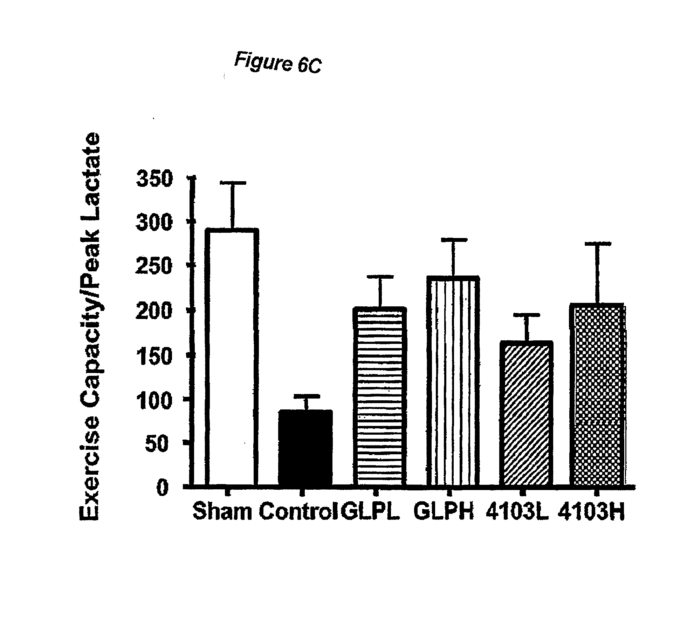

Fig. 6A-C demonstrates that chronic treatment with GLP-1 or compound 4103 results in an attenuated baseline plasma lactate level and improves exercise capacity to peak lactate ratio following MI-induced CHF. Designations "L" and "H" indicate low and high dose of drug, respectively.

Long-term treatment with GLP-1, an exendin, or an exendin or GLP-1 agonist can improve cardiac function, attenuate cardiac remodeling and enhance exercise capacity in a congestive heart failure animal model. Chronic treatment with GLP-1, an exendin, or an exendin or GLP-1 agonist also improves exercise performance and improves insulin sensitivity associated with CHF. GLP-1 and incretin mimetics therefore represent a potentially novel therapeutic approach for the treatment of congestive heart failure.

### Example 6. GLP-1 Used In Combination with ACE Inhibitors for Treatment of Congestive Heart Failure

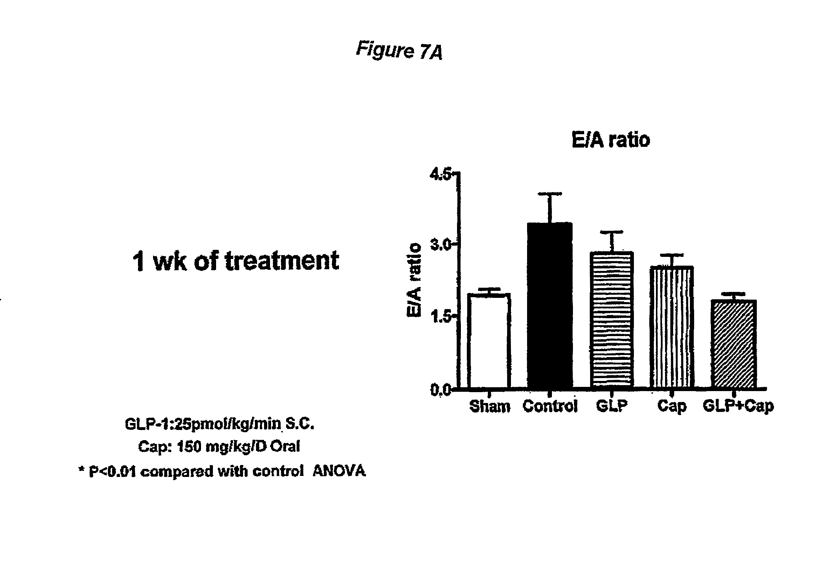

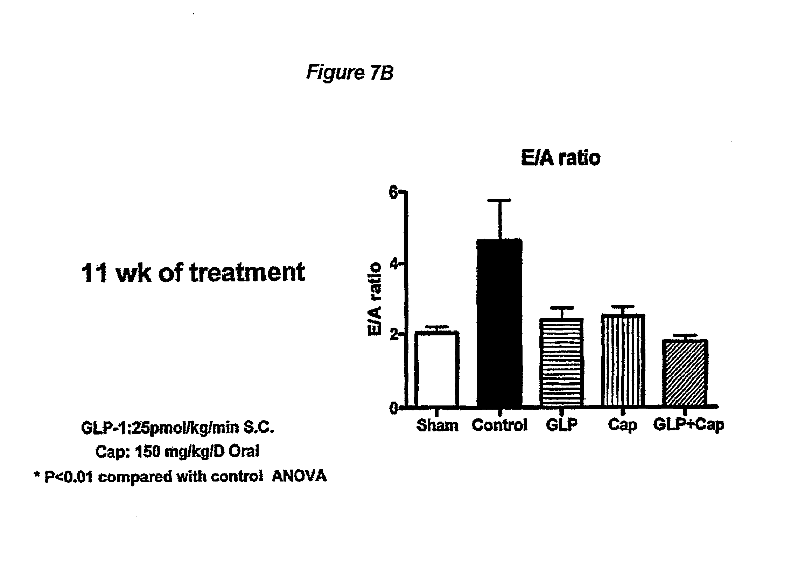

Sprague Dawley rats undergo left coronary artery ligation to induce myocardial infarction (MI) and subsequently CHF as described in Example 5. Starting two weeks after coronary ligation, rats are treated with GLP-1, captopril (150 mg/kg/D oral; "Cap"), combination therapy (GLP-1 and captopril; "GLP+Cap")), or vehicle for 11 weeks. GLP-1 was provided at 25 pmol/kg/min subcutaneously, and captopril was provided at 150 mg/kg/D orally. Combination therapy with GLP-1 and captopril has an additive effect on recovery of E/A ratio, a measurement of cardiac diastolic function. Fig. 7A-B.

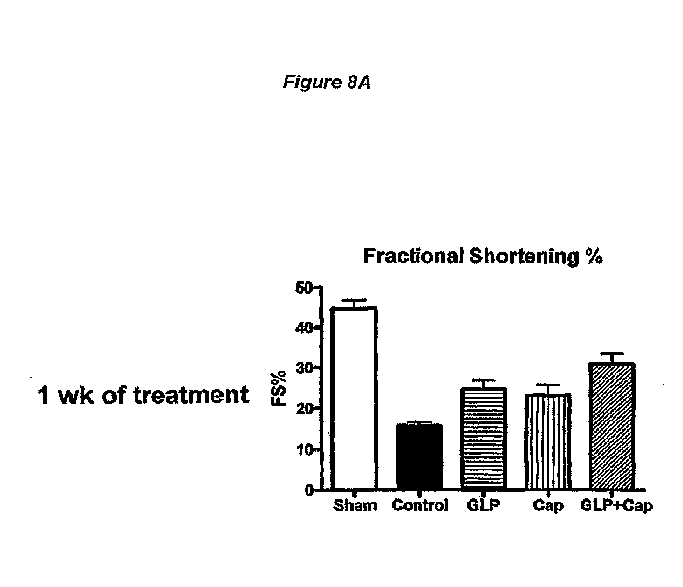

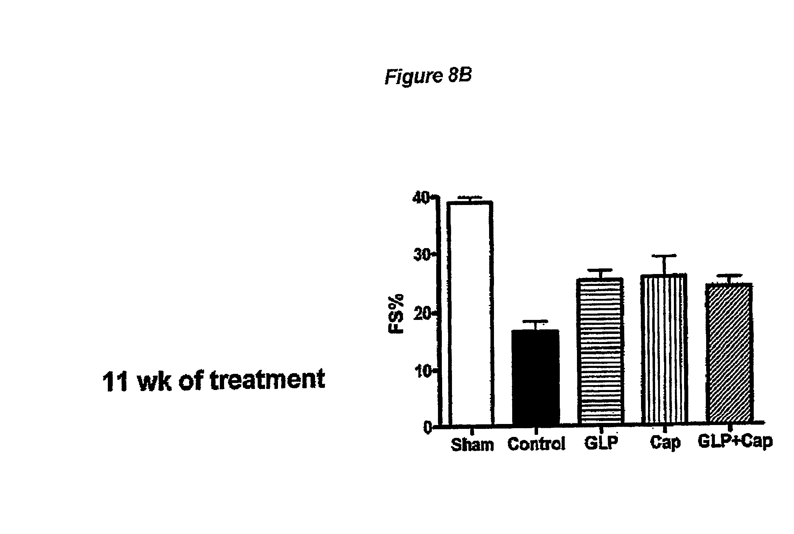

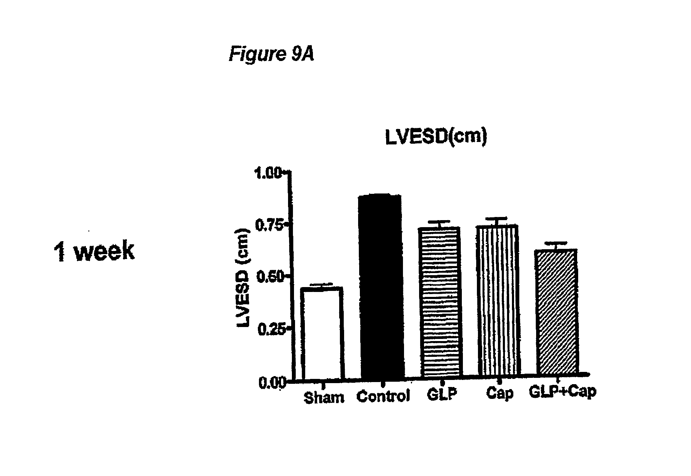

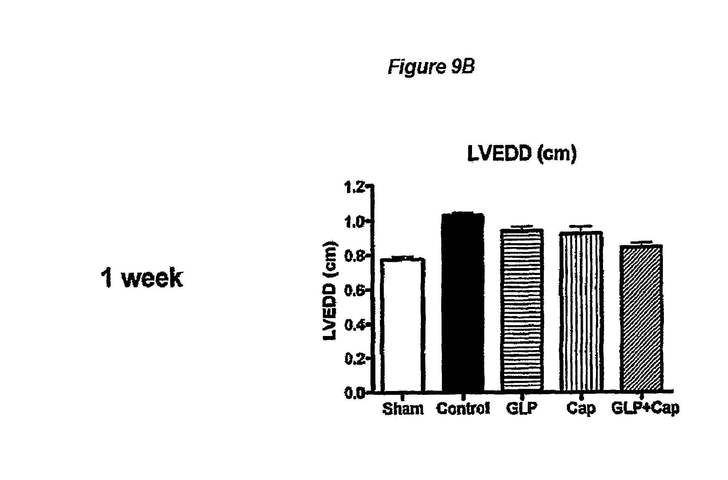

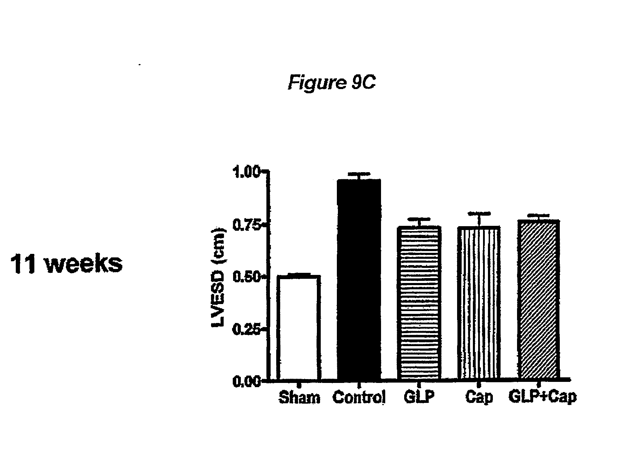

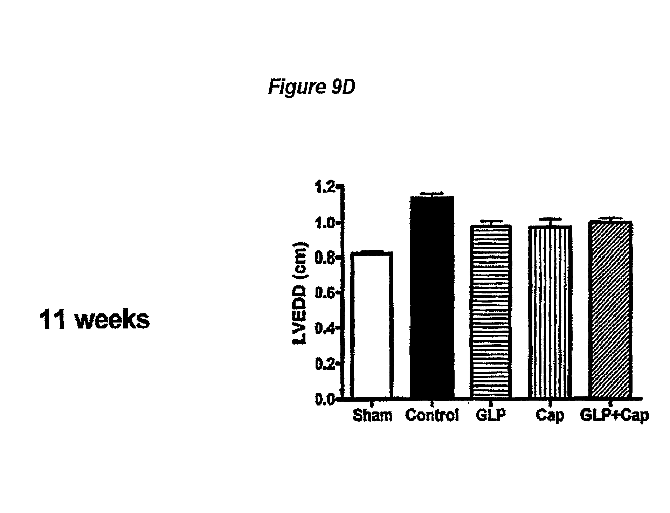

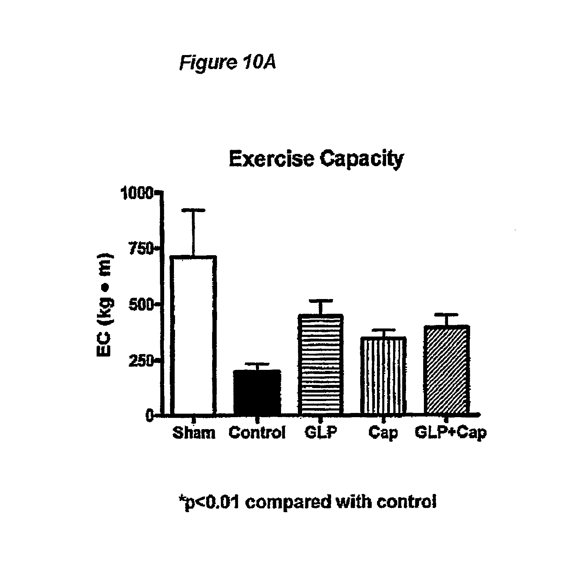

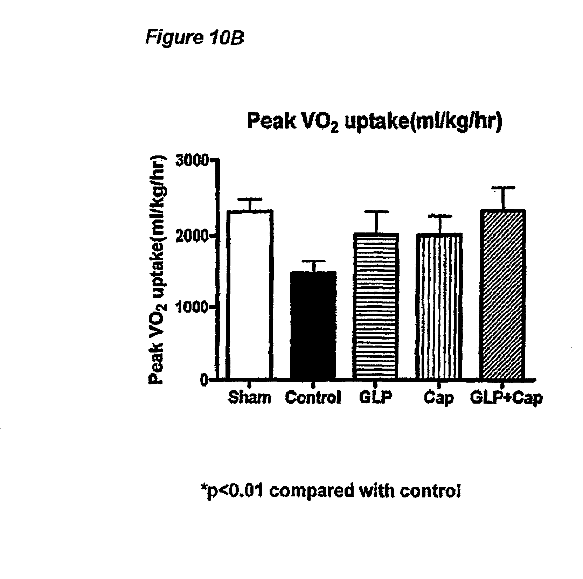

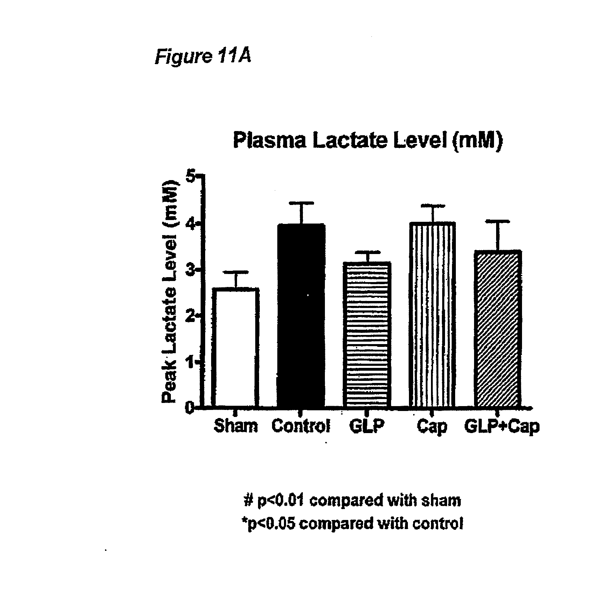

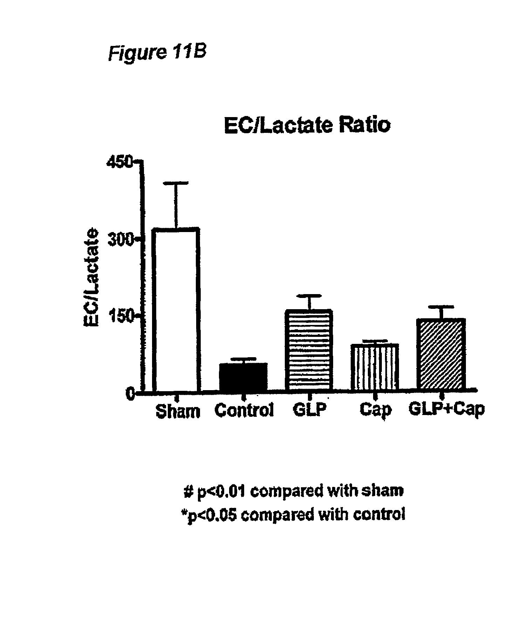

Fig. 8A-B demonstrates that combination therapy with GLP-1 and captopril has an additive effect on improvement of cardiac contractility. Fractional shortening percentage is calculated from LVEDD and LVESD, and is a measurement of cardiac muscle contractility. Combination therapy with GLP-1 and captopril has an additive effect in attenuation of enlargement of left ventricle chamber size, Fig. 9A-B, and improves exercise capacity and efficiency is MI-CHF rats. Fig. 10A-B. Combination therapy with GLP-1 and captopril has an additive effect in the response of exercise capacity-to-peak lactate ratio, and baseline plasma lactate, following MI-induced CHF. Fig. 11A-B.

Taken together these results show that combination therapy with GLP-1 and captopril provides additive cardioprotective effects in the early stage of congestive heart failure.

### Example 7. MI-CHF Screening Model

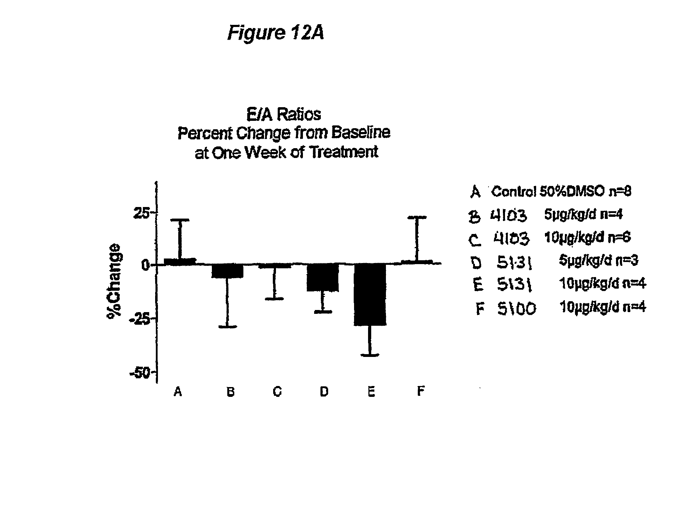

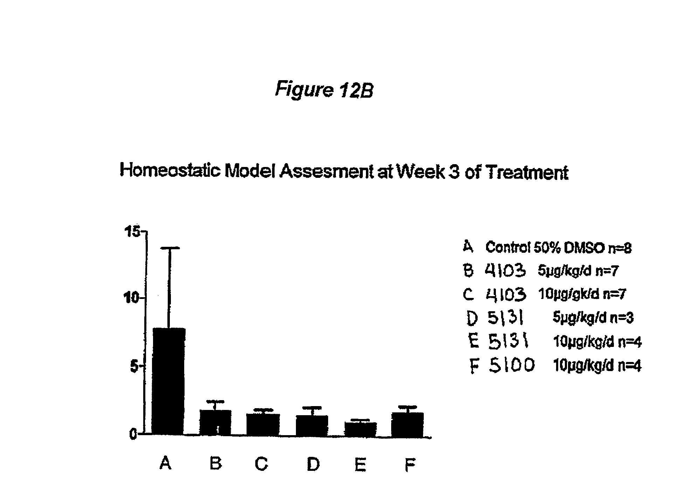

Sprague Dawley rats undergo left coronary artery ligation to induce myocardial infarction (MI) and subsequently CHF as described in Example 5. Starting at 2 weeks after coronary ligation, rats are treated with test compounds at 5 µg/kg/d or 10 µg/kg/d, or vehicle for three weeks. E/A Ratio is measured at one week, according to the procedure described in Example 5. HOMA is measured at three weeks, according to the procedure described in Example 5. As shown, this screening model can be used to identify exendin analogs capable of improving cardiac function and insulin sensitivity following MI-induced CHF. Fig. 12A-B.

### ADDITIONAL ASPECTS OF THE INVENTION

1. A polypeptide comprising the amino acid sequence (SEQ ID NO: 4)
   HGDGTFTSDLSKQMEEEAVRLFIEWLKNGGPSSGAPPPS.
2. A polypeptide comprising the amino acid sequence (SEQ ID NO: 5)
   HGEGTFTSELSKQMEEEAVRLFIEWLKNGGPSSGAPPPS.
3. A polypeptide comprising the amino acid sequence (SEQ ID NO: 6)
   HGEGTFTSDLSKQMEEEAVRLFIEWLKQGGPSSGAPPPS.
4. A polypeptide comprising the amino acid sequence (SEQ ID NO: 7)
   HGEGTFTSDLSKQAEEEAVRLFIEWLKQGGPSSGAPPPS.
5. A polypeptide comprising the amino acid sequence (SEQ ID NO: 8)
   HGEGTFTSDLSKQIEEEAVRLFIEWLKQGGPSSGAPPPS.
6. A polypeptide comprising the amino acid sequence (SEQ ID NO: 9)
   HGEGTFTSDLSKQLEEEAVRLFIEWLKQGGPSSGAPPPS.
7. A polypeptide comprising the amino acid sequence (SEQ ID NO: 10)
   HGEGTFTTDLSKQLEEEAVRLFIEWLKQGGPSSGAPPPS.
8. A polypeptide comprising the amino acid sequence (SEQ ID NO: 11)
   HGEGTFTSDFSKQLEEEAVRLFIEWLKQGGPSSGAPPPS.
9. A polypeptide comprising the amino acid sequence (SEQ ID NO: 12)
   HGEGTFTS[D(OMe)]LSKQLEEEAVRLFlEWLKQGGPSSGAPPPS.
10. A polypeptide comprising the amino acid sequence (SEQ ID NO: 13)
   HGEGTFTS[D(OMe)]LSKQAEEEAVRLFIEWLKQGGPSSGAPPPS.
11. A polypeptide comprising the amino acid sequence (SEQ ID NO: 14)
   HGEGTFTSDLSKQLEEEAVRLFIEWLKQGGPSSGFPPPS.
12. A polypeptide comprising the amino acid sequence (SEQ ID NO: 15)
   HGEGTFTSDLSKQLEEEAVRLFTEWLKNGGPSSGFPPPS.
13. A polypeptide comprising the amino acid sequence (SEQ ID NO: 16)
   HGEGTFTSDLSKQLEEEAVRLFIEWLKQGGPSSGLPPPS.
14. A polypeptide comprising the amino acid sequence (SEQ ID NO: 17)
   HGEGTFTSDLSKQLEEEAVRLFIEWLKQGGPSSGPPPPS.
15. A polypeptide comprising the amino acid sequence (SEQ ID NO: 18)
   HGEGTFTSDLSKQLEEEAVRLFIEWLKQGGPSSGTPPPS.
16. A polypeptide comprising the amino acid sequence (SEQ ID NO: 19)
   HGEGTFTSDLSKQLEEEAVRLFIEWLKQGGPSSGVPPPS.
17. A polypeptide comprising the amino acid sequence (SEQ ID NO: 20)
   HGEGTFTSDLSKQLEEEAVRLFIEWLKQGGPSSGRPPPS.
18. A polypeptide comprising the amino acid sequence (SEQ ID NO: 21)
   HGEGTFTSDLSKQLEEEAVRLFIEWLKQGGPSSGKPPPS.
19. A polypeptide comprising the amino acid sequence (SEQ ID NO: 22)
   HGEGTFTSNLSKQLEEEAVRLFIEWLKQGGPSSGAPPPS,
20. A polypeptide comprising the amino acid sequence (SEQ ID NO: 23)
   HGEGTFTSDKSKQLEEEAVRLFIEWLKQGGPSSGAPPPS.
21. A polypeptide comprising the amino acid sequence (SEQ ID NO: 24)
   HGEGTFTSDWSKQLEEEAVRLFIEWLKQGGPSSGAPPPS.
22. A polypeptide comprising the amino acid sequence (SEQ ID NO: 25)
   HGEGTFTSDESKQLEEEAVRLFIEWLKQGGPSSGAPPPS.
23. A polypeptide comprising the amino acid sequence (SEQ ID NO: 26)
   HGEGTFTSDVTQQLEEEAVRLFIEWLKQGGPSSGAPPPS.
24. A polypeptide comprising the amino acid sequence (SEQ NO: 27)
   HGEGTFTSDLSKQLEEKAAKEFIEWLKQGGPSSGAPPPS.
25. A polypeptide comprising the amino acid sequence (SEQ ID NO: 28)
   HGEGTFTSDLSKQLEEKAVRLFIEWLKQGGPSSGAPPPS.
26. A polypeptide comprising the amino acid sequence (SEQ ID NO: 30)
   HGEGTYTNDLSKQLEEEAVRLFIEWLKQGGPSSGAPPPS.
27. A polypeptide comprising the amino acid sequence (SEQ ID NO: 31)
   HGEGTFTSDVTEYLEEEAVRLFIEWLKQGGPSSGAPPPS.
28. A polypeptide comprising the amino acid sequence (SEQ ID NO: 32)
   HGEGTYTNDVTEYLEEEAVRLFIEWLKQGGPSSGAPPPS.
29. A polypeptide comprising the amino acid sequence (SEQ ID NO: 33)
   HGEGTYTNDVTEYLEEEAVRLFIEWLKNGGPSSGAPPPS.
30. A polypeptide comprising the amino acid sequence (SEQ ID NO: 34)
   HGEGTYTNDVSSYLEEEAARLFIEWLKQGGPSSGAPPPS.
31. A polypeptide comprising the amino acid sequence (SEQ ID NO: 35)
   HGEGTYTNDVSSYLEGQAARLFIEWLQGGPSSGAPPPS.
32. A polypeptide comprising the amino acid sequence (SEQ ID NO: 36)
   HGEGTFTSDLSKQLEERAVRLFIEWLKQGGPSSGAPPPS.
33. A polypeptide comprising the amino acid sequence (SEQ ID NO: 37)
   HGEGTFTSDLSKQKEEEAVRLFIEWLKQGGPSSGAPPPS.
34. A polypeptide comprising the amino acid sequence (SEQ ID NO: 38)
   HGEGTFTSDLSKQLEEEAVRLFIEYLKNGGPSSGAPPPS.
35. A polypeptide comprising the amino acid sequence (SEQ ID NO: 39)
   HGEGTYTNDVTEYLEEEAVRLFIEWLKNGGPSSGAPPPS.
36. A polypeptide comprising the amino acid sequence (SEQ ID NO: 40)
   HGEGTFTSDLSKQLEEEAVRLFIEWLKNGGPSSGFPPPS.
37. A polypeptide comprising the amino acid sequence (SEQ ID NO: 41)
   HGEGTFTSDLSKQSEEEAVRLFIEWLKQGGPSSGAPPPS.
38. A polypeptide comprising the amino acid sequence (SEQ ID NO: 46)
   HGEGTFTSDLSKQLEEEAVRLFIEWLKQ[OctG]GPSSGAPPPS.
39. A polypeptide comprising the amino acid sequence (SEQ ID NO: 47)
   HGEGTFTSDLSKQLEEEAVRLFIEWLKQG[OctG]PSSGAPPPS.
40. A polypeptide comprising the amino acid sequence (SEQ ID NO: 48)
   HGEGTFTSDLSKQLEEEAVRLFIEWLKQGGPSS[OctG]APPPS.
41. A polypeptide comprising the amino acid sequence (SEQ ID NO: 49)
   HGEGTFTSDLSKQAEEEAVRLFIEWLKNGGPSS[OctG]APPPS.
42. A polypeptide comprising the amino acid sequence (SEQ ID NO: 50)
   HGEGTFTSDLSKQAEEEAVRLFIEFLKNGGPSS[OctG]APPPS.
43. A polypeptide comprising the amino acid sequence (SEQ ID NO: 51)
   HGEGTFTSDLSKQLEEEAVRLFIEWLKNGKPKK[OctG]RYS-OH.
44. A polypeptide comprising the amino acid sequence (SEQ ID NO: 52)
   HGEGTFTSDLSKQLEEEAVRLFIEWLKNGGPSKE[OctG]IS-OH.
45. A polypeptide comprising the amino acid sequence (SEQ ID NO: 53)
   HGEGTFTSDLSKQAEEEAVRLFIEWLKNGKPKK[OctG]RYS-OH.
46. A polypeptide comprising the amino acid sequence (SEQ ID NO: 54)
   HGEGTFTSDLSKQLEEEAVRLFIEFLKNGKPKK[OctG]RYS-OH,
47. A polypeptide comprising the amino acid sequence (SEQ ID NO: 56)
   HGEGTFTSDLSKQLEEEAVRLFIEWLIQGGPSKEIIS-OH.
48. A polypeptide comprising the amino acid sequence (SEQ ID NO: 57)
   HGEGTFTSDVTQQLEEEAVRLFIEWLIQGGPSKEIIS-OH.
49. A polypeptide comprising the amino acid sequence (SEQ ID NO: 58)
   HGEGTFTSDLSKQLEEKAAKEFIEWLIQGGPSKEIIS-OH.
50. A polypeptide comprising the amino acid sequence (SEQ ID NO: 59)
   HGEGTFTSDLSKQLEEKAVRLFIEWLIQGGPSKEIIS-OH.
51. A polypeptide comprising the amino acid sequence (SEQ ID NO: 60)
   HGEGTFTSDLSKQLEEEAVRLFIEWLKQGGPSKEIIS-OH.
52. A polypeptide comprising the amino acid sequence (SEQ ID NO: 62)
   HGEGTFTSDLSKQLEEEAVRLFIEWLIKGRP-OH.
53. A polypeptide comprising the amino acid sequence (SEQ ID NO: 66)
   HGEGTFTSDLSKQLEEEAVRLFIEWLKQGKPKKIRYS-OH.
54. A polypeptide comprising the amino acid sequence (SEQ ID NO: 63)
   HGEGTFTSDLSKQLEEEAVRLFIEWLKQGKP-OH.
55. A polypeptide comprising the amino acid sequence (SEQ ID NO: 64)
   HGEGTFTSDLSKQLEEEAVRLFIEWLIQGKP-OH.
56. A polypeptide comprising the amino acid sequence (SEQ ID NO: 67)
   HGEGTFTSDLSKQLEEEAVRLFIEWLKNGGKPKKIRYS-OH.
57. A polypeptide comprising the amino acid sequence (SEQ ID NO: 68)
   HGEGTFTSDLSKQLEEEAVRLFIEWLKNGKPGKGKIRYS-OH.
58. A polypeptide comprising the amino acid sequence (SEQ ID NO: 69)
   HGEGTFTSDLSKQLEEEAVRLFIEWLKNPGGKEIIS-OH.
59. A polypeptide comprising the amino acid sequence (SEQ ID NO: 70)
   HGEGTFTSDLSKQLEEEAVRLFIEWLKNGGPGGKEIIS-OH.
60. A polypeptide comprising the amino acid sequence (SEQ ID NO: 80)
   HAEGTFTSDVSSYLEGQAAKEFIAWLVKGRGGG[DPP4I1].
61. A polypeptide comprising the amino acid sequence (SEQ ID NO: 81)
   HAEGTFTSDVSSYLEGQAAKEFIAWLVKGRGGG[DPP4I2].
62. A polypeptide comprising the amino acid sequence (SEQ ID NO: 82)
   HAEGTFTSDVSSYLEGQAAKEFIAWLVKGRGGGGIPI.
63. A polypeptide comprising the amino acid sequence (SEQ ID NO: 83)
   HAEGTFTSDVSSYLEGQAAKEFIAWLVKGRPSGGGIPI.
64. A polypeptide comprising the amino acid sequence (SEQ ID NO: 84)
   HAFGTFTSDVSSYLEGQAAKEFIAWLVKGRGGGIPI.
65. A polypeptide comprising the amino acid sequence (SEQ ID NO: 85)
   [VPI1]-HAEGTFTSDVSSYLEGQAAKEFLAWLVKGR.
66. A polypeptide comprising the amino acid sequence (SEQ ID NO: 86)
   [VPI1]-HAEGTFTSDVSSYLEGQAAKEFIAWLVKGR.
67. A polypeptide comprising the amino acid sequence (SEQ ID NO: 87)
   HAHGEGTFTSDLSKQLEEEAVRLFIEWLKQGGPSSGAPPPS.
68. A polypeptide comprising the amino acid sequence (SEQ ID NO: 88)
   HAEHGEGTFTSDLSKQLEEEAVRLFIEWLKQGGPSSGAPPPS.
69. A polypeptide comprising the amino acid sequence (SEQ ID NO: 89)
   HAEGHGEGTFTSDLSKQLEEEAVRLFIEWLKQGGPSSGAPPPS.
70. A polypeptide comprising the amino acid sequence (SEQ ID NO: 90)
   YAHGEGTFTSDLSKQLEEEAVRLFIEWLKNGGPSSGAPPPS.
71. A polypeptide comprising the amino acid sequence (SEQ ID NO: 91)
   HSHGEGTFTSDLSKQLEEEAVRLFIEWLKNGGPSSGAPPPS.
72. A polypeptide comprising the amino acid sequence (SEQ ID NO: 92)
   YPHGEGTFTSDLSKQLEEEAVRLFIEWLKNGGPSSGAPPPS.
73. A polypeptide comprising the amino acid sequence (SEQ ID NO: 93)
   Ado-HAEGTFTSDVSSYLEGQAAKEFIAWLVKGR.
74. A polypeptide comprising the amino acid sequence (SEQ ID NO: 96)
   HGEGTFTSDLSKQLEEEAVRLFIEFLKN.
75. A polypeptide comprising the amino acid sequence (SEQ ID NO: 97)
   HGEGTFTSDLSKQLEEEAVRLFIEWLKN.
76. A polypeptide comprising the amino acid sequence (SEQ ID NO: 101)
   HGEGTFTSDLSKQLLEEAVRLKVEFLKN.
77. A polypeptide comprising the amino acid sequence (SEQ ID NO: 102)
   HGEGTFTSDLSKQLQEEAVRLLNEFLKN.
78. A polypeptide comprising the amino acid sequence (SEQ ID NO: 103)
   HGEGTFTSDLSKQVEEEAVRLKNEFLKN.
79. A polypeptide comprising the amino acid sequence (SEQ ID NO: 104)
   HGEGTFTSDLSKQVLEEAVRLLIEFLKN.
80. A polypeptide comprising the amino acid sequence (SEQ ID NO: 98)
   HGEGTFTSDLSKQVQEEAVRLFVEFLKN,
81. A polypeptide comprising the amino acid sequence (SEQ ID NO: 105)
   HGEGTFTSDLSKQQEEEAVRLLVEFLKN.
82. A polypeptide comprising the amino acid sequence (SEQ ID NO: 106)
   HGEGTFTSDLSKQQLEEAVRLFNEFLKN.
83. A polypeptide comprising the amino acid sequence (SEQ ID NO: 99)
   HGEGTFTSDLVKTLEAEAVRKFIEFLKN.
84. A polypeptide comprising the amino acid sequence (SEQ ID NO: 100)
   HGEGTFTSDLSKQ[OctG]BEEAVRLFIEWLKQGGPSSGAPPPS.
85. A method for preventing or treating congestive heart failure comprising administering to a subject in need of prevention or treatment of congestive heart failure, an amount of at least one of the peptides of any of the preceding effective to treat or prevent congestive heart failure.
86. A method for improving cardiac function associated with congestive heart failure comprising administering to a subject in need of improved cardiac function, an amount of at least one of the polypeptides of 1-84 effective to improve cardiac function.
87. A method for attenuating cardiac remodeling comprising administering to a subject in need of attenuation of cardiac remodeling an amount of at least one of the polypeptides of 1-84 effective to attenuate cardiac remodeling.
88. A method of limiting infarct size comprising administering to a subject in need of limiting infarct size, an amount of at least one of the polypeptides of 1-84 effective to limit infarct size.
89. A method of attenuating insulin resistance comprising administering to a subject in need of limiting insulin resistance an amount of at least one of the polypeptides of 1-84 effective to limit insulin resistance.
90. A method of improving exercise capacity in a subject with congestive heart failure comprising administering to a subject in need of improving exercise capacity an amount of at least one of the polypeptides of 1-84 effective to improve exercise capacity.
91. A method for treating diabetes mellitus comprising administering to a subject in need of such treatment an amount of at least one of the polypeptides of 1-84 effective to treat diabetes mellitus.
92. A method of treating insulin resistance comprising administering to a subject in need of such treatment an amount of at least one of the polypeptides of 1-84 effective to treat insulin resistance.
93. A method of treating postprandial hyperglycemia comprising administering to a subject in need of such treatment an amount of at least one of the polypeptides of 1-84 effective to treat postprandial hyperglycemia.
94. A method of lowering blood glucose comprising administering to a subject in need of such lowering an amount of at least one of the polypeptides of 1-84 effective to lower blood glucose.
95. A method of stimulating insulin response comprising administering to a subject in need of such insulin response an amount of at least one of the polypeptides of 1-84 effective to stimulate an insulin response.
96. A method for reducing food intake comprising administering to a subject in need of or desirous of such reduction an amount of at least one of the polypeptides of 1-84 effective to reduce food intake.
97. A method for reducing appetite comprising administering to a subject in need of or desirous of such reduction an amount of at least one of the polypeptides of 1-84 effective to reduce appetite.
98. A method for treating obesity comprising administering to a subject in need of such treatment an amount of at least one of the polypeptides of 1-84 effective to treat obesity.
99. A method for treating obesity-related cardiac disease comprising administering to a subject in need of such treatment an amount of at least one of the polypeptides of 1-84 effective to treat obesity-related cardiac disease.
100. A method for treating dyslipidemia comprising administering to a subject in need of such treatment an amount of at least one of the polypeptides of 1-84 effective to lower plasma lipids.
101. A method for treating hypertriglyceridemia comprising administering to a subject in need of such treatment an amount of at least one of the polypeptides of 1-84 effective to lower plasma triglycerides.
102. The use of at least one of the polypeptides of any of 1-84 to treat or prevent congestive heart failure.
103. The use of at least one of the polypeptides of any of 1-84 to improve cardiac function associated with congestive heart failure.
104. The use of at least one of the polypeptides of any of 1-84 to attenuate cardiac remodeling.
105. The use of at least one of the polypeptides of any of 1-84 to limit infarct size.
106. The use of at least one of the polypeptides of any of 1-84 to attenuate insulin resistance.
107. The use of at least one of the polypeptides of any of 1-84 to improve exercise capacity in a subject having congestive heart failure.
108. The use of at least one of the polypeptides of any of 1-84 to treat diabetes mellitus.
109. The use of at least one of the polypeptides of any of 1-84 to treat insulin resistance.
110. The use of at least one of the polypeptides of any of 1-84 to treat postprandial hyperglycemia.
111. The use of at least one of the polypeptides of any of 1-84 to lower blood glucose.
112. The use of at least one of the polypeptides of any of 1-84 to stimulate insulin release.
113. The use of at least one of the polypeptides of any of 1-84 to reduce food intake.
114. The use of at least one of the polypeptides of any of 1-84 to reduce appetite.
115. The use of at least one of the polypeptides of any of 1-84 to treat obesity.
116. The use of at least one of the polypeptides of any of 1-84 to treat obesity-related cardiac disease.
117. The use of at least one of the polypeptides of any of 1-84 to treat obesity-related congestive heart failure.
118. The use of at least one of the polypeptides of any of 1-84 to treat dyslipidemia.
119. The use of at least one of the polypeptides of any of 1-84 to treat hypertriglyceridemia.

## Claims

1. A polypeptide comprising the amino acid sequence of any one of SEQ ID NOS. 27, 44, 60, 61, 29, 42, 43, 45, 55, 65 or 71 to 79.

2. A polypeptide according to claim 1 comprising the amino acid sequence of any one of SEQ ID NOS. 27, 44, 60 or 61.

3. A polypeptide according to claim 1 or claim 2 comprising the amino acid sequence of SEQ ID NO. 27.

4. A polypeptide according to claim 1 or claim 2 comprising the amino acid sequence of SEQ ID NO. 44.

5. A polypeptide according to claim 1 or claim 2 comprising the amino acid sequence of SEQ ID NO. 60.

6. A polypeptide according to claim 1 or claim 2 comprising the amino acid sequence of SEQ ID NO. 61.

7. A polypeptide according to any one of claims 1 to 6, further comprising a polyamino acid, a fatty acid, a polyethylene glycol polymer, albumin, gelatin, an immunoglobulin, a monoclonal antibody, a catalytic antibody, or a C₁-C₂₀ alkyl side chain.

8. A pharmaceutical composition comprising a polypeptide according to any one of claims 1 to 7 and a pharmaceutically acceptable carrier.

9. A polypeptide comprising the amino acid sequence of any one of SEQ ID NOS. 27, 44, 60, 61, 29, 42, 43, 45, 55, 65 or 71 to 79 for use in the treatment or prevention of congestive heart failure.

10. A polypeptide for use according to claim 9 in the treatment of congestive heart failure.

11. A polypeptide for use according to claim 10 wherein the treatment is chronic treatment.

12. A polypeptide for use according to any one of claims 9 to 11 wherein the polypeptide is as defined in any one of claims 2 to 7.

13. A polypeptide for use according to any one of claims 9 to 12 wherein the polypeptide comprises the amino acid sequence of SEQ ID NO. 27.

14. A polypeptide for use according to any one of claims 9 to 12 wherein the polypeptide comprises the amino acid sequence of SEQ ID NO. 44.

15. A polypeptide for use according to any one of claims 9 to 12 wherein the polypeptide comprises the amino acid sequence of SEQ ID NOS. 60 or 61.
